# EUROPEAN PATENT APPLICATION

(11) **EP 4 056 586 A1**
(43) Date of publication of application: **14.09.2022**
(21) Application number: 20885858.9
(22) Date of filing: 09.11.2020
(51) Int. Cl.: C07K 14/705, C07K 16/28, A61K 47/68, A61P 35/00, A61K 39/00

(54) **EPITOPE OF REGULATORY T CELL SURFACE ANTIGEN, AND ANTIBODY SPECIFICALLY BINDING THERETO**

(30) Priority: 08.11.2019 KR 20190142251
(71) Applicant: Good T Cells, Inc., Seodaemun-gu Seoul 03722 (KR)
(72) Inventor: KIM, Jung Ho, Seoul 04136 (KR); KIM, Beom Seok, Seoul 04029 (KR)
(74) Representative: Müller, Christian Stefan Gerd
(86) International application number: PCT/KR2020/015656
(87) International publication number: WO 2021/091359

(57) **Abstract**

The present invention relates to an epitope of Lrig-1 (leucine-rich repeats and immunoglobulin-like domains 1) protein, which is an antigen present on the surface of a regulatory T cell, and an antibody or antigen-binding fragment that binds specifically thereto.

## Description

### Technical Field

The present invention relates to an epitope of Lrig-1 (leucine-rich and immunoglobulin-like domains 1) protein, which is an antigen present on the surfaces of regulatory T cells, and an antibody or antigen-binding fragment that binds specifically thereto.

### Background Art

One of the most remarkable properties of every normal individual's immune system is its ability to recognize and eliminate non-self antigens while not reacting harmfully to that of self-antigenic substances. Such unresponsiveness to self-antigens is called immunologic unresponsiveness or tolerance. Self-tolerance occurs either by elimination of lymphocytes that may have specific receptors for self-antigens or by inactivation of self-reactive lymphocytes after contact with self-antigens. Abnormalities in the induction or maintenance of self-tolerance lead to immune responses against self-antigens, which may result in disorders called autoimmune diseases.

For the treatment of the autoimmune disease, the concept of suppressor T cells with the possibility of presence of T cells capable of controlling and suppressing the effector function of conventional T cells was first proposed by Gershon in the early 1970s (R. K. Gershon and K. Kondo, Immunology, 1970, 18: 723-37). Since then, research has been conducted to elucidate the biological properties and functions of regulatory T cells in many fields of immunology.

Accordingly, the regulatory T cells (Treg) play an important role in naturally preventing the occurrence of excessive inflammation and immune responses, but it has been reported that, when autoimmune diseases and chronic inflammatory diseases occur, the function and number of regulatory T cells remarkably decrease. Thus, for patients with immune diseases and inflammatory diseases, it is important that regulatory T cells are generated at normal levels, which can be one of methods for treatment of these diseases.

Until now, studies on genes and proteins present specifically in regulatory T cells have been conducted, suggesting that substances such as CD25, CTLA4, CD62L, CD38, CD103, GITR, and CD45RB may correspond to marker substances. However, genes or proteins that can target regulatory T cells alone have not yet been reported.

Meanwhile, the variable domain of an antibody includes three hypervariable regions, called complementarity determining regions (hereinafter referred to as "CDRs"), and four framework regions. The CDRs mainly serve to bind to an antigenic determinant (epitope) of an antigen. The CDRs of each chain are typically referred to as CDR1, CDR2, and CDR3, numbered sequentially starting from the N-terminus, and are also typically identified by the chain in which the particular CDR is located.

### DISCLOSURE

### Technical Problem

An object of the present invention is to provide an epitope of Lrig-1 (leucine-rich and immunoglobulin-like domains 1) protein present on the surface of a regulatory T cells (Treg cell).

Another object of the present invention is to provide an antibody or antigen-binding fragment capable of binding specifically to the epitope.

Still another object of the present invention is to provide a pharmaceutical composition for preventing or treating cancer containing, as an active ingredient, the antibody or antigen-binding fragment capable of specifically binding to the epitope.

Yet another object of the present invention is to provide an antibody-drug conjugate (ADC) in which a drug, for example, an anticancer drug, is conjugated to the antibody.

Still yet another object of the present invention is to provide a pharmaceutical composition for preventing or treating cancer containing the antibody-drug conjugate as an active ingredient.

A further object of the present invention is to provide a method of preventing or treating cancer using the antibody or antigen-binding fragment capable of specifically binding to the epitope, and the antibody-drug conjugate.

However, objects to be achieved by the present invention are not limited to the objects mentioned above, and other objects not mentioned herein will be clearly understood by those of ordinary skill in the art from the following description.

### Technical Solution

The present invention is directed to an epitope of Lrig-1 (leucine-rich and immunoglobulin-like domains 1) protein or an antibody or antigen-binding fragment that binds specifically to the epitope.

In the present invention, the "Lrig-1 protein" is a transmembrane protein present on the surfaces of regulatory T cells, and is composed of leucine-rich repeats (LRRs) and three immunoglobulin-like domains on the extracellular or lumen side, a cell transmembrane sequence, and a cytoplasmic tail region. The LRIG gene family includes LRIG1, LRIG2, and LRIG3, and the amino acids constituting each member of the family are highly conserved. The LRIG1 gene is highly expressed in normal skin, and can be expressed in basal and hair follicle cells to regulate proliferation of epithelial stem cells. Thus, the LRIG1 gene plays an important role in maintaining homeostasis of the epidermis, and its absence may lead to psoriasis or skin cancer. It has been reported that a deletion of chromosome region 3p14.3 in which LRIG1 is located is likely to develop into cancer cells. In fact, it was found that expression of LRIG1 greatly decreased in renal cell carcinoma and cutaneous squamous cell carcinoma. In recent years, it has also been found that Lrig-1 is expressed in only about 20 to 30% of cancers. Meanwhile, for the purpose of the present invention, the Lrig-1 protein may be a protein present in humans or mice, without being limited thereto.

In one embodiment of the present invention, the Lrig-1 protein may be a Lrig-1 protein derived from mammals, including primates such as humans and monkeys, rodents such as mice and rats, and the like.

In one embodiment of the present invention, the Lrig-1 protein may be a human-derived Lrig-1 protein represented by SEQ ID NO: 1, which may be encoded by the nucleic acid sequence represented by SEQ ID NO: 2, without being limited thereto.

In another embodiment of the present invention, the Lrig-1 protein may be a mouse-derived Lrig-1 protein represented by SEQ ID NO: 3, which may be encoded by the nucleic acid sequence represented by SEQ ID NO: 4, without being limited thereto.

In another embodiment of the present invention, the Lrig-1 protein may be an extracellular domain of the Lrig-1 protein, without being limited thereto.

The Lrig-1 extracellular domain in the present invention may be an extracellular domain of Lrig-1 protein derived from mammals including primates such as humans and monkeys, rodents such as mice and rats, and the like. For the purpose of the present invention, the extracellular protein of the Lrig-1 protein may be an extracellular domain of Lrig-1 protein derived from a human or mouse, without being limited thereto.

In one embodiment of the present invention, the extracellular domain of the Lrig-1 protein may be represented by SEQ ID NO: 5, which corresponds to amino acid positions 35 to 794 in the amino acid sequence of the human-derived Lrig-1 protein, without being limited thereto.

In another example of the present invention, the extracellular domain of the Lrig-1 protein may be represented by SEQ ID NO: 6, which corresponds to amino acid positions 35 to 794 in the amino acid sequence of the mouse-derived Lrig-1 protein, without being limited thereto.

Hereinafter, the present invention will be described in more detail.

### 1. Epitope of Lrig-1 protein

According to one embodiment of the present invention, the epitope of the Lrig-1 protein may consist of some amino acids (e.g., 2 to 50, 6 to 45, or 10 to 44 amino acids) of the Lrig-1 protein represented by SEQ ID NOs: 1, 3, or 5, without being limited thereto. Another embodiment of the present invention provides an epitope of Lrig-1 protein comprising at least one polypeptide selected from the group consisting of polypeptides consisting of the amino acid sequence represented by SEQ ID NOs: 7 to 70.

In one embodiment of the present invention, the epitope of the Lirg-1 protein may be an epitope comprising a polypeptide consisting of the amino acid sequence represented by SEQ ID NO: 71 or SEQ ID NO: 72, without being limited thereto.

The epitope of the present invention may be a conformational epitope.

The "conformational epitope" of the present invention consists of a discontinuous amino acid sequence, unlike a one-dimensional linear epitope consisting of a continuous sequence. This conformational epitope reacts with the three-dimensional structure of the antigen-binding site of an antibody.

### 2. Nucleic acid molecule encoding an epitope of Lrig-1 protein

Another embodiment of the present invention provides a nucleic acid molecule encoding the epitope provided in the present invention.

Nucleic acid molecules of the present invention include all nucleic acid molecules having polynucleotide sequences which are translated into the amino acid sequences of the polypeptides provided by the present invention, as known to those skilled in the art. Therefore, various polynucleotide sequences with open reading frames (ORFs) may be produced, which are also all included in the nucleic acid molecules of the present invention.

### 3. Expression vector into which nucleic acid molecule encoding epitope of Lrig-1 protein has been inserted

Another embodiment of the present invention provides an expression vector into which the isolated nucleic acid molecule provided in the present invention has been inserted.

In the present invention, the "vector" is any nucleic acid molecule capable of transporting another nucleic acid linked thereto. One type of vector is a "plasmid," which refers to a circular double-stranded DNA to which an additional DNA segment may be ligated. Another type of vector is a phage vector. Yet another type of vector is a viral vector, where an additional DNA segment can be ligated into the viral genome. Certain vectors are capable of autonomous replication in a host cell into which they have been introduced (for example, bacterial vectors having a bacterial origin of replication are episomal mammalian vectors). Other vectors (e.g., non-episomal mammalian vectors) can be integrated into the genome of a host cell upon introduction into the host cell, and thus are replicated along with the host genome. In addition, certain vectors are capable of directing expression of genes to which they are operatively linked. Such vectors are referred to herein as "recombinant expression vectors" or simply "expression vectors." In general, expression vectors useful in recombinant DNA techniques are often in the form of plasmids. In the present specification, the terms "plasmid" and "vector" may be used interchangeably as the plasmid is the most commonly used form of vector.

Specific examples of the expression vector in the present invention may be selected from the group consisting of commercially widely used pCDNA vectors, F, R1, RP1, Col, pBR322, ToL, Ti vectors; cosmids; phages such as lambda, lambdoid, M13, Mu, p1 P22, Qµµ , T-even, T2, T3, T7; and plant viruses, without being limited thereto. Any expression vector known as expression vectors to those skilled in the art may be used in the present invention, and the expression vector is selected depending on the nature of the target host cell. Introduction of a vector into a host cell may be performed by calcium phosphate transfection, viral infection, DEAE-dextran-mediated transfection, lipofectamine transfection, or electroporation, without being limited thereto, and those skilled in the art may adopt and use an introduction method appropriate for the expression vector and the host cell which are used. The vector may preferably contain at least one selection marker, without being limited thereto, and selection can be performed using a vector that contains no selection marker, depending on whether or not a product is produced. The selection marker is chosen depending on the target host cell, which is done using methods already known to those skilled in the art, and thus the present invention has no limitation thereon.

In order to facilitate purification of the protein encoded by the nucleic acid molecule of the present invention, a tag sequence may be inserted into and fused to the expression vector. Examples of the tag include, but are not limited to, hexa-histidine tag, hemagglutinin tag, myc tag, or flag tag, and any tag known to those skilled in the art which facilitates purification may be used in the present invention.

### 4. Host cell line transformed with expression vector into which nucleic acid molecule encoding epitope of Lrig-1 protein has been inserted

Another embodiment of the present invention provides a host cell line transformed with the expression vector provided in the present invention.

In the present invention, the term "host cell" includes individual cells or cell cultures which may be or have been recipients of the vector(s) for incorporation of a polypeptide insert. The host cells include progeny of a single host cell, and the progeny may not necessarily be completely identical (in morphology or in genomic DNA complement) to the original parent cell due to natural, accidental, or intentional mutation. The host cells include cells transfected *in vivo* with the polynucleotide(s) of the present invention.

In the present invention, the host cells may include cells of mammalian, plant, insect, fungal, or cellular origin, and may be, for example, bacterial cells such as *E. coli, Streptomyces, Salmonella typhimurium*; fungal cells such as yeast cells and *Pichia pastoris*; insect cells such as Drosophila and *Spodoptera* Sf9 cells; animal cells such as Chinese hamster ovary (CHO) cells, SP2/0 (mouse myeloma), human lymphoblastoid, COS, NSO (mouse myeloma), 293T, Bowes melanoma cells, HT-1080, baby hamster kidney (BHK) cells, human embryonic kidney (HEK) cells, or PERC.6 (human retinal cells); or plant cells, without being limited thereto, any cell type known to those skilled in the art which may be used as a host cell line may be used.

The transformation method of the present invention is any method of introducing a desired vector into the host cell, and may include any known method capable of introducing the vector into the host cell. For example, a method using CaCl₂, electroporation, microinjection, calcium phosphate precipitation, electroporation, liposome-mediated transfection, DEAE-dextran treatment, gene bombardment, and transfection using virus, may be used, without being limited thereto.

### 5. Antibody or antigen-binding fragment

Another embodiment of the present invention provides an antibody or antigen-binding fragment that binds specifically to an epitope of the present invention.

In the present invention, the antibody is a full-length antibody or a part of the antibody, has the ability to bind to the Lrig-1 protein, and includes any antibody fragment that binds to the Lrig-1 antigen determining region in a manner competitive with the binding molecule of the present invention.

In the present invention, the term "antibody" refers to a protein molecule which serves as a receptor that specifically recognizes an antigen, including an immunoglobulin molecule that is immunologically reactive with a particular antigen. For the purpose of the present invention, the antigen may be Lrig-1 protein present on the surfaces of regulatory T cells. Preferably, the antibody may specifically recognize the leucine-rich region or immunoglobulin-like domain of the Lrig-1 protein, without being limited thereto.

In the present invention, the "immunoglobulin" has a heavy chain and a light chain, and each of the heavy chain and the light chain comprises a constant region and a variable region. The variable region of each of the light chain and the heavy chain contains three hypervariable regions, called complementarity determining regions (hereinafter referred to as "CDRs"), and four framework regions. The CDRs primarily serve to bind to an antigenic determinant (epitope) of an antigen. The CDRs of each chain are typically referred to as CDR1, CDR2, and CDR3, numbered sequentially starting from the N-terminus, and are also typically identified by the chain in which the particular CDR is located.

In the present invention, the "full-length antibody" has a structure having two full-length light chains and two full-length heavy chains, wherein each of the light chains is linked to the heavy chain via a disulfide bond. Examples of the full-length antibody include IgA, IgD, IgE, IgM, and IgG. Subtypes of the IgG include IgG1, IgG2, IgG3, and IgG4.

In addition, as used herein, the term "antigen-binding fragment" refers to a fragment having an antigen-binding function. Examples of the antigen-binding fragment include: (1) a Fab fragment consisting of a light chain variable region (VL), a heavy chain variable region (VH), a light chain constant region (CL), and a heavy chain constant region 1 (CH1); (2) a Fd fragment consisting of VH and CH1 domains; (3) a Fv fragment consisting of VL and VH domains of a single antibody; (4) a dAb fragment consisting of a VH domain; (5) an isolated CDR region; (6) a F(ab')₂ fragment which is a bivalent fragment including two linked Fab fragments; (7) a single-chain Fv molecule (scFv) in which a VH domain and a VL domain are linked together by a peptide linker that allows the two domains to associate to form an antigen-binding site; (8) a bispecific single-chain Fv dimer; and (9) a diabody which is a multivalent or multispecific fragment constructed by gene fusion. The antigen-binding fragment may be obtained as a Fab or F(ab')2 fragment when a proteolytic enzyme, for example, papain or pepsin is used, and the antigen-binding fragment may be produced through a genetic recombination technique.

In addition, in the present invention, the antibody may be, without limitation, a monoclonal antibody, a polyclonal antibody, a chimeric antibody, a humanized antibody, a human antibody, a bivalent, bispecific molecule, a minibody, a domain antibody, a bispecific antibody, an antibody mimetic, a unibody, a diabody, a triabody, a tetrabody, or a fragment thereof.

In addition, as used herein, the term "monoclonal antibody" refers to an antibody molecule having a single molecular composition, which is obtained from a population of substantially identical antibodies, and exhibits single binding specificity and affinity for a particular epitope.

In the present invention, the "chimeric antibody" is an antibody which is obtained by recombination of a variable region of a mouse antibody and a constant region of a human antibody, and has a greatly improved immune response compared to the mouse antibody.

In addition, as used herein, the term "humanized antibody" refers to an antibody obtained by modifying the protein sequence of an antibody of non-human species origin so that the protein sequence is similar to an antibody variant naturally produced in humans. For example, the humanized antibody may be produced by recombining mouse-derived CDRs with a human antibody-derived FR to obtain a humanized variable region, and recombining the humanized variable region with a constant region of a desired human antibody.

As used herein, the term "binding" or "specific binding" refers to the affinity of the antibody or antibody composition of the present invention for an antigen. In antigen-antibody binding, the "specific binding" is distinguishable from non-specific background binding, typically when the dissociation constant (Kd) is less than 1×10⁻⁵ M, less than 1×10⁻⁶M, or less than 1×10⁻⁷ M. Specific binding can be detected by methods known in the art, such as ELISA, surface plasmon resonance (SPR), immunoprecipitation, and coprecipitation, which include an appropriate control that can distinguish between non-specific binding and specific binding.

The antibody or antigen-binding fragment of the present invention may exist as a multimer, such as a dimer, a trimer, a tetramer, or a pentamer, which includes at least a portion of the antigen-binding activity of a monomer. Such multimers also include a homomultimer or a heteromultimer. Antibody multimers contain a large number of antigen-binding sites, and thus have excellent antigen-binding activity compared to monomers. Antibody multimers are also easily used to produce multifunctional (bifunctional, trifunctional or tetrafunctional) antibodies.

As used herein, the term "multifunctional" refers to an antibody or antigen-binding fragment which has two or more activities or functions (e.g., antigen-binding activity, enzymatic activity, and ligand- or receptor-binding activity). For example, the antibody of the present invention may bind to a polypeptide having enzymatic activity, such as luciferase, acetyltransferase, or galactosidase. Multifunctional antibodies also include multivalent or multispecific (e.g., bispecific, trispecific, etc.) forms of antibodies.

### 6. Pharmaceutical composition containing antibody or antigen-binding fragment as active ingredient

In addition, the antibody according to the present invention may bind specifically to an epitope comprising a polypeptide represented by any one of SEQ ID NOs: 7 to 72 present on regulatory T cells, thereby activating, maintaining or inhibiting the function of the regulatory T cells to regulate the activity of effector T cells, thereby preventing or treating cancer.

In the present invention, the term "cancer" indicates or refers to a physiological condition typically characterized by unregulated cell growth in mammals. Cancer to be prevented, ameliorated or treated in the present invention may be a solid tumor consisting of an agglomerate generated by abnormal cell growth in a solid organ, and may be, without limitation, gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, or lung cancer, depending on the location of the solid organ.

### 7. Antibody-drug conjugate (ADC)

Another embodiment of the present invention provides an antibody-drug conjugate (ADC) comprising: the antibody or antigen-binding fragment provided by the present invention; and a drug.

As used herein, the term "antibody-drug conjugate (ADC)" refers to a form in which the drug and the antibody are chemically linked to each other without degrading the biological activities of the antibody and the drug. In the present invention, the antibody-drug conjugate refers to a form in which the drug is conjugated to the N-terminal amino acid residue of the heavy and/or light chain of the antibody, specifically, a form in which the drug is conjugated to the N-terminal α-amine group of the heavy and/or light chain of the antibody.

As used herein, the "drug" may mean any substance having a certain biological activity for a cell, which is a concept including DNA, RNA, or a peptide. The drug may be in a form containing a reactive group capable of reacting and crosslinking with an α-amine group, and also includes a form to which a linker containing a reactive group capable of reacting and crosslinking with an α-amine group is connected.

In the present invention, examples of the reactive group capable of reacting and crosslinking with the α-amine group include, without particular limitation, any amine-reactive groups known in the art, which are capable of reacting and crosslinking with the N-terminal α-amine group of the heavy or light chain of the antibody. The reactive group may, for example, be any one of isothiocyanate, isocyanate, acyl azide, NHS ester, sulfonyl chloride, aldehyde, glyoxal, epoxide, oxirane, carbonate, aryl halide, imidoester, carbodiimide, anhydride, and fluorophenyl ester, without being limited thereto.

In the present invention, the antibody-drug conjugate comprises, as the antibody or antigen-binding fragment, an antibody or antigen-binding fragment that binds specifically to the epitope of the present invention, that is, an epitope of Lrig-1 protein, or an epitope comprising a polypeptide consisting of some amino acids of the amino acid sequence of the extracellular domain of the Lrig-1 protein, or an epitope comprising a polypeptide consisting of the amino acid sequence represented by Formula 1, or an epitope comprising a polypeptide represented by the amino acid sequence of any one of SEQ ID NOs: 48 to 113, wherein the drug may be any drug capable of treating a disease targeted by the Lrig-1 antibody, for example, an anticancer drug capable of treating cancer, without being limited thereto.

In one embodiment of the present invention, the anticancer drug may be, without limitation, any drug that may be used for the prevention, amelioration or treatment of cancer. For example, the anticancer drug may be selected from the group consisting of nitrogen mustard, imatinib, oxaliplatin, rituximab, erlotinib, neratinib, lapatinib, gefitinib, vandetanib, nilotinib, semaxanib, bosutinib, axitinib, cediranib, lestaurtinib, trastuzumab, gefitinib, bortezomib, sunitinib, carboplatin, sorafenib, bevacizumab, cisplatin, cetuximab, Viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracil, oteracil, azacitidine, methotrexate, uracil, cytarabine, fluorouracil, fludarabine, enocitabine, flutamide, capecitabine, decitabine, mercaptopurine, thioguanine, cladribine, carmofur, raltitrexed, docetaxel, paclitaxel, irinotecan, belotecan, topotecan, vinorelbine, etoposide, vinblastine, idarubicin, mitomycin, bleomycin, dactinomycin, pirarubicin, aclarubicin, peplomycin, temsirolimus, temozolomide, busulfan, ifosfamide, cyclophosphamide, melphalan, altretamine, dacarbazine, thiotepa, nimustine, chlorambucil, mitolactol, leucovorin, tretinoin, exemestane, aminoglutethimide, anagrelide, olaparib, navelbine, fadrozole, tamoxifen, toremifene, testolactone, anastrozole, letrozole, vorozole, bicalutamide, lomustine, 5FU, vorinostat, entinostat, and carmustine, without being limited thereto.

### 8. Pharmaceutical composition containing antibody-drug conjugate (ADC) as active ingredient

Another embodiment of the present invention provides a pharmaceutical composition for preventing or treating cancer containing, as an active ingredient, the antibody or antigen-binding fragment provided by the present invention, or the antibody-drug conjugate (ADC).

In the present invention, the antibody or antigen-binding fragment or the antibody-drug conjugate obtained by conjugating the drug thereto, which is contained in an active ingredient in the pharmaceutical composition, may bind specifically to an epitope comprising a polypeptide represented by any one of the amino acid sequences of SEQ ID NOs: 7 to 72, thereby inhibiting the function of regulatory T cells and maintaining or increasing the activity of effector T cells, thereby very effectively treating cancer.

The cancer in the present invention may be a solid tumor consisting of an agglomerate generated by abnormal cell growth in a solid organ. In a specific embodiment, the cancer may be, without limitation, gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, or lung cancer, depending on the location of the solid organ.

### 9. Others

Meanwhile, in the present invention, "prevention" may include, without limitation, any action that blocks the symptoms of a disease or suppresses or delays the symptoms of the disease by using the pharmaceutical composition of the present invention.

In addition, in the present invention, "treatment" may include, without limitation, any action that alleviates or beneficially alters symptoms of a disease by using the pharmaceutical composition of the present invention.

In the present invention, the pharmaceutical composition may be in the form of capsules, tablets, granules, injections, ointments, powders, or beverages, and the pharmaceutical composition may be for administration to humans.

In the present invention, for use, the pharmaceutical composition may be formulated in the form of oral preparations such as powders, granules, capsules, tablets, and aqueous suspensions, preparations for external use, suppositories, and sterile injectable solutions, according to the respective conventional methods, without being limited thereto. The pharmaceutical composition of the present invention may further contain pharmaceutically acceptable carriers. As the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preserving agent, a pain-relieving agent, a solubilizer, an isotonic agent, a stabilizer, and the like may be used for injection; and a base, an excipient, a lubricant, a preserving agent, and the like may be used for topical administration. In addition, the pharmaceutical composition of the present invention may be prepared in various dosage forms by being mixed with the pharmaceutically acceptable carriers as described above. For example, for oral administration, the pharmaceutical composition may be formulated in the form of tablets, troches, capsules, elixirs, suspensions, syrups, wafers, or the like. For injection, the pharmaceutical composition may be formulated in the form of unit dosage ampoules or in multiple-dosage forms. In addition, the pharmaceutical composition may be formulated into solutions, suspensions, tablets, capsules, sustained-release preparations, or the like.

Meanwhile, examples of carriers, diluents or excipients suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. In addition, a filler, an anti-coagulant, a lubricant, a wetting agent, a fragrance, an emulsifier, a preservative, and the like may further be contained.

The routes of administration of the pharmaceutical composition of the present invention include oral, intravenous, intramuscular, intraarterial, intramedullary, intradural, intracardiac, transdermal, subcutaneous, intraperitoneal, intranasal, intestinal, topical, sublingual, or rectal routes, without being limited thereto. Oral or parenteral administration is preferred.

In the present invention, the "parenteral" includes subcutaneous, transdermal, intravenous, intramuscular, intra-articular, intra-synovial, intrasternal, intradural, intralesional and intra-cranial injection or infusion techniques. The pharmaceutical composition of the present invention may also be formulated as suppositories for intrarectal administration.

The pharmaceutical composition of the present invention may vary depending on a variety of factors, including the activity of a specific compound used, the patient's age, body weight, general health status, sex and diet, the time of administration, the route of administration, the rate of excretion, drug combination, and the severity of a specific disease to be prevented or treated. The dosage of the pharmaceutical composition may vary depending on the patient's condition and body weight, the severity of disease, the drug form, and the route and duration of administration, but may be appropriately selected by those skilled in the art, and the pharmaceutical composition may be administered at a dose of 0.0001 to 50 mg/kg/day or 0.001 to 50 mg/kg/day. This administration may be done once a day or several times a day. The dose is not intended to limit the scope of the present invention in any way. The pharmaceutical composition according to the present invention may be formulated in the form of pills, sugar-coated tablets, capsules, liquids, gels, syrups, slurries, or suspensions.

### 10. Method for treatment

Another embodiment of the present invention is directed to a method for a method for preventing or treating cancer comprising a step of administering, to a subject, the antibody or antigen-binding fragment or the antibody-drug conjugate (ADC) according to the present invention.

In the present invention, the term "cancer" indicates or refers to a physiological condition typically characterized by unregulated cell growth in mammals. Cancer to be prevented, ameliorated or treated in the present invention may be a solid tumor consisting of an agglomerate generated by abnormal cell growth in a solid organ, and may be, without limitation, gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, or lung cancer, depending on the location of the solid organ.

The antibody or antigen-binding fragment or antibody-drug conjugate of the present invention may bind specifically to an epitope comprising a polypeptide represented by the amino acid sequence of any one of SEQ ID NOs: 7 to 72, thereby inhibiting the function of regulatory T cells and maintaining or increasing the activity of effector T cells, thereby very effectively treating cancer.

As used herein, the term "subject" refers to a subject suspected of developing cancer, and the subject suspected of developing cancer means mammals, including humans, mice, and domestic animals, that have developed or is likely to develop the disease in question. However, the term includes, without limitation, any subject that may be treated with the antibody or antibody-drug conjugate of the present invention.

The method of the present invention may comprise administering a pharmaceutically effective amount of the antibody or the antibody-drug conjugate. The suitable total daily dose of the antibody or the antibody-drug conjugate may be determined by an attending physician or veterinarian within the scope of sound medical judgment, and the antibody or the antibody-drug conjugate may be administered once or several times. However, for the purposes of the present invention, a specific therapeutically effective amount for a particular patient is preferably applied differently depending on various factors, including the type and degree of reaction to be achieved, the specific composition, including whether other agents are used as the case may be, the patient's age, body weight, general health status, sex, and diet, the time of administration, the route of administration, the secretion rate of the composition, the duration of treatment, and drugs used simultaneously or in combination with the specific composition, and similar factors well known in the medical field.

Meanwhile, the method for preventing or treating cancer may be, without limitation, a combination therapy that further comprises administering a compound or substance having therapeutic activity against one or more cancer diseases.

In the present invention, the "combination" should be understood to refer to simultaneous, individual, or sequential administration. When the administration is done in a sequential or individual manner, the second component should be administered at intervals such that beneficial effects of the combination are not lost.

In the present invention, the dosage of the antibody or antibody-drug conjugate may be about 0.0001 µg to 500 mg per kg of patient's body weight, without being limited thereto.

### Advantageous Effects

The antibody or antigen-binding fragment that binds specifically to the epitope of Lrig-1 protein according to the present invention may bind specifically to the epitope of the present invention, which is present on regulatory T cells, thereby inhibiting the function of the regulatory T cells and maintaining or increasing the activity of effector T cells. Thus, it may be very efficiently used for the prevention, amelioration or treatment of cancer.

### Brief Description of Drawings

FIG. 1 shows the structure of Lrig-1 protein according to one embodiment of the present invention.
FIG. 2 shows the structure of Lrig-1 protein according to one embodiment of the present invention.
FIG. 3 shows the expression level of Lrig-1 mRNA measured according to one example of the present invention.
FIG. 4 shows the expression level of Lrig-1 mRNA measured according to one example of the present invention.
FIG. 5 shows the expression level of Lrig-1 mRNA measured according to one example of the present invention.
FIG. 6 shows the expression levels of Lrig-1, Lrig-2, and Lrig-3 mRNAs measured according to one example of the present invention.
FIG. 7 shows the results of comparing the expression levels of Lrig-1 protein in regulatory T cells and non-regulatory T cells, measured according to one example of the present invention.
FIG. 8 shows expression of Lrig-1 protein on the surfaces of regulatory T cells, measured according to one example of the present invention.
FIG. 9 shows the results of analyzing the binding affinities of antibodies (GTC210-01, GTC210-02, GTC210-03, GTC210-04, GTC110-01, GTC110-02, GTC110-03 and GTC110-04) for Lrig-1 protein in one example of the present invention.
FIG. 10 shows the results of analyzing the mechanisms of regulating Lrig-1 protein-induced Stat3 phosphorylation in regulatory T cells by Lrig-1 protein-specific monoclonal antibodies (GTC210-01, GTC210-02, GTC210-03, GTC210-04, GTC110-01, GTC110-02, GTC110-03 and GTC110-04) in one example of the present invention.
FIG. 11 shows the experimental design of cancer treatment using Lrig-1 protein-specific monoclonal antibodies in one example of the present invention.
FIG. 12 shows cancer therapeutic effects obtained using Lrig-1 protein-specific monoclonal antibodies (GTC110-01, GTC110-02, GTC110-03 and GTC110-04) in one example of the present invention.
FIGS. 13 to 15 show conditions for mass spectrometry according to one example of the present invention.
FIGS. 16 and 17 show the results of covalent labeling mass spectrometry (MS) performed using chymotrypsin for the extracellular domain of Lirg-1 protein in one example of the present invention.
FIGS. 18 and 19 show the results of covalent labeling mass spectrometry (MS) performed using trypsin for the extracellular domain of Lirg-1 protein in one example of the present invention.
FIGS. 20 and 21 show the results of covalent labeling mass spectrometry (MS) performed using Asp-N+Lys-C for the extracellular domain of Lirg-1 protein in one example of the present invention.
FIG. 22 shows the results of crosslinking mass spectrometry (MS) performed using chymotrypsin for the extracellular domain of Lirg-1 protein in one example of the present invention.
FIG. 23 shows the results of crosslinking mass spectrometry (MS) performed using trypsin for the extracellular domain of Lirg-1 protein in one example of the present invention.
FIG. 24 shows the results of crosslinking mass spectrometry (MS) performed using Asp-N+Lys-C for the extracellular domain of Lirg-1 protein in one example of the present invention.
FIG. 25 shows an epitope region of the extracellular domain of Lrig-1 protein, to which a GTC 110-04 antibody specifically binds, in an example of the present invention.

### Best Mode

The present invention is directed to an epitope of Lrig-1 (leucine-rich and immunoglobulin-like domains 1) protein or an antibody or antigen-binding fragment that specifically binds to the epitope.

In one embodiment of the present invention, the extracellular domain of the Lrig-1 protein may be represented by SEQ ID NO: 5, which corresponds to amino acid positions 35 to 794 in the amino acid sequence of the human-derived Lrig-1 protein, without being limited thereto.

In another example of the present invention, the extracellular domain of the Lrig-1 protein may be represented by SEQ ID NO: 6, which corresponds to amino acid positions 35 to 794 in the amino acid sequence of the mouse-derived Lrig-1 protein, without being limited thereto.

### Mode for Invention

Hereinafter, the present invention will be described in more detail with reference to examples. These examples are only for illustrating the present invention in more detail, and it will be obvious to those of ordinary skill in the art that the scope of the present invention according to the subject matter of the present invention is not limited by these examples.

### Examples

### [Preparation Example 1] T cell subset culture

In order to examine whether the Lrig-1 protein is expressed only in regulatory T cells (Treg) cells, Th0, Th1, Th2, Th17 and iTreg, which are T cell subsets, were prepared. The iTreg refers to cells whose differentiation has been artificially induced in a medium having the following composition, unlike nTreg which has been naturally isolated.

The T cell subsets were obtained by isolating naive T cells from mouse spleens, and then inducing the isolated cells to differentiate into each T cell subset by 72 hours of culture in RPMI1640 (Invitrogen Gibco, Grand Island, NY) nutrient medium, which contained 10% fetal bovine serum (FBS; hyclone, logan, UT) and further contained the components shown in Table 1 below, in an incubator at 37°C under 5% CO₂.

**[Table 1]**

| Differentiated cells | Composition |
|---|---|
| Th0 | anti-CD3 and anti-CD28 |
| Th1 | IL-12 and anti-IL-4 antibody |
| Th2 | IL-4 and anti-IFNβ |
| Th17 | IL-6, TGFβ anti-IFNβ and anti-IL-4 |
| iTreg | IL-2 and TGFβ |

### [Example 1] Structural analysis of Lrig-1

The three-dimensional steric structure of the extracellular domain of Lrig-1 protein was predicted to produce an antibody specific for the Lrig-1 protein, a protein present on the surfaces of regulatory T cells.

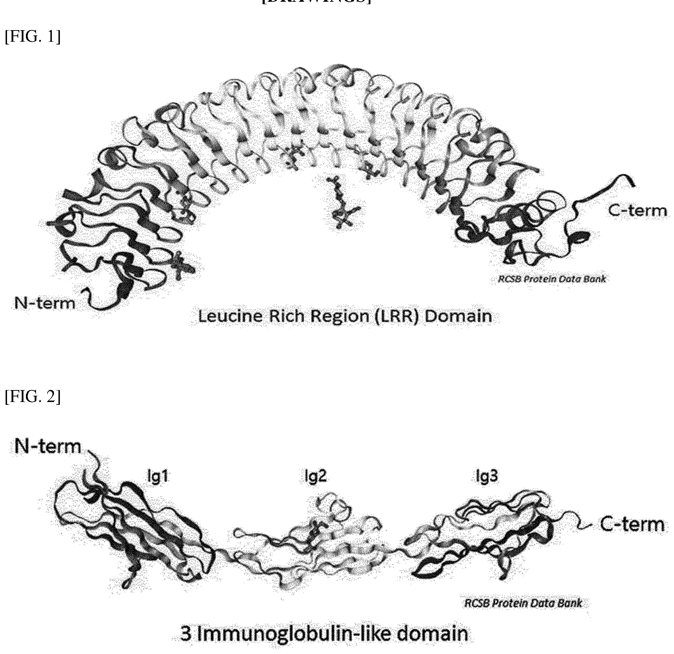

First, in order to predict the epitope by analyzing the three-dimensional steric structure of the extracellular domain (ECD) of the Lrig-1 protein, tools of Uniprot (http://www.uniprot.org) and RCSB Protein Data Bank (http://www.rcsb.org/pdb) were used to predict the three-dimensional steric structure, and the results are shown in FIGS. 1 and 2.

As shown in FIG. 1, a total of 15 leucine-rich regions (LRR1 to LRR15) were present in the Lrig-LRR domain (amino acid positions 41 to 494) in the extracellular domain of the Lrig-1 protein. Each of the LRR domains consisted of 23 to 27 amino acids and contained 3 to 5 leucine residues.

In addition, as shown in FIG. 2, three immunoglobulin-like domains were present at positions 494 to 781 in the amino acid sequence of the Lrig-1 protein in the extracellular domain of the Lrig-1 protein.

### [Example 2] Confirmation of specific expression of Lrig-1 mRNA in regulatory T cells

Verification was made as to whether the Lrig-1 protein could act as a biomarker specific for regulatory T cells.

For the verification, CD4⁺ T cells were isolated from mouse spleens by magnet-activated cell sorting (MACS) using CD4 beads. Subsequently, regulatory T (CD4⁺CD25⁺T) cells and non-regulatory T (CD4⁺CD25⁻T) cells were isolated by fluorescence-activated cell sorting (FACS) using CD25 antibody. For the isolated cells and the cells differentiated in Preparation Example 1, mRNA was extracted using Trizol, and then genomic DNA was removed from genomic RNA using a gDNA extraction kit (Qiagen) according to the protocol provided by the manufacturer. The gDNA-removed mRNA was synthesized into cDNA through the BDsprint cDNA Synthesis Kit (Clonetech).

Real-time polymerase chain reaction (RT PCR) was performed to quantitatively analyze the expression level of Lrig-1 mRNA in the cDNA.

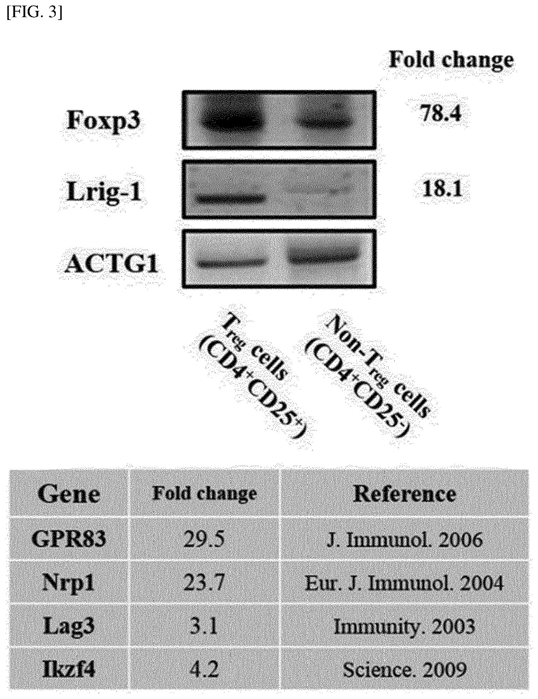

The real-time polymerase chain reaction was performed with primers shown in Table 2 below using SYBR Green (Molecular Probes) according to the protocol provided by the manufacturer for 40 cycles, each consisting of 95°C for 3 minutes, 61°C for 15 seconds, and 72°C for 30 seconds. The relative gene expression level was calculated using the ΔCT method and normalized using HPRT. The results are shown in FIGS. 3 to 6.

**[Table 2]**

| Primer | Sequence |
|---|---|
| Mouse Lrig-1 | Forward 5' - GAC GGA ATT CAG TGA GGA GAA CCT - 3' |
| | Reverse 5' - CAA CTG GTA GTG GCA GCT TGT AGG - 3' |
| Mouse Lrig-2 | forward 5' - TCA CAA GGA ACA TTG TCT GAA CCA- 3' |
| | reverse 5' - GCC TGA TCT AAC ACA TCC TCC TCA- 3' |
| Mouse Lrig-3 | forward 5' - CAG CAC CTT GAG CTG AAC AGA AAC - 3' |
| | reverse 5' - CCA GCC TTT GGT AAT CTC GGT TAG - 3' |
| Mouse FOXP3 | forward 5' - CTT TCA CCT ATC CCA CCC TTA TCC - 3' |
| | reverse 5' - ATT CAT CTA CGG TCC ACA CTG CTC - 3' |
| ACTG1 | forward 5' - GGC GTC ATG GTG GGC ATG GG - 3' |
| | reverse 5' - ATG GCG TGG GGA AGG GCG TA - 3' |

As shown in FIG. 3, it can be seen that the expression level of Lrig-1 in the regulatory T (CD4⁺CD25⁺ T) cells was 18.1 times higher than that in the non-regulatory T (CD4⁺CD2S⁻ T) cells. This was an about 10 times higher expression level than those of Lag3 and Ikzf4, which are previously known markers for regulatory T cells.

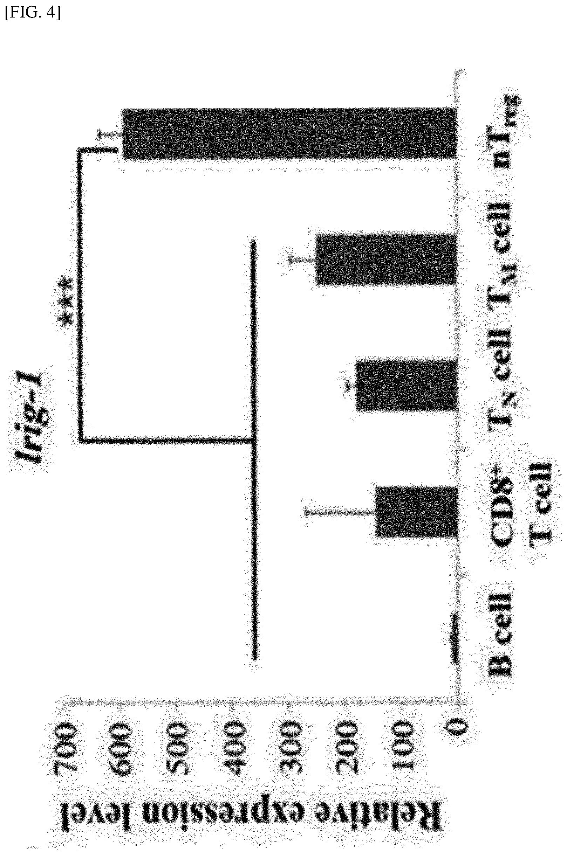

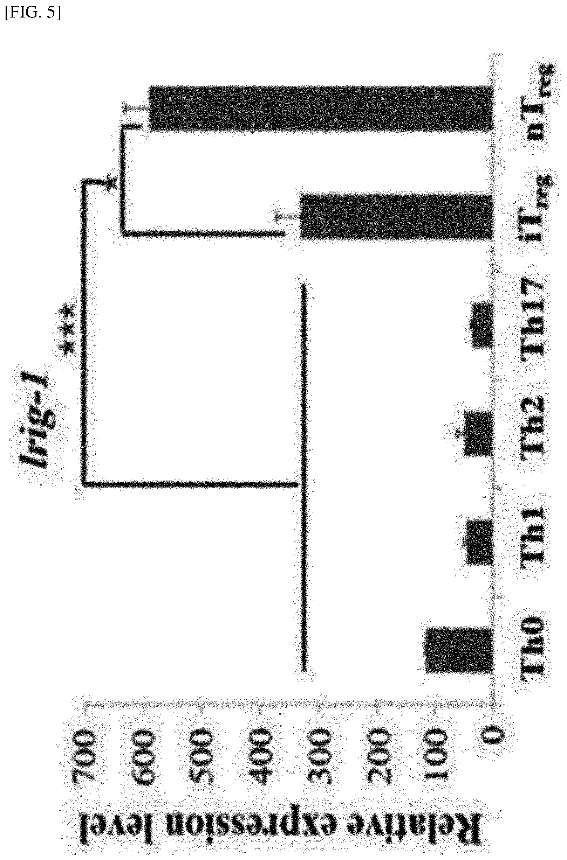

In addition, as shown in FIGS. 4 and 5, the expression level of Lrig-1 mRNA was remarkably high in the regulatory T cells compared to other types of immune cells, and in particular, was remarkably high in naturally isolated regulatory T cells (nTreg) compared to induced regulatory T cells (iTreg).

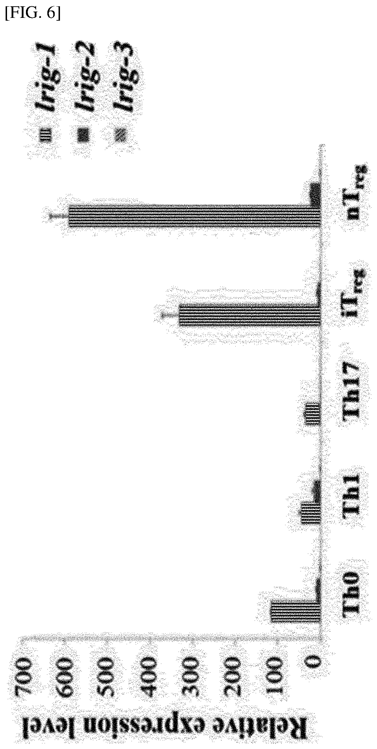

In addition, as shown in FIG. 6, the expression level of Lrig-1 was the highest among the expression levels of Lrig-1, Lrig-2 and Lrig-3 belonging to the Lrig family.

From the above results, it can be seen that the Lrig-1 protein according to the present invention is expressed specifically in regulatory T cells, in particular, naturally occurring regulatory T cells.

### [Example 3] Analysis of specific expression of Lrig-1 protein in regulatory T cells

Analysis was performed as to whether the Lrig-1 protein expressed from Lrig-1 mRNA was specifically expressed only in regulatory T cells.

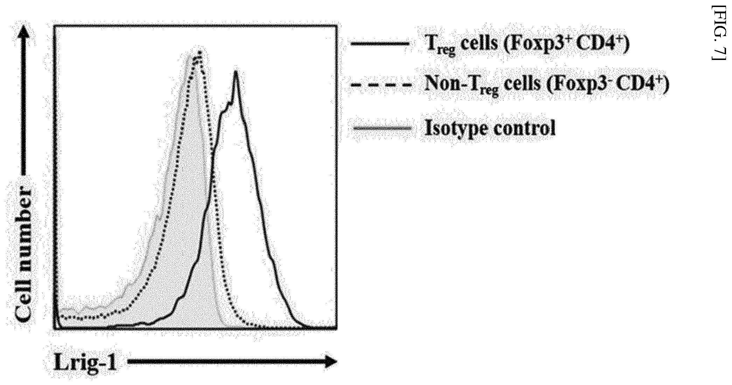

Using FOXP3-RFP-knocked-in mice having red fluorescence protein (RFP) conjugated to the FOXP3 promoter, which is a transcription factor specific for regulatory T cells, CD4⁺ T cells were isolated from the mouse spleens by magnet-activated cell sorting (MACS) using CD4 beads. Subsequently, regulatory T (CD4⁺RFP⁺ T) cells and non-regulatory T (CD4⁺RFP⁻ T) cells were isolated by fluorescence-activated cell sorting (FACS) using RFP protein. The isolated cells were stained with the purchased Lrig-1 antibody, a negative control was stained with an isotype antibody, and the expression levels of Lrig-1 were measured by fluorescence-activated cell sorting. The results are shown in FIG. 7.

As shown in FIG. 7, the non-regulatory T cells indicated by the dotted line showed almost the same Lrig-1 expression level as the negative control, whereas there were a large number of cells with a high Lrig-1 expression level in the regulatory T cells.

From the above results, it can be seen that the Lrig-1 protein according to the present invention is expressed specifically in regulatory T cells.

### [Example 41 Confirmation of specific expression of Lrig-1 protein on surface of regulatory T cells

Even when the Lrig-1 protein is expressed on the surfaces of regulatory T cells so that it becomes a target of cell therapy, the target therapy can be more effectively achieved. Accordingly, analysis was performed as to whether the Lrig-1 protein was expressed on the surfaces of the regulatory T cells.

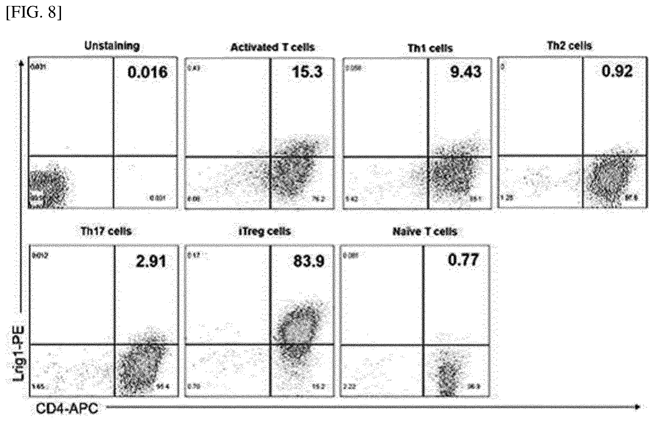

Each of the differentiated T cell subsets of Preparation Example 1 was stained with anti-CD4-APC and anti-Lrig-1-PE antibodies, and the expression levels of Lrig-1 on the surface of each subset was measured using fluorescence-activated cell sorting (FACS). The results are shown in FIG. 8.

As shown in FIG. 8, the expression levels of Lrig-1 in the activated T cells, Th1 cells, Th2 cells, Th17 cells, and naive T cells were 0.77 to 15.3, whereas the expression level of Lrig-1 in the differentiation-induced T cells (iTreg cells) was as high as 83.9.

From the above results, it can be seen that the Lrig-1 protein according to the present invention is not only expressed specifically in regulatory T (Treg) cells, but also expressed at a higher level, particularly on the surfaces of the Treg cells.

### [Production Examples 1 to 81 Production of monoclonal antibodies specific for Lrig-1 protein

Antibodies specific for the Lrig-1 protein according to the present invention were produced. The produced antibodies were antibodies capable of binding to any site on the Lrig-1 protein without being specified to a certain epitope.

In order to produce the antibodies, cells expressing the Lrig-1 protein were produced. More specifically, a DNA fragment corresponding to SEQ ID NO: 2 and pcDNA (hygro) were cleaved with a cleavage enzyme, incubated at 37°C, and ligated to each other to produce pcDNA into which the DNA sequence of the Lrig-1 protein has been inserted. The produced pcDNA into which SEQ ID NO: 2 has been inserted was introduced into L cells through transfection so that the Lrig-1 protein could be expressed on the surfaces of the L cells.

Light and heavy chain amino acid sequences capable of binding to the Lrig-1 protein expressed on the cell surfaces were selected from the Human scFv library, thereby selecting a total of eight heavy and light chains.

Monoclonal antibodies were produced by fusing the selected heavy and light chain amino acid sequences with the mlgG2a Fc region or the human IgG1 Fc region. The sequences of the monoclonal antibodies are shown in Table 3 below.

**[Table 3]**

| | Clone | Position | Amino acid sequence | Sequence information |
|---|---|---|---|---|
| Production Example 1 | GTC210-01 clone | Heavy chain | EVQLLESGGG LVQPGGSLRL SCAASGFTFS GYDMSWVRQA PGKGLEWVSL IYPDSGNKYY 60 ADSVKGRFTI SRDNSKNTLY LQMNSLRAED | SEQ ID NO: 147 |
| | | | TAVYYCARDA GLSWAGAFDY WGQGTLVTVS 120 SASTTAPSVY PLAPVCGDTT GSSVTLGCLV KGYFPEPVTL TWNSGSLSSG VHTFPAVLQS 180 DLYTLSSSVT VTSSTWPSQS ITCNVAHPAS STKVDKKIEP RGPTIKPCPP CKCPAPNLLG 240 GPSVFIFPPK IKDVLMISLS PIVTCVVVDV SEDDPDVQIS WFVNNVEVHT AQTQTHREDY 300 NSTLRVVSAL PIQHQDWMSG KEFKCKVNNK DLPAPIERTI SKPKGSVRAP QVYVLPPPEE 360 EMTKKQVTLT CMVTDFMPED IYVEWTNNGK TELNYKNTEP VLDSDGSYFM YSKLRVEKKN 420 WVERNSYSCS VVHEGLHNHH TTKSFSRTPG K | |
| | | Light chain | QSVLTQPPSA SGTPGQRVTI SCSGSSSNIG SNYVTWYQQL PGTAPKLLIY SDSHRPSGVP 60 DRFSGSKSGT SASLAISGLQ SEDEADYYCG SWDYSLSAYV FGGGTKLTVL RTVAAPTVSI 120 FPPSSEQLTS GGASVVCFLN NFYPKDINVK WKIDGSERQN GVLNSWTDQD SKDSTYSMSS 180 TLTLTKDEYE RHNSYTCEAT HKTSTSPIVK SFNRNEC | SEQ ID NO: 148 |
| Production Example 2 | GTC210-02 clone | Heavy chain | EVQLLESGGG LVQPGGSLRL SCAASGFTFS NYYMSWVRQA PGKGLEWVSG ISPGDSSTYY 60 ADSVKGRFTI SRDNSKNTLY LQMNSLRAED | SEQ ID NO: 149 |
| | | | TAVYYCAKGL YSNPNEPFDY WGQGTLVTVS 120 SASTTAPSVY PLAPVCGDTT GSSVTLGCLV KGYFPEPVTL TWNSGSLSSG VHTFPAVLQS 180 DLYTLSSSVT VTSSTWPSQS ITCNVAHPAS STKVDKKIEP RGPTIKPCPP CKCPAPNLLG 240 GPSVFIFPPK IKDVLMISLS PIVTCVVVDV SEDDPDVQIS WFVNNVEVHT AQTQTHREDY 300 NSTLRVVSAL PIQHQDWMSG KEFKCKVNNK DLPAPIERTI SKPKGSVRAP QVYVLPPPEE 360 EMTKKQVTLT CMVTDFMPED IYVEWTNNGK TELNYKNTEP VLDSDGSYFM YSKLRVEKKN 420 WVERNSYSCS VVHEGLHNHH TTKSFSRTPG K | |
| | | Light chain | QSVLTQPPSA SGTPGQRVTI SCTGSSSNIG SNYVSWYQQL PGTAPKLLIY DDSQRPSGVP 60 DRFSGSKSGT SASLAISGLR SEDEADYYCG TWDYSLNGYV FGGGTKLTVL RTVAAPTVSI 120 FPPSSEQLTS GGASVVCFLN NFYPKDINVK WKIDGSERQN GVLNSWTDQD SKDSTYSMSS 180 TLTLTKDEYE RHNSYTCEAT HKTSTSPIVK SFNRNEC | SEQ ID NO: 150 |
| Production Example 3 | GTC210-03 clone | Heavy chain | EVQLLESGGG LVQPGGSLRL SCAASGFTFS SYDMSWVRQA PGKGLEWVSG ISPDGSNIYY 60 ADSVKGRFTI SRDNSKNTLY LQMNSLRAED | SEQ ID NO: 151 |
| | | | TAVYYCAKVG LRCRYEACSY AYGMDVWGQG 120 TLVTVSSAST TAPSVYPLAP VCGDTTGSSV TLGCLVKGYF PEPVTLTWNS GSLSSGVHTF 180 PAVLQSDLYT LSSSVTVTSS TWPSQSITCN VAHPASSTKV DKKIEPRGPT IKPCPPCKCP 240 APNLLGGPSV FIFPPKIKDV LMISLSPIVT CVVVDVSEDD PDVQISWFVN NVEVHTAQTQ 300 THREDYNSTL RVVSALPIQH QDWMSGKEFK CKVNNKDLPA PIERTISKPK GSVRAPQVYV 360 LPPPEEEMTK KQVTLTCMVT DFMPEDIYVE WTNNGKTELN YKNTEPVLDS DGSYFMYSKL 420 RVEKKNWVER NSYSCSVVHE GLHNHHTTKS FSRTPGK | |
| | | Light chain | QSVLTQPPSA SGTPGQRVTI SCSGSSSNIG SNYVSWYQQL PGTAPKLLIY SDSHRPSGVP 60 DRFSGSKSGT SASLAISGLR SEDEADYYCA TWDSSLNGYV FGGGTKLTVL RTVAAPTVSI 120 FPPSSEQLTS GGASVVCFLN NFYPKDINVK WKIDGSERQN GVLNSWTDQD SKDSTYSMSS 180 TLTLTKDEYE RHNSYTCEAT HKTSTSPIVK SFNRNEC | SEQ ID NO: 152 |
| Production | GTC 210- | Heavy chain | EVQLLESGGG LVQPGGSLRL SCAASGFTFS | SEQ ID NO: |
| Example 4 | 04 clone | | NYDMSWVRQA PGKGLEWVSS ISPSSGSIYY 60 ADSVKGRFTI SRDNSKNTLY LQMNSLRAED TAVYYCAKDL DAFWRPSFDY WGQGTLVTVS 120 STTAPSVYPL APVCGDTTGS SVTLGCLVKG YFPEPVTLTW NSGSLSSGVH TFPAVLQSDL 180 YTLSSSVTVT SSTWPSQSIT CNVAHPASST KVDKKIEPRG PTIKPCPPCK CPAPNLLGGP 240 SVFIFPPKIK DVLMISLSPI VTCVVVDVSE DDPDVQISWF VNNVEVHTAQ TQTHREDYNS 300 TLRVVSALPI QHQDWMSGKE FKCKVNNKDL PAPIERTISK PKGSVRAPQV YVLPPPEEEM 360 TKKQVTLTCM VTDFMPEDIY VEWTNNGKTE LNYKNTEPVL DSDGSYFMYS KLRVEKKNWV 420 ERNSYSCSVV HEGLHNHHTT KSFSRTPGK | 153 |
| | | Light chain | QSVLTQPPSA SGTPGQRVTI SCTGSSSNIG NNNVNWYQQL PGTAPKLLIY SDSHRPSGVP 60 DRFSGSKSGT SASLAISGLR SEDEADYYCG SWDDSLSAYV FGGGTKLTVL RTVAAPTVSI 120 FPPSSEQLTS GGASVVCFLN NFYPKDINVK WKIDGSERQN GVLNSWTDQD SKDSTYSMSS 180 TLTLTKDEYE RHNSYTCEAT HKTSTSPIVK SFNRNEC | SEQ ID NO: 154 |
| Production Example 5 | GTC110-01 clone | Heavy chain | EVQLLESGGG LVQPGGSLRL SCAASGFTFS NYDMSWVRQA PGKGLEWVSS ISPSSGSIYY 60 ADSVKGRFTI SRDNSKNTLY LQMNSLRAED TAVYYCAKDL DAFWRPSFDY WGQGTLVTVS 120 SASTTAPSVY PLAPVCGDTT GSSVTLGCLV KGYFPEPVTL TWNSGSLSSG VHTFPAVLQS 180 DLYTLSSSVT VTSSTWPSQS ITCNVAHPAS STKVDKKIEP RGPTIKPCPP CKCPAPNLLG 240 GPSVFIFPPK IKDVLMISLS PIVTCVVVDV SEDDPDVQIS WFVNNVEVHT AQTQTHREDY 300 NSTLRVVSAL PIQHQDWMSG KEFKCKVNNK DLPAPIERTI SKPKGSVRAP QVYVLPPPEE 360 EMTKKQVTLT CMVTDFMPED IYVEWTNNGK TELNYKNTEP VLDSDGSYFM YSKLRVEKKN 420 WVERNSYSCS VVHEGLHNHH TTKSFSRTPG K | SEQ ID NO: 155 |
| | | Light chain | QSVLTQPPSA SGTPGQRVTI SCTGSSSNIG NNSVTWYQQL PGTAPKLLIY ADNNRPSGVP 60 DRFSGSKSGT SASLAISGLR SEDEADYYCA AWDSSLSAYV FGGGTKLTVL RTVAAPTVSI 120 FPPSSEQLTS GGASVVCFLN NFYPKDINVK WKIDGSERQN GVLNSWTDQD SKDSTYSMSS 180 TLTLTKDEYE RHNSYTCEAT HKTSTSPIVK SFNRNEC | SEQ ID NO: 156 |
| Production Example 6 | GTC110-02 clone | Heavy chain | EVQLLESGGG LVQPGGSLRL SCAASGFTFS DYYMSWVRQA PGKGLEWVSG ISHDSGSKYY 60 ADSVKGRFTI SRDNSKNTLY LQMNSLRAED TAVYYCARHW TTFDYWGQGT LVTVSSASTT 120 APSVYPLAPV CGDTTGSSVT LGCLVKGYFP EPVTLTWNSG SLSSGVHTFP AVLQSDLYTL 180 SSSVTVTSST WPSQSITCNV AHPASSTKVD KKIEPRGPTI KPCPPCKCPA PNLLGGPSVF 240 IFPPKIKDVL MISLSPIVTC VVVDVSEDDP DVQISWFVNN VEVHTAQTQT HREDYNSTLR 300 VVSALPIQHQ DWMSGKEFKC KVNNKDLPAP IERTISKPKG SVRAPQVYVL PPPEEEMTKK 360 QVTLTCMVTD FMPEDIYVEW TNNGKTELNY KNTEPVLDSD GSYFMYSKLR VEKKNWVERN 420 SYSCSVVHEG LHNHHTTKSF SRTPGK | SEQ ID NO: 157 |
| | | Light chain | QSVLTQPPSA SGTPGQRVTI SCSGSSSNIG SNNVTWYQQL PGTAPKLLIY ANSNRPSGVP 60 DRFSGSKSGT SASLAISGLR SEDEADYYCG AWDYSLSAYV FGGGTKLTVL RTVAAPTVSI 120 FPPSSEQLTS GGASVVCFLN NFYPKDINVK WKIDGSERQN GVLNSWTDQD SKDSTYSMSS 180 | SEQ ID NO: 158 |
| | | | TLTLTKDEYE RHNSYTCEAT HKTSTSPIVK SFNRNEC | |
| Production Example 7 | GTC110-03 clone | Heavy chain | EVQLLESGGG LVQPGGSLRL SCAASGFTFS NYAMSWVRQA PGKGLEWVSA IYPGGGSIYY 60 ADSVKGRFTI SRDNSKNTLY LQMNSLRAED TAVYYCARDI LPCPWGRCYY DYAMDVWGQG 120 TLVTVSSAST TAPSVYPLAP VCGDTTGSSV TLGCLVKGYF PEPVTLTWNS GSLSSGVHTF 180 PAVLQSDLYT LSSSVTVTSS TWPSQSITCN VAHPASSTKV DKKIEPRGPT IKPCPPCKCP 240 APNLLGGPSV FIFPPKIKDV LMISLSPIVT CVVVDVSEDD PDVQISWFVN NVEVHTAQTQ 300 THREDYNSTL RVVSALPIQH QDWMSGKEFK CKVNNKDLPA PIERTISKPK GSVRAPQVYV 360 LPPPEEEMTK KQVTLTCMVT DFMPEDIYVE WTNNGKTELN YKNTEPVLDS DGSYFMYSKL 420 RVEKKNWVER NSYSCSVVHE GLHNHHTTKS FSRTPGK | SEQ ID NO: 159 |
| | | Light chain | QSVLTQPPSA SGTPGQRVTI SCSDSSSNIG SNTVSWYQQL PGTAPKLLIY ADNNRPSGVP 60 DRFSGSKSGT SASLAISGLR SEDEADYYCG TWDYSLSGYV FGGGTKLTVL RTVAAPTVSI 120 FPPSSEQLTS GGASVVCFLN NFYPKDINVK | SEQ ID NO: 160 |
| | | | WKIDGSERQN GVLNSWTDQD SKDSTYSMSS 180 TLTLTKDEYE RHNSYTCEAT HKTSTSPIVK SFNRNEC | |
| Production Example 8 | GTC110-04 clone | Heavy chain\ | EVQLLESGGG LVQPGGSLRL SCAASGFTFS NYAMSWVRQA PGKGLEWVSV ISHGGGSTYY 60 ADSVKGRFTI SRDNSKNTLY LQMNSLRAED TAVYYCARVI SNCHLGVCYY SNGMDVWGQG 120 TLVTVSSAST TAPSVYPLAP VCGDTTGSSV TLGCLVKGYF PEPVTLTWNS GSLSSGVHTF 180 PAVLQSDLYT LSSSVTVTSS TWPSQSITCN VAHPASSTKV DKKIEPRGPT IKPCPPCKCP 240 APNLLGGPSV FIFPPKIKDV LMISLSPIVT CVVVDVSEDD PDVQISWFVN NVEVHTAQTQ 300 THREDYNSTL RVVSALPIQH QDWMSGKEFK CKVNNKDLPA PIERTISKPK GSVRAPQVYV 360 LPPPEEEMTK KQVTLTCMVT DFMPEDIYVE WTNNGKTELN YKNTEPVLDS DGSYFMYSKL 420 RVEKKNWVER NSYSCSVVHE GLHNHHTTKS FSRTPGK | SEQ ID NO: 161 |
| | | Light chain | QSVLTQPPSA SGTPGQRVTI SCSGSSSNIG NNDVYWYQQL PGTAPKLLIY SDSQRPSGVP 60 DRFSGSKSGT SASLAISGLR SEDEADYYCG | SEQ ID NO: 162 |
| | | | TWDYSLSGYV FGGGTKLTVL RTVAAPTVSI 120 FPPSSEQLTS GGASVVCFLN NFYPKDINVK WKIDGSERQN GVLNSWTDQD SKDSTYSMSS 180 TLTLTKDEYE RHNSYTCEAT HKTSTSPIVK SFNRNEC | |
| Production Example 9 | GTC110-04 humanized antibody | Heavy chain | EVQLLESGGG LVQPGGSLRL SCAASGFTFS NYAMSWVRQA PGKGLEWVSV ISHGGGSTYY 60 ADSVKGRFTI SRDNSKNTLY LQMNSLRAED TAVYYCARVI SNCHLGVCYY SNGMDVWGQG 120 TLVTVSSAST KGPSVFPLAP SSKSTSGGTA ALGCLVKDYF PEPVTVSWNS GALTSGVHTF 180 PAVLQSSGLY SLSSVVTVPS SSLGTQTYIC NVNHKPSNTK VDKKVEPKSC DKTHTCPPCP 240 APELLGGPSV FLFPPKPKDT LMISRTPEVT CVVVDVSHED PEVKFNWYVD GVEVHNAKTK 300 PREEQYNSTY RVVSVLTVLH QDWLNGKEYK CKVSNKALPA PIEKTISKAK GQPREPQVYT 360 LPPSREEMTK NQVSLTCLVK GFYPSDIAVE WESNGQPENN YKTTPPVLDS DGSFFLYSKL 420 TVDKSRWQQG NVFSCSVMHE ALHNHYTQKS LSLSPGKY | SEQ ID NO: 163 |
| | | Light chain | QSVLTQPPSA SGTPGQRVTI SCSGSSSNIG NNDVYWYQQL PGTAPKLLIY SDSQRPSGVP 60 | SEQ ID NO: 164 |
| | | | DRFSGSKSGT SASLAISGLR SEDEADYYCG TWDYSLSGYV FGGGTKLTVL RTVAAPSVFI 120 FPPSDEQLKS GTASVVCLLN NFYPREAKVQ WKVDNALQSG NSQESVTEQD SKDSTYSLSS 180 TLTLSKADYE KHKVYACEVT HQGLSSPVTK SFNRGEC | |

### [Example 5] Evaluation of binding affinities of antibodies according to the present invention for Lrig-1 protein

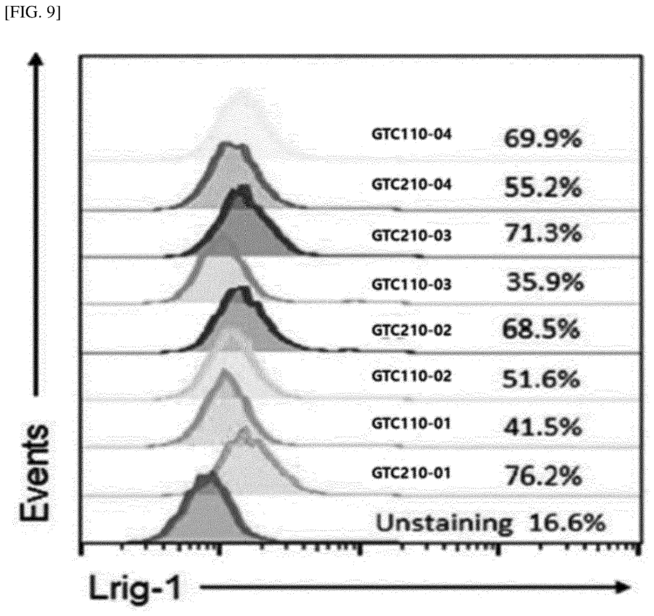

In order to examine whether the monoclonal antibodies according to the present invention, produced in Production Examples 1 to 8, well recognize Lrig-1, each of the antibodies of Production Examples 1 to 8 was bound to L cells that stably express Lrig-1, and then an eFlour 670-conjugated secondary antibody capable of recognizing mouse antibodies was added thereto. Next, the binding affinities of the monoclonal antibodies for the Lrig-1 protein were analyzed using FACS. The results are shown in FIG. 9.

As shown in FIG. 9, it could be confirmed that all the Lrig-1 protein-specific monoclonal antibodies according to the present invention effectively recognized and bound to the Lrig-1 protein present on the surfaces of the L cells.

### [Example 6] Regulation of signaling pathway in regulatory T cells by antibodies according to the present invention

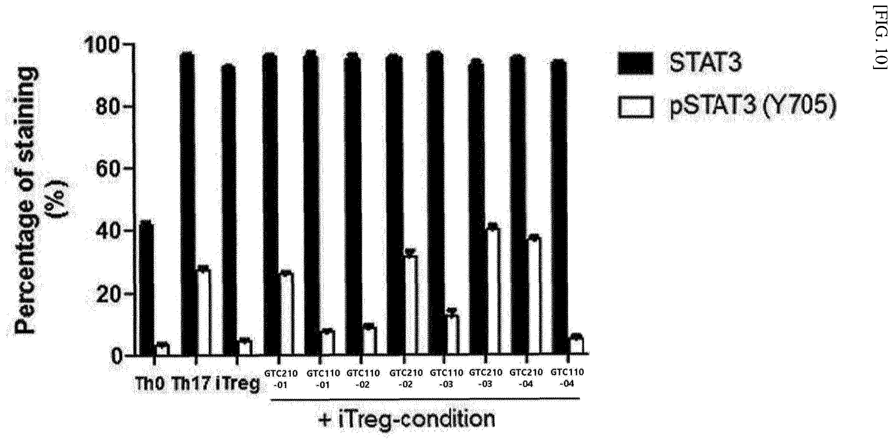

In order to analyze how the monoclonal antibodies according to the present invention, produced in Production Examples 1 to 8, affect the signaling pathway in regulatory T cells through the Lrig-1 protein, Lrig-1 present on the surfaces of the regulatory T cells was stimulated by treating the regulatory T cells with the antibodies of Production Examples 1 to 8, and then the tyrosine phosphorylation level of Stat3 protein present in the stimulated regulatory T cells was analyzed by phosphotyrosine immunoblot assay. The results are shown in FIG. 10.

As shown in FIG. 10, it could be confirmed that the Lrig-1 protein-specific monoclonal antibodies (GTC210-01, GTC210-02, GTC210-03 and GTC210-04) according to the present invention increased phosphorylation of Stat3 to the same level as that in Th17 cells. Meanwhile, it could be confirmed that the Lrig-1 protein-specific monoclonal antibodies (GTC110-01, GTC110-02, GTC110-03 and GTC110-04) according to the present invention continued to maintain and decrease the phosphorylation of Stat3 at the same level as that in iTreg cells.

### [Example 7] Cancer therapeutic effects of GTC110-01, GTC110-02, GTC110-03 and GTC110-04 antibodies

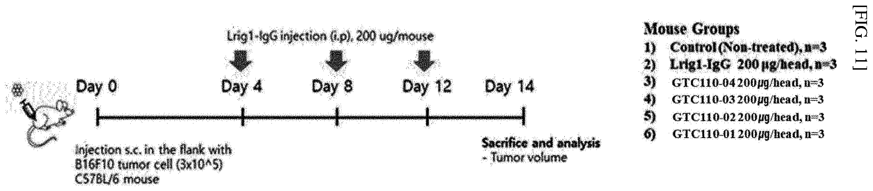

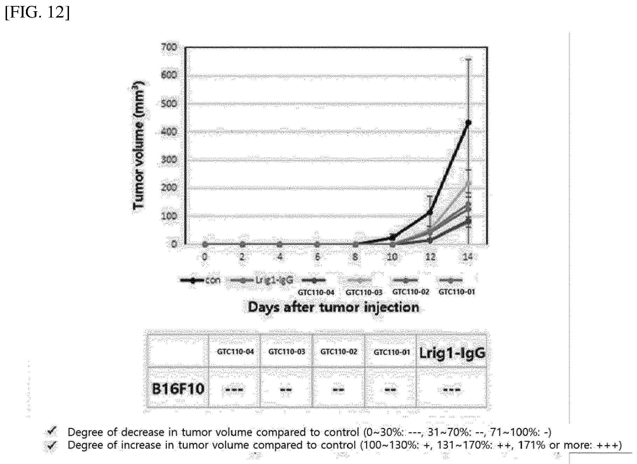

In order to evaluate the solid cancer therapeutic effects of the monoclonal antibodies (GTC110-01, GTC110-02, GTC110-03 and GTC110-04) according to the present invention, produced in Production Examples 5 to 8, as shown in FIG. 11, B16F10 melanoma cells were subcutaneously injected into the dorsal area of mice in an amount of 3 × 10⁵ cells, and then each of the antibodies of Production Examples 5 to 8 was intraperitoneally injected into the mice in an amount of 200 ug on days 4, 8, and 12. After transplantation of the melanoma cells, time-dependent changes in the tumor volume were measured, and the results of the measurement are shown in FIG. 12.

As shown in FIG. 12, it could be confirmed that the tumor volume remarkably decreased when the cells were treated with the Lrig-1 protein-specific monoclonal antibodies (GTC110-01, GTC110-02, GTC110-03 and GTC110-04) according to the present invention, compared to the negative control group which was not treated with the antibody.

Thereby, it can be seen that the Lrig-1 protein-specific monoclonal antibodies according to the present invention can inhibit the growth of various solid cancer cells, thereby effectively preventing, ameliorating or treating such cancers.

### [Example 8] Identification of epitope of Lrig-1 protein according to the present invention

In order to discover a conformational epitope, to which the GTC 110-04 antibody of Production Example 8 can bind, in the extracellular domain of the Lrig-1 protein, the following experiment was conducted.

### [8-1] Covalent labeling mass spectroscopy (MS) (CL-MS)

### [8-1-1] Epitope identification using chymotrypsin

An antigen-antibody conjugate was prepared by mixing the GTTC110-04 antibody of Preparation Example 8 and the extracellular domain (antigen) of Lrig-1 protein represented by SEQ ID NO: 4 at a ratio of 2: 1 and sufficiently reacting the mixture, and the non-antibody-conjugated extracellular domain (antigen) of Lrig-1 protein was also prepared. Next, DEPC (diethyl pyrocarbonate) was added to each of the samples so as not to exceed 1%, and then allowed to react sufficiently at 37°C for 1 minute, and the reaction was completed by adding imidazole. After completion of the reaction, methanol was added to each of the samples, and the supernatant was removed by centrifugation. Thereafter, each sample was resuspended in a solution containing 8 M urea and 0.1 M NaCl, and then chymotrypsin was added thereto so that the peptide bond could be broken.

Each of the samples in which the peptide bond was broken as described above was subjected to fluid chromatography (Vanquish^{™} Flex Quaternary UHPLC (Thermo SCIENTIFIC)) using a Hypersil GOLD^{™} (1.9 µm, 1 × 100 mm) column at 30°C under the conditions shown in Table 4 below.

**[Table 4]**

| Step | Total time (min) | Flow rate (µl/min) | Mobile phase A^{∗} (%) | Mobile phase B^{∗∗} (%) |
|---|---|---|---|---|
| 0 | 0.00 | 100.00 | 95.0 | 5.0 |
| 1 | 5.00 | 100.00 | 95.0 | 5.0 |
| 2 | 7.00 | 100.00 | 90.0 | 10.0 |
| 3 | 48.00 | 100.00 | 70.0 | 30.0 |
| 4 | 55.00 | 100.00 | 5.0 | 95.0 |
| 5 | 65.00 | 100.00 | 5.0 | 95.0 |
| 6 | 66.00 | 100.00 | 95.00 | 5.0 |
| 7 | 71.00 | 100.00 | 95.00 | 5.0 |
| ^{∗} mobile phase A (distilled water/0.2% formic acid) | | | | |
| ^{∗∗} mobile phase B (acetonitrile/0.2% formic acid) | | | | |

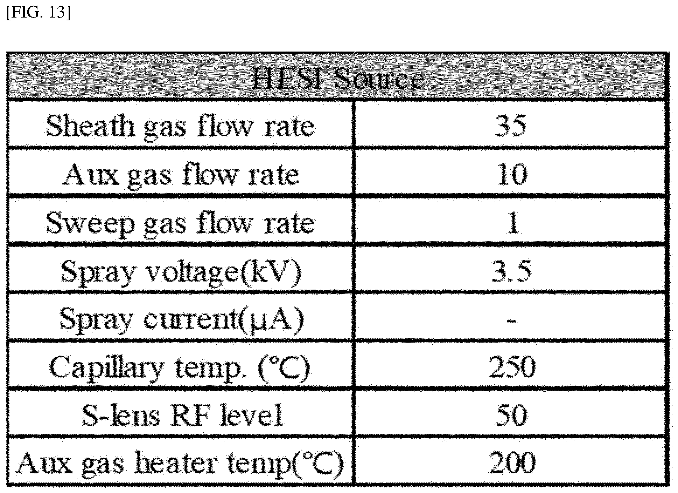

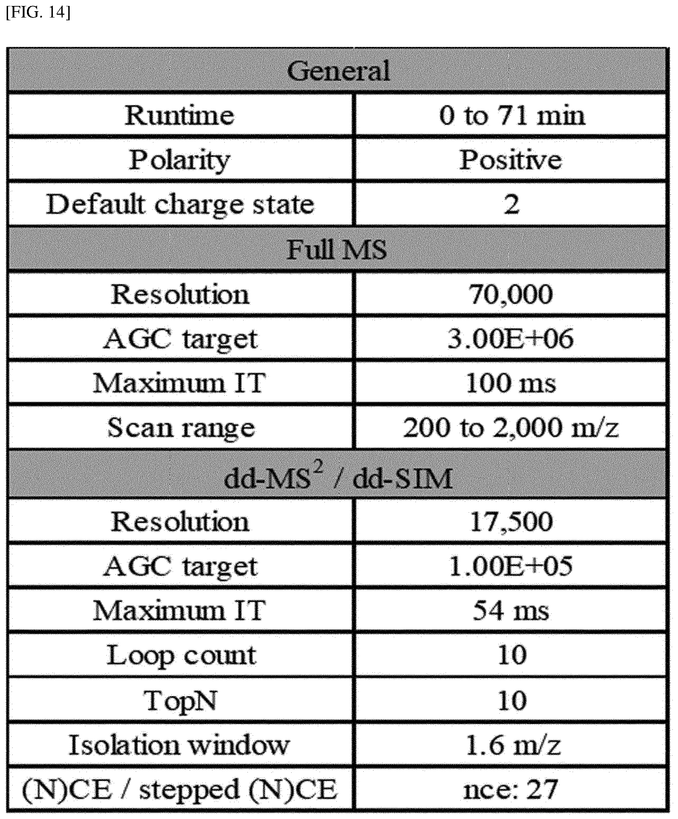

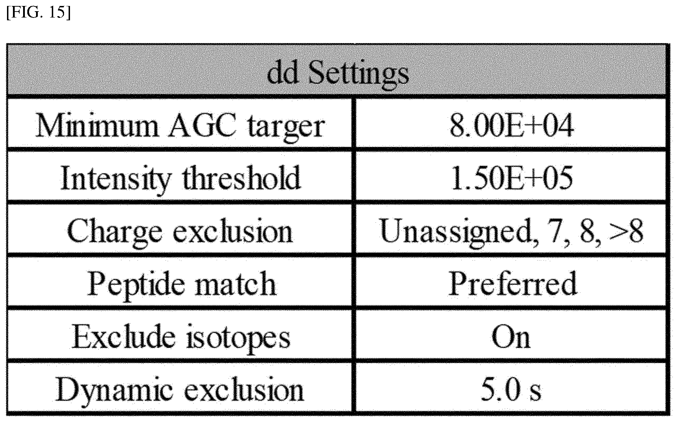

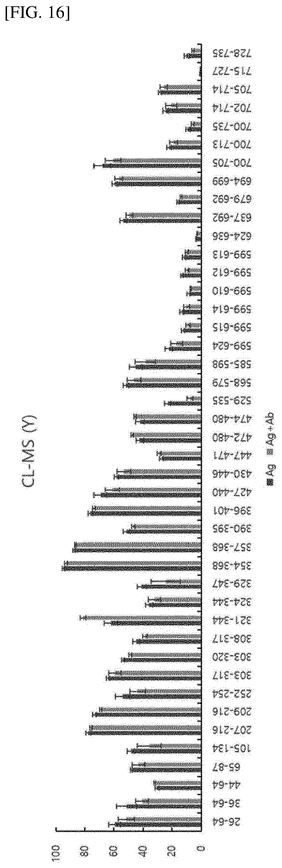

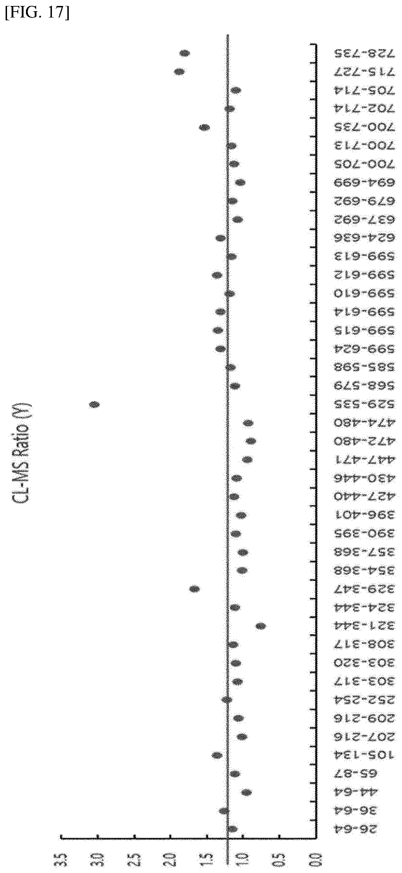

Thereafter, mass spectrometry (Q Exactive^{™} Plus Mass Spectrometer (Thermo SCIENTIFIC)) was performed under the conditions shown in FIGS. 13 to 15, and the measured mass spectrometry values of the antigen-antibody conjugate and the antigen were compared, and the results were shown in FIG. 16 and Table 5 below. Furthermore, from the measured mass spectrometry values, the ratio of the antigen-antibody conjugate/the antigen was calculated, and the results are shown in FIG. 17.

**[Table 5]**

| **Amino acid positions** | **Antigen** | | **Antigen-antibody conjugate** | | **Difference** | **Ratio** | ***p* value** |
|---|---|---|---|---|---|---|---|
| | **Mean** | **S.D** | **mean** | **S.D** | | | |
| 26-64 | 59.56 | 4.11 | 51.51 | 5.56 | 8.05 | 1.16 | ^{∗∗} |
| **36-64** | 51.37 | 7.00 | 40.77 | 4.25 | 10.60 | 1.26 | ^{∗∗} |
| 44-64 | 30.46 | 1.29 | 31.87 | 0.66 | -1.41 | 0.96 | ^{∗} |
| 65-87 | 47.90 | 0.74 | 43.09 | 4.36 | 4.81 | 1.11 | ^{∗} |
| **105-134** | 48.26 | 2.60 | 35.54 | 8.20 | 12.72 | 1.36 | ^{∗∗} |
| 207-216 | 77.52 | 1.61 | 75.76 | 0.72 | 1.77 | 1.02 | * |
| 209-216 | 73.44 | 1.19 | 69.09 | 0.63 | 4.34 | 1.06 | *** |
| **252-254** | 53.84 | 5.08 | 43.75 | 5.22 | 10.09 | 1.23 | ^{∗∗} |
| 303-317 | 64.17 | 1.22 | 59.52 | 4.26 | 4.65 | 1.08 | ^{∗} |
| 303-320 | 53.78 | 0.94 | 48.69 | 0.82 | 5.10 | 1.10 | *** |
| 308-317 | 44.52 | 2.44 | 38.84 | 1.67 | 5.68 | 1.15 | *** |
| 321-344 | 62.11 | 4.53 | 81.23 | 1.84 | -19.12 | 0.76 | *** |
| 324-344 | 35.87 | 2.41 | 32.23 | 4.19 | 3.64 | 1.11 | ^{∗} |
| **329-347** | 40.96 | 2.95 | 24.52 | 10.14 | 16.44 | 1.67 | ^{∗∗} |
| 354-368 | 94.66 | 0.98 | 92.95 | 1.02 | 1.71 | 1.02 | ^{∗∗} |
| 357-368 | 87.35 | 0.80 | 86.48 | 0.59 | 0.88 | 1.01 | * |
| 390-395 | 51.34 | 2.27 | 46.62 | 1.05 | 4.72 | 1.10 | *** |
| 396-401 | 76.00 | 1.59 | 73.84 | 0.93 | 2.15 | 1.03 | ^{∗∗} |
| 427-440 | 69.31 | 4.49 | 61.15 | 4.78 | 8.16 | 1.13 | ^{∗∗} |
| 430-446 | 58.32 | 1.57 | 53.15 | 4.67 | 5.17 | 1.10 | * |
| 447-471 | 27.21 | 1.42 | 28.88 | 1.33 | -1.67 | 0.94 | * |
| 472-480 | 42.21 | 2.48 | 47.49 | 0.82 | -5.28 | 0.89 | *** |
| 474-480 | 42.02 | 3.00 | 45.34 | 0.87 | -3.32 | 0.93 | ^{∗} |
| **529-535** | 22.96 | 2.26 | 7.52 | 2.03 | 15.44 | 3.05 | *** |
| 568-579 | 51.68 | 2.04 | 46.19 | 4.75 | 5.49 | 1.12 | ^{∗∗} |
| 585-598 | 44.89 | 4.28 | 38.20 | 7.01 | 6.69 | 1.18 | * |
| **599-624** | 22.09 | 2.48 | 16.84 | 4.15 | 5.25 | 1.31 | ^{∗∗} |
| **599-615** | 12.33 | 1.08 | 9.13 | 1.40 | 3.20 | 1.35 | *** |
| **599-614** | 13.25 | 1.26 | 10.06 | 2.00 | 3.19 | 1.32 | ^{∗∗} |
| 599-610 | 8.54 | 1.11 | 7.16 | 0.49 | 1.38 | 1.19 | ^{∗∗} |
| **599-612** | 12.89 | 0.77 | 9.49 | 1.18 | 3.39 | 1.36 | *** |
| 599-613 | 11.49 | 1.32 | 9.85 | 0.94 | 1.65 | 1.17 | ^{∗∗} |
| **624-636** | 3.40 | 0.20 | 2.58 | 0.21 | 0.82 | 1.32 | *** |
| 637-692 | 53.44 | 2.57 | 49.35 | 2.29 | 4.09 | 1.08 | ^{∗∗} |
| 679-692 | 15.77 | 0.70 | 13.62 | 0.74 | 2.15 | 1.16 | *** |
| 694-699 | 59.44 | 1.75 | 56.79 | 2.66 | 2.65 | 1.05 | ^{∗} |
| 700-705 | 67.98 | 5.79 | 60.47 | 5.42 | 7.51 | 1.12 | ^{∗} |
| 700-713 | 21.98 | 1.53 | 18.80 | 2.73 | 3.18 | 1.17 | ^{∗} |
| **700-735** | 9.04 | 1.23 | 5.87 | 1.02 | 3.17 | 1.54 | *** |
| 702-714 | 24.37 | 2.05 | 20.49 | 3.86 | 3.88 | 1.19 | * |
| 705-714 | 28.28 | 1.17 | 25.69 | 2.46 | 2.59 | 1.10 | ^{∗} |
| **715-727** | 0.66 | 0.26 | 0.35 | 0.11 | 0.31 | 1.88 | ^{∗∗} |
| **728-735** | 10.05 | 1.56 | 5.58 | 0.81 | 4.47 | 1.80 | *** |

As shown in FIGS. 16 and 17 and Table 5, it was confirmed that the polypeptides consisting of the amino acids at positions 36 to 64, 105 to 134, 252 to 254, 329 to 347, 529 to 535, 599 to 612, 599 to 614, 599 to 615, 599 to 624, 624 to 636, 700 to 735, 715 to 727, and 728 to 735, respectively, in the extracellular domain of Lrig-1 protein represented by SEQ ID NO: 4, had an antigen-antibody conjugate/antigen ratio of 1.2 or more, suggesting that these polypeptides were likely to be selected as epitopes of the Lrig-1 protein. Furthermore, it was confirmed that the polypeptides consisting of the amino acid sequences represented by SEQ ID NOs: 7 to 13 showed a more significant actual difference value, and thus were more likely to be selected as epitopes.

### [8-1-2] Epitope identification using trypsin

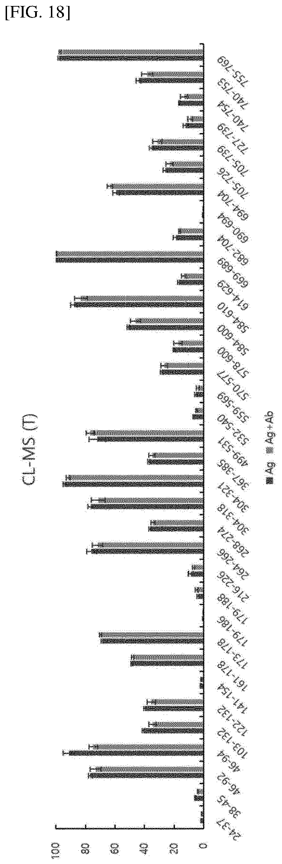

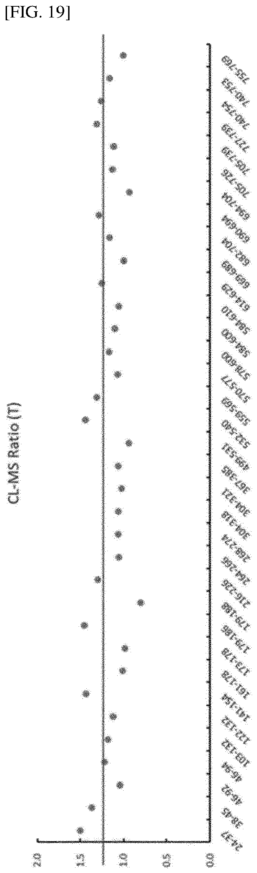

An experiment for epitope identification was conducted in the same manner as described in [8-1-1] above, except that trypsin was used instead of chymotrypsin as the enzyme. The results are shown in FIGS. 18 and 19 and Table 6 below.

**[Table 6]**

| **Amino acid positions** | **Antigen** | | **Antigen-antibody conjugate** | | **difference** | **Ratio** | ***p* value** |
|---|---|---|---|---|---|---|---|
| | **Mean** | **S.D** | **mean** | **S.D** | | | |
| **24-37** | 1.67 | 0.34 | 1.11 | 0.19 | 0.56 | 1.51 | ^{∗∗} |
| **38-45** | 5.70 | 0.33 | 4.15 | 0.15 | 1.55 | 1.37 | *** |
| 46-92 | 76.64 | 1.30 | 73.22 | 3.67 | 3.42 | 1.05 | ^{∗} |
| 46-94 | 91.22 | 3.99 | 74.75 | 2.92 | 16.47 | 1.22 | *** |
| 103-132 | 40.74 | 1.01 | 34.52 | 2.64 | 6.23 | 1.18 | *** |
| 122-132 | 39.75 | 0.85 | 35.32 | 2.70 | 4.43 | 1.13 | ^{∗∗} |
| **141-154** | 1.99 | 0.42 | 1.38 | 0.35 | 0.61 | 1.44 | ^{∗∗} |
| 161-178 | 48.76 | 0.58 | 48.00 | 0.75 | 0.75 | 1.02 | ^{∗} |
| 173-178 | 68.68 | 0.96 | 69.78 | 0.72 | -1.10 | 0.98 | * |
| **179-186** | 0.45 | 0.09 | 0.31 | 0.08 | 0.14 | 1.46 | ^{∗∗} |
| 179-188 | 3.91 | 0.71 | 4.87 | 0.97 | -0.96 | 0.80 | ^{∗} |
| **216-226** | 8.82 | 1.60 | 6.80 | 0.93 | 2.03 | 1.30 | ^{∗∗} |
| 264-266 | 75.97 | 3.22 | 71.79 | 3.45 | 4.18 | 1.06 | * |
| 268-274 | 36.31 | 0.82 | 34.17 | 1.44 | 2.14 | 1.06 | ^{∗∗} |
| 304-318 | 76.26 | 2.16 | 71.49 | 4.45 | 4.77 | 1.07 | ^{∗} |
| 304-321 | 94.20 | 0.90 | 91.94 | 1.35 | 2.26 | 1.02 | ^{∗∗} |
| 367-385 | 37.10 | 0.76 | 34.89 | 2.04 | 2.22 | 1.06 | * |
| 499-531 | 72.06 | 5.48 | 76.85 | 2.75 | -4.79 | 0.94 | * |
| **532-540** | 7.00 | 0.24 | 4.85 | 0.56 | 2.16 | 1.44 | *** |
| **559-569** | 5.17 | 1.00 | 3.93 | 0.77 | 1.24 | 1.31 | ^{∗} |
| 570-577 | 28.67 | 0.69 | 26.69 | 2.20 | 1.98 | 1.07 | ^{∗} |
| 578-600 | 20.13 | 0.54 | 17.24 | 2.84 | 2.89 | 1.17 | * |
| 584-600 | 51.23 | 0.73 | 46.30 | 3.49 | 4.93 | 1.11 | ^{∗∗} |
| 584-610 | 87.90 | 2.41 | 83.13 | 4.53 | 4.77 | 1.06 | ^{∗} |
| **614-629** | 16.83 | 0.83 | 13.41 | 1.55 | 3.42 | 1.25 | *** |
| 669-689 | 99.84 | 0.01 | 99.88 | 0.01 | -0.03 | 1.00 | *** |
| 682-704 | 18.83 | 1.57 | 16.22 | 0.47 | 2.61 | 1.16 | ^{∗∗} |
| **690-694** | 0.47 | 0.11 | 0.37 | 0.09 | 0.11 | 1.29 | * |
| 694-704 | 59.11 | 2.39 | 63.48 | 1.79 | -4.37 | 0.93 | ^{∗∗} |
| 705-726 | 26.02 | 1.55 | 23.05 | 2.64 | 2.97 | 1.13 | ^{∗} |
| 705-739 | 35.21 | 1.46 | 31.44 | 3.05 | 3.77 | 1.12 | ^{∗∗} |
| **727-739** | 12.04 | 1.75 | 9.20 | 1.58 | 2.85 | 1.31 | ^{∗∗} |
| 740-754 | 16.35 | 0.37 | 12.98 | 2.51 | 3.37 | 1.26 | ^{∗∗} |
| **740-753** | 44.06 | 1.69 | 38.00 | 3.84 | 6.07 | 1.16 | ^{∗∗} |
| 755-769 | 97.94 | 0.77 | 97.13 | 0.58 | 0.81 | 1.01 | ^{∗} |

As shown in FIGS. 18 and 19 and Table 6 above, it was confirmed that the polypeptides consisting of the amino acids at positions 24 to 37, 38 to 45, 46 to 94, 141 to 154, 179 to 186, 216 to 226, 532 to 540, 559 to 569, 614 to 629, 690 to 694, 727 to 739, 740 to 754, respectively, in the extracellular domain of Lrig-1 protein represented by SEQ ID NO: 4, had an antigen-antibody conjugate/antigen ratio of 1.2 or more, suggesting that these polypeptides were likely to be selected as epitopes of the Lrig-1 protein. Furthermore, it was confirmed that the polypeptides consisting of the amino acid sequences represented by SEQ ID NOs: 14 to 22 showed a more significant actual difference value, and thus were more likely to be selected as epitopes.

### [8-1-3] Epitope identification using Asp-N + Lys-C

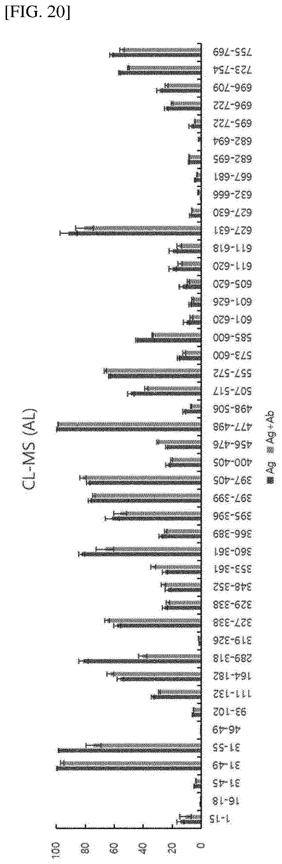

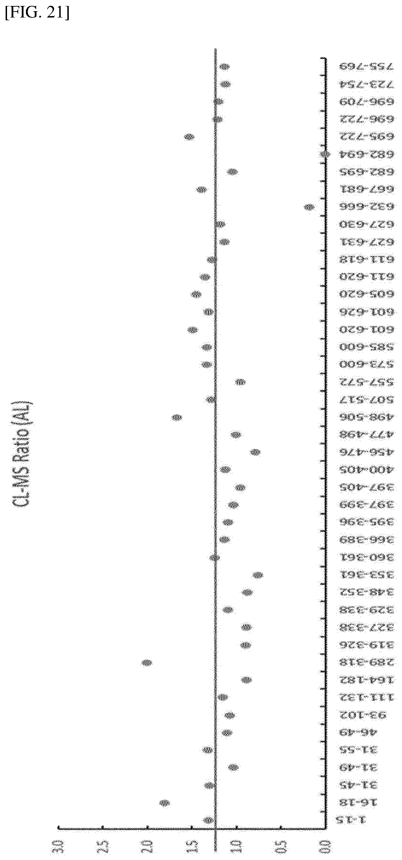

An experiment for epitope identification was conducted in the same manner as described in [8-1-1] above, except that Asp-*N* + Lys-C was used instead of chymotrypsin as the enzyme. The results are shown in FIGS. 20 and 21 and Table 7 below.

**[Table 7]**

| **Amino acid positions** | **Antigen** | | **Antigen-antibody conjugate** | | **difference** | **Ratio** | ***p* value** |
|---|---|---|---|---|---|---|---|
| | **mean** | **S.D** | **mean** | **S.D** | | | |
| **1-15** | 14.43 | 2.40 | 10.99 | 3.78 | 3.44 | 1.31 | * |
| **16-18** | 0.84 | 0.11 | 0.46 | 0.05 | 0.37 | 1.81 | ^{∗∗} |
| **31-45** | 5.01 | 0\.20 | 3.83 | 0.08 | 1.18 | 1.31 | *** |
| 31-49 | 99.66 | 0.12 | 95.79 | 1.18 | 3.87 | 1.04 | *** |
| **31-55** | 98.23 | 0.17 | 74.26 | 5.16 | 23.98 | 1.32 | *** |
| 46-49 | 0.45 | 0.03 | 0.41 | 0.01 | 0.04 | 1.10 | ^{∗∗} |
| 93-102 | 5.74 | 0.41 | 5.35 | 0.12 | 0.38 | 1.07 | ^{∗} |
| 111-132 | 33.35 | 0.92 | 28.93 | 0.61 | 4.42 | 1.15 | *** |
| 164-182 | 55.69 | 2.33 | 62.83 | 2.27 | -7.14 | 0.89 | *** |
| **289-318** | 81.31 | 3.28 | 40.46 | 2.89 | 40.86 | 2.01 | *** |
| 319-326 | 1.66 | 0.08 | 1.86 | 0.03 | -0.20 | 0.89 | *** |
| 327-338 | 57.92 | 2.43 | 65.19 | 1.55 | -7.27 | 0.89 | *** |
| 329-338 | 25.18 | 1.80 | 22.95 | 1.06 | 2.22 | 1.10 | ^{∗∗} |
| 348-352 | 22.88 | 2.16 | 26.19 | 1.63 | -3.32 | 0.87 | ^{∗∗} |
| 353-361 | 25.13 | 1.63 | 33.14 | 1.62 | -8.00 | 0.76 | *** |
| **360-361** | 82.60 | 2.14 | 66.35 | 6.14 | 16.24 | 1.24 | *** |
| 366-389 | 27.95 | 1.36 | 24.58 | 0.83 | 3.36 | 1.14 | *** |
| 395-396 | 61.42 | 4.84 | 55.89 | 4.32 | 5.52 | 1.10 | * |
| 397-399 | 76.77 | 0.99 | 73.96 | 0.83 | 2.82 | 1.04 | *** |
| 397-405 | 77.87 | 1.09 | 81.73 | 2.21 | -3.87 | 0.95 | ^{∗∗} |
| 400-405 | 22.98 | 1.52 | 20.48 | 0.55 | 2.50 | 1.12 | ^{∗∗} |
| 456-476 | 23.65 | 0.77 | 30.16 | 0.50 | -6.51 | 0.78 | *** |
| 477-498 | 100.00 | 0.00 | 98.88 | 0.04 | 1.12 | 1.01 | *** |
| **498-506** | 11.99 | 0.91 | 7.16 | 0.46 | 4.82 | 1.67 | *** |
| **507-517** | 48.71 | 2.15 | 37.83 | 1.31 | 10.87 | 1.29 | *** |
| 557-572 | 63.44 | 0.59 | 66.15 | 0.74 | -2.71 | 0.96 | *** |
| **573-600** | 15.68 | 0.87 | 11.78 | 0.98 | 3.90 | 1.33 | *** |
| **585-600** | 44.58 | 0.66 | 33.48 | 0.52 | 11.10 | 1.33 | *** |
| **601-620** | 10.27 | 2.06 | 6.88 | 0.83 | 3.39 | 1.49 | ^{∗∗} |
| **601-626** | 7.82 | 0.71 | 5.97 | 0.52 | 1.85 | 1.31 | *** |
| **605-620** | 12.99 | 2.31 | 8.95 | 0.88 | 4.03 | 1.45 | ^{∗∗} |
| **611-620** | 19.72 | 2.71 | 14.61 | 1.30 | 5.11 | 1.35 | *** |
| **611-618** | 19.38 | 2.98 | 15.17 | 1.53 | 4.22 | 1.28 | ^{∗∗} |
| 627-631 | 91.56 | 5.88 | 80.51 | 6.05 | 11.05 | 1.14 | ^{∗∗} |
| 627-630 | 7.59 | 0.53 | 6.42 | 0.20 | 1.17 | 1.18 | *** |
| 632-666 | 0.34 | 0.15 | 1.88 | 0.32 | -1.54 | 0.18 | *** |
| **667-681** | 4.25 | 0.29 | 3.04 | 0.11 | 1.21 | 1.40 | *** |
| 682-695 | 8.62 | 0.34 | 8.28 | 0.15 | 0.34 | 1.04 | * |
| 682-694 | 0.01 | 0.01 | 1.52 | 0.33 | -1.51 | 0.01 | *** |
| **695-722** | 7.06 | 1.46 | 4.61 | 0.28 | 2.45 | 1.53 | ^{∗∗} |
| **696-722** | 24.25 | 1.22 | 19.92 | 0.59 | 4.33 | 1.22 | *** |
| **696-709** | 28.84 | 2.10 | 23.96 | 0.97 | 4.88 | 1.20 | *** |
| 723-754 | 56.36 | 0.47 | 50.13 | 0.43 | 6.23 | 1.12 | *** |
| 755-769 | 61.79 | 1.18 | 54.57 | 1.37 | 7.22 | 1.13 | *** |

As shown in FIGS. 20 and 21 and Table 7 above, it was confirmed that the polypeptides consisting of the amino acids at positions 1 to 15, 16 to 18, 31 to 45, 31 to 55, 289 to 318, 360 to 361, 498 to 506, 507 to 517, 573 to 600, 585 to 600, 601 to 620, 601 to 626, 605 to 620, 611 to 618, 611 to 620, 667 to 681, 695 to 722, 696 to 709, and 696 to 722, respectively, in the extracellular domain of Lrig-1 protein represented by SEQ ID NO: 4, had an antigen-antibody conjugate/antigen ratio of 1.2 or more, suggesting that these polypeptides were likely to be selected as epitopes of the Lrig-1 protein. Furthermore, it was confirmed that the polypeptides consisting of the amino acid sequences represented by SEQ ID NOs: 23 to 28 showed a more significant actual difference value, and thus were more likely to be selected as epitopes.

As a result of performing additional analysis in a manner that overlaps the results of the epitope identification performed in [8-1-1] to [8-1-3] above, it could be seen that the polypeptides listed in the results were likely to be epitopes, and in particular, the polypeptides consisting of the amino acid sequences represented by SEQ ID NOs: 29 to 32 were more likely to be epitopes of the Lrig-1 protein for the GTC110-04 antibody.

### [8-2] Cross-linking mass spectroscopy (XL-MS)

### [8-2-1] Epitope identification using chymotrypsin

Two antigen-antibody conjugate samples were prepared by mixing the GTC110-04 antibody of Production Example 8 and the extracellular domain (antigen) of Lrig-1 protein represented by SEQ ID NO: 4 at a ratio of 2:1 and allowing the mixture to react sufficiently. Next, DSG (disuccinimidyl glutarate, 20593, Thermo) sufficiently dissolved in DMSO (dimethyl sulfoxide) was added to one of the samples at a ratio of 1:50 (sample: DSG) (T%), and only DMSO was added to the other sample (C%). Thereafter, each of the samples was allowed to react sufficiently at 25°C for 1 hour. Then, in the same manner as in [8-1-1], each sample was treated with chymotrypsin and subjected to LC-MS analysis to obtain mass spectrometry values.

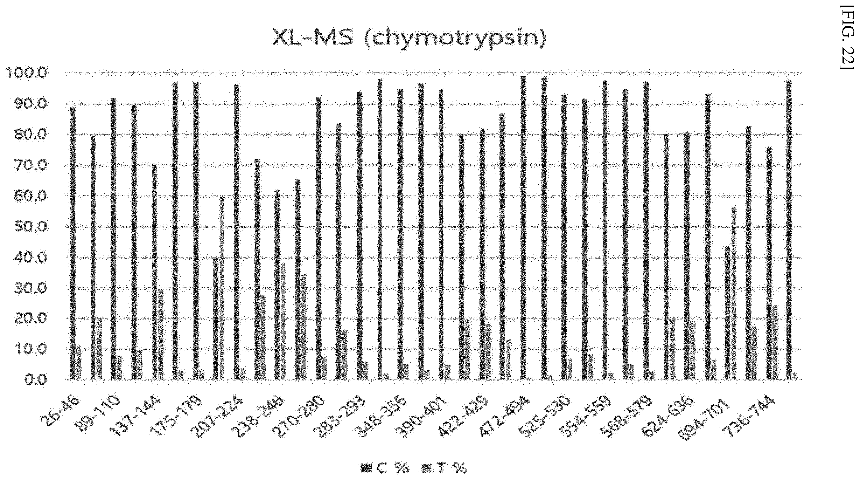

Thereafter, the difference between mass spectrometry values between the sample (T%) to which DSG was added and the sample (C%) to which only DMSO was added was examined. Among the epitopes having the difference, binding auxiliary regions (mono type and loop type) that were not epitopes were excluded, and regions having a difference of 10% or less were finally selected. The results are shown in FIG. 22 and Table 8 below.

**[Table 8]**

| **Amino acid positions** | **C%** | **T%** | **Linker site** | **C%** | **T%+M/L** |
|---|---|---|---|---|---|
| 26-46 | 88.9 | 11.1 | T36 | 50.8 | 49.2 |
| 66-86 | 79.6 | 20.4 | | | |
| 89-110 | 92.0 | 8.0 | K102 | 69.2 | 30.8 |
| 126-136 | 90.1 | 9.9 | | | |
| 137-144 | 70.5 | 29.5 | | | |
| 158-174 | 96.8 | 3.2 | K163 | 65.1 | 34.9 |
| 175-179 | 97.1 | 2.9 | K175 | 52.5 | 47.5 |
| 198-208 | 40.2 | 59.8 | | | |
| 207-224 | 96.4 | 3.6 | K207 | 82.3 | 17.7 |
| 222-237 | 72.2 | 27.8 | | | |
| 238-246 | 62.0 | 38.0 | | | |
| 252-269 | 65.4 | 34.6 | K267 | 47.2 | 52.8 |
| 270-280 | 92.2 | 7.8 | K274 | 87.3 | 12.7 |
| 279-282 | 83.6 | 16.4 | | | |
| 283-293 | 94.0 | 6.0 | T286 | 50.2 | 49.8 |
| 305-344 | 98.0 | 2.0 | K318 | 55.2 | 44.8 |
| 348-356 | 94.7 | 5.3 | K352 | 64.5 | 35.5 |
| 357-368 | 96.7 | 3.3 | K359,365 | 88.7 | 11.3 |
| 390-401 | 94.7 | 5.3 | K394 | 85.7 | 14.3 |
| 417-420 | 80.3 | 19.7 | | | |
| 422-429 | 81.7 | 18.3 | | | |
| 441-453 | 86.7 | 13.3 | K441 | 74.7 | 25.3 |
| **472-494** | 99.1 | 0.9 | K476 | 97.1 | 2.9 |
| **495-511** | 98.5 | 1.5 | K497 | 98.2 | 1.8 |
| **525-530** | 92.9 | 7.1 | T525 | 92.7 | 7.3 |
| 531-541 | 91.7 | 8.3 | Y541 | 78.9 | 21.1 |
| 554-559 | 97.7 | 2.3 | K556 | 83.1 | 16.9 |
| **560-567** | 94.7 | 5.3 | T560 | 92.7 | 7.3 |
| **568-579** | 97.1 | 2.9 | T568, K569 | 94.9 | 5.1 |
| 585-598 | 80.2 | 19.8 | | | |
| 624-636 | 80.8 | 19.2 | T626 | 80.7 | 19.3 |
| **680-693** | 93.3 | 6.7 | | 93.3 | 6.7 |
| 694-701 | 43.5 | 56.5 | | | |
| 715-727 | 82.6 | 17.4 | | | |
| 736-744 | 75.8 | 24.2 | | | |
| 745-762 | 97.5 | 2.5 | K754 | 91.8 | 8.2 |

As shown in FIG. 22 and Table 8 above, it was confirmed that the polypeptides consisting of the amino acids at positions 472 to 494 (SEQ ID NO: 32), 495 to 511 (SEQ ID NO: 33), 525 to 530 (SEQ ID NO: 34), 560 to 567 (SEQ ID NO: 35), 568 to 579 (SEQ ID NO: 36), 680 to 693 (SEQ ID NO: 37), and 745 to 762 (SEQ ID NO: 38), respectively, in the extracellular domain of Lrig-1 protein represented by SEQ ID NO: 4, showed a very low crosslinking level of 5% or less or 10% or less, suggesting that these polypeptides were likely to be selected as epitopes of the Lrig-1 protein.

### [8-2-2] Epitope identification using trypsin

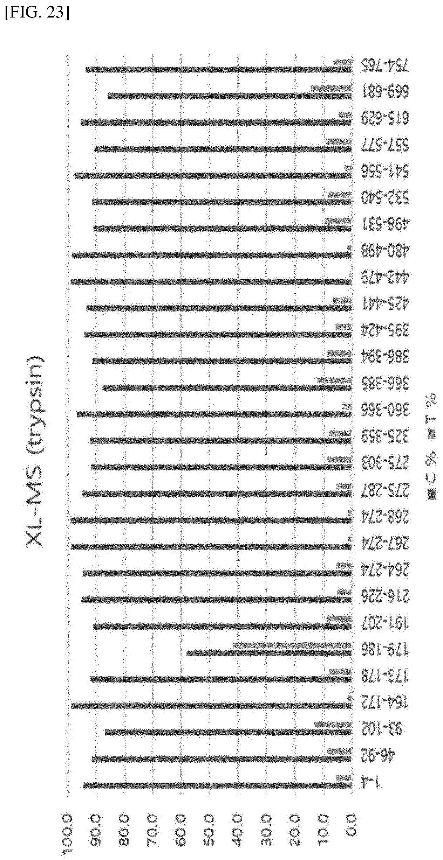

An experiment for epitope identification was conducted in the same manner as described in [8-2-1] above, except that trypsin was used instead of chymotrypsin as the enzyme. The results are shown in FIG. 23 and Table 9 below.

**[Table 9]**

| **Amino acid positions** | **C %** | **T %** | **Linker site** | **C %** | **T % + M/L** |
|---|---|---|---|---|---|
| **1-4** | 94.6 | 5.4 | | 94.6 | 5.4 |
| **46-92** | 91.4 | 8.6 | | 91.4 | 8.6 |
| 93-102 | 86.8 | 13.2 | | | |
| **164-172** | 98.6 | 1.4 | | 98.6 | 1.4 |
| 173-178 | 91.9 | 8.1 | K175 | 73.8 | 26.2 |
| 179-186 | 58.1 | 41.9 | | | |
| 191-207 | 91.0 | 9.0 | K207 | 77.0 | 23.0 |
| 216-226 | 95.0 | 5.0 | K215 | 88.6 | 11.4 |
| **264-274** | 94.6 | 5.4 | | 94.6 | 5.4 |
| **267-274** | 98.7 | 1.3 | K267 | 92.9 | 7.1 |
| **268-274** | 98.9 | 1.1 | | 98.9 | 1.1 |
| **275-287** | 94.8 | 5.2 | | 94.8 | 5.2 |
| **275-303** | 91.6 | 8.4 | | 91.6 | 8.4 |
| 325-359 | 92.1 | 7.9 | K352 | 89.9 | 10.1 |
| 360-366 | 96.7 | 3.3 | K361, 365 | 58.0 | 42.0 |
| 366-385 | 87.8 | 12.2 | | | |
| **386-394** | 91.3 | 8.7 | | 91.3 | 8.7 |
| **395-424** | 94.2 | 5.8 | K396, 415 | 93.6 | 6.4 |
| **425-441** | 93.3 | 6.7 | | 93.3 | 6.7 |
| **442-479** | 99.0 | 1.0 | | 99.0 | 1.0 |
| **480-498** | 98.4 | 1.6 | | 98.4 | 1.6 |
| **498-531** | 90.9 | 9.1 | | 90.9 | 9.1 |
| **532-540** | 91.4 | 8.6 | | 91.4 | 8.6 |
| **541-556** | 97.5 | 2.5 | K556 | 96.3 | 3.7 |
| 557-577 | 90.8 | 9.2 | K569 | 87.9 | 12.1 |
| **615-629** | 95.3 | 4.7 | | 95.3 | 4.7 |
| 669-681 | 85.8 | 14.2 | | | |
| 754-765 | 93.6 | 6.4 | K754 | 53.0 | 47.0 |

As shown in FIG. 23 and Table 9 above, it was confirmed that the polypeptides consisting of the amino acids at positions 1 to 4 (SEQ ID NO: 80), 46 to 92 (SEQ ID NO: 40), 164 to 172 (SEQ ID NO: 41), 264 to 274 (SEQ ID NO: 42), 267 to 274 (SEQ ID NO: 43), 268 to 274 (SEQ ID NO: 44), 275 to 287 (SEQ ID NO: 45), 275 to 303 (SEQ ID NO: 46), 386 to 394 (SEQ ID NO: 47), 395 to 424 (SEQ ID NO: 48), 425 to 441 (SEQ ID NO: 49), 442 to 479 (SEQ ID NO: 50), 480 to 498 (SEQ ID NO: 51), 498 to 531 (SEQ ID NO: 52), 532 to 540 (SEQ ID NO: 53), 541 to 556 (SEQ ID NO: 54), and 615 to 629 (SEQ ID NO: 55), respectively, in the extracellular domain of Lrig-1 protein represented by SEQ ID NO: 4, showed a very low crosslinking level of 5% or less or 10% or less, suggesting that these polypeptides were likely to be selected as epitopes of the Lrig-1 protein.

### [8-2-3] Epitope identification using Asp-N + Lys-C

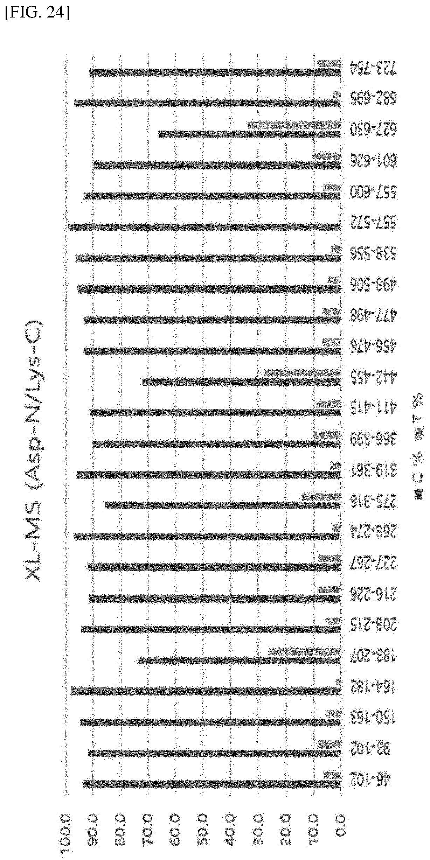

An experiment for epitope identification was conducted in the same manner as described in [8-2-1] above, except that Asp-*N* + Lys-*C* was used instead of chymotrypsin as the enzyme. The results are shown in FIG. 24 and Table 10 below.

**[Table 10]**

| **Amino acid positions** | **C %** | **T %** | **Linker site** | **C %** | **T % + M/L** |
|---|---|---|---|---|---|
| **46-102** | 93.7 | 6.3 | | 93.7 | 6.3 |
| **93-102** | 91.6 | 8.4 | | 91.6 | 8.4 |
| **150-163** | 94.5 | 5.5 | | 94.5 | 5.5 |
| **164-182** | 98.0 | 2.0 | | 98.0 | 2.0 |
| 183-207 | 73.7 | 26.3 | | | |
| 208-215 | 94.3 | 5.7 | K215 | 85.1 | 14.9 |
| **216-226** | 91.4 | 8.6 | | 91.4 | 8.6 |
| **227-267** | 91.8 | 8.2 | | 91.8 | 8.2 |
| **268-274** | 97.0 | 3.0 | | 97.0 | 3.0 |
| 275-318 | 85.6 | 14.4 | | | |
| **319-361** | 96.0 | 4.0 | K352, 361 | 91.6 | 8.4 |
| 366-399 | 90.1 | 9.9 | K394, 396 | 89.9 | 10.1 |
| **411-415** | 91.1 | 8.9 | | 91.1 | 8.9 |
| 442-455 | 72.2 | 27.8 | | | |
| **456-476** | 93.3 | 6.7 | | 93.3 | 6.7 |
| **477-498** | 93.4 | 6.6 | | 93.4 | 6.6 |
| **498-506** | 95.5 | 4.5 | | 95.5 | 4.5 |
| **538-556** | 96.4 | 3.6 | | 96.4 | 3.6 |
| **557-572** | 99.3 | 0.7 | T560 | 98.5 | 1.5 |
| **557-600** | 93.6 | 6.4 | | 93.6 | 6.4 |
| 601-626 | 89.7 | 10.3 | | | |
| 627-630 | 66.0 | 34.0 | K629 | 64.7 | 35.3 |
| 682-695 | 97.1 | 2.9 | K694 | 79.4 | 20.6 |
| 723-754 | 91.4 | 8.6 | K754 | 84.1 | 15.9 |

As shown in FIG. 24 and Table 10 above, it was confirmed that the polypeptides consisting of the amino acids at positions 46 to 102 (SEQ ID NO: 56), 93 to 102 (SEQ ID NO: 57), 150 to 163 (SEQ ID NO: 58), 164 to 182 (SEQ ID NO: 59), 216 to 226 (SEQ ID NO: 60), 227 to 267 (SEQ ID NO: 61), 268 to 274 (SEQ ID NO: 62), 319 to 361 (SEQ ID NO: 63), 411 to 415 (SEQ ID NO: 64), 456 to 476 (SEQ ID NO: 65), 477 to 498 (SEQ ID NO: 66), 498 to 506 (SEQ ID NO: 67), 538 to 556 (SEQ ID NO: 68), 557 to 572 (SEQ ID NO: 69), and 557 to 600 (SEQ ID NO: 70), respectively, in the extracellular domain of Lrig-1 protein represented by SEQ ID NO: 4, showed a very low crosslinking level of 5% or less or 10% or less, suggesting that these polypeptides were likely to be selected as epitopes of the Lrig-1 protein.

As a result of performing additional analysis in a manner that overlaps the results of the epitope identification performed in [8-2-1] to [8-2-3] above, it could be seen that the polypeptides listed in the results were likely to be epitopes, and in particular, the polypeptide consisting of the amino acid sequences represented by any one of SEQ ID NOs: 32 to 36, SEQ ID NOs: 40 to 42, and SEQ ID NOs: 64 and 68 was more likely to be an epitope of the Lrig-1 protein for the GTC110-04 antibody.

### [8-3] Epitope prediction

Based on the sequence of specific epitope sequences obtained through the process of overlapping the results of epitope identification obtained in [8-1] and [8-2] above, an experiment for epitope identification was conducted in the same manner as in [8-1] above. The results are shown in Tables 11 and 12 below.

**[Table 11]**

| **Enzyme** | **Amino acid positions** | **Antigen** | **Antigen-antibody conjugate** | **Difference** | **Ratio** |
|---|---|---|---|---|---|
| Asp-N Lys-C | 31-45 | 5.01 | 3.83 | 1.18 | 1.31 |
| Asp-N Lys-C | 31-55 | 98.23 | 74.26 | 23.98 | 1.32 |
| Chymotrypsin | 36-64 | 51.37 | 40.77 | 10.60 | 1.26 |
| Trypsin | 38-45 | 5.70 | 4.15 | 1.55 | 1.37 |
| Trypsin | 46-94 | 91.22 | 74.75 | 16.47 | 1.22 |

**[Table 12]**

| **Enzyme** | **Amino acid positions** | **Antigen** | **Antigen-antibody conjugate** | **Difference** | **Ratio** |
|---|---|---|---|---|---|
| Trypsin | 24-37 | 1.67 | 1.11 | 0.56 | 1.51 |
| Chymotrypsin | 26-64 | 59.56 | 51.51 | 8.05 | 1.16 |
| Asp-N Lys-C | 31-49 | 99.66 | 95.79 | 3.87 | 1.04 |
| Chymotrypsin | 38-64 | 53.47 | 46.30 | 7.16 | 1.15 |
| Asp-N Lys-C | 62-92 | 65.30 | 65.51 | -0.22 | 1.00 |
| Chymotrypsin | 65-87 | 47.90 | 43.09 | 4.81 | 1.11 |
| Chymotrypsin | 88-94 | 89.93 | 89.48 | 0.44 | 1.00 |

As shown in Tables 11 and 12, it was confirmed that the polypeptide consisting of the amino acids at positions 46 to 87 or 46 to 64 in the extracellular domain of Lrig-1 protein represented by SEQ ID NO: 4 showed a great difference in the ratio in the results obtained using various enzymes.

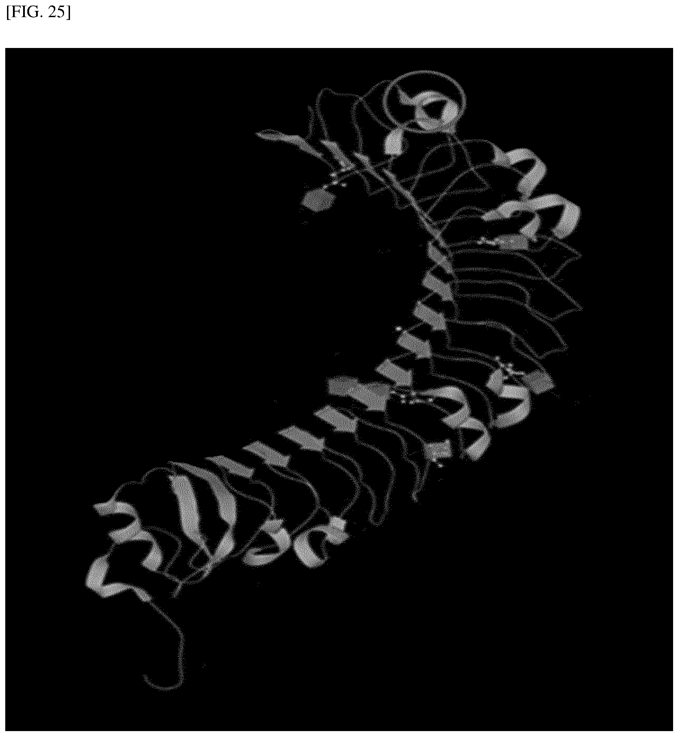

From the above results, it can be seen that the GTC110-04 antibody may bind specifically to the polypeptide consisting of the amino acids at positions 46 to 87 or 46 to 64 in the amino acid sequence of the extracellular domain of the Lrig-1 protein (circled region in FIG. 25).

Although the present invention has been described in detail above, the scope of the present invention is not limited thereto, and it will be apparent to those of ordinary skill in the art that various modifications and changes are possible without departing from the technical spirit of the present invention as defined in the appended claims.

### Industrial Applicability

The antibody or antigen-binding fragment that binds specifically to the epitope of Lrig-1 protein according to the present invention may bind specifically to the epitope of the present invention, which is present on regulatory T cells, thereby inhibiting the function of the regulatory T cells and maintaining or increasing the activity of effector T cells. Thus, it may be very efficiently used for the prevention, amelioration or treatment of cancer.

## Claims

1. An epitope of Lrig-1 (leucine-rich and immunoglobulin-like domains 1) protein comprising at least one polypeptide selected from the group consisting of polypeptides consisting of the amino acid sequences represented by SEQ ID NOs: 7 to 70.

2. An epitope of Lrig-1 (leucine-rich and immunoglobulin-like domains 1) protein comprising a polypeptide consisting of the amino acid sequences represented by SEQ ID NO: 71 or SEQ ID NO: 72.

3. An antibody or antigen-binding fragment that binds specifically to an epitope comprising at least one polypeptide selected from the group consisting of polypeptides consisting of the amino acid sequences represented by SEQ ID NOS: 7 to 70.

4. An antibody or antigen-binding fragment that binds specifically to an epitope comprising a polypeptide consisting of the amino acid sequence represented by SEQ ID NO: 71 or SEQ ID NO: 72.

5. A nucleic acid molecule encoding the epitope of claim 1 or 2.

6. An expression vector into which the nucleic acid molecule of claim 5 has been inserted.

7. A host cell line transfected with the expression vector of claim 6.

8. A pharmaceutical composition for preventing or treating cancer containing the antibody or antigen-binding fragment of claim 3 or 4 as an active ingredient.

9. The pharmaceutical composition of claim 8, wherein the cancer is gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, or lung cancer

10. An antibody-drug conjugate comprising: the antibody or antigen-binding fragment of claim 3 or 4; and a drug.

11. A method for preventing or treating cancer comprising a step of administering, to a subject, a pharmaceutically effective amount of an antibody or antigen-binding fragment that binds specifically to an epitope comprising at least one polypeptide selected from the group consisting of polypeptides consisting of the amino acid sequences represented by SEQ ID NOS: 7 to 70.

12. A method for preventing or treating cancer comprising a step of administering, to a subject, a pharmaceutically effective amount of an antibody or antigen-binding fragment that binds specifically to an epitope comprising a polypeptide consisting of the amino acid sequence represented by SEQ ID NO: 71 or SEQ ID NO: 72.


<110>    Good T Cells, Inc.

<120>    Epitope of regulatory T cell surface antigen and an antibody
         specifically binding to the epitope thereof

<130>    POPB204290PCT

<150>    KR 10-2019-0142251
<151>    2019-11-08

<160>    164

<170>    KoPatentIn 3.0

<210>    1
<211>    1093
<212>    PRT
<213>    Homo sapiens

<400>    1


<210>   2
<211>   3282
<212>   DNA
<213>   Homo sapiens

<400>   2
atggcgcggc cggtccgggg agggctcggg gccccgcgcc gctcgccttg ccttctcctt      60

ctctggctgc ttttgcttcg gctggagccg gtgaccgccg cggccggccc gcgggcgccc     120

tgcgcggccg cctgcacttg cgctggggac tcgctggact gcggtgggcg cgggctggct     180

gcgttgcccg gggacctgcc ctcctggacg cggagcctaa acctgagtta caacaaactc     240

tctgagattg accctgctgg ttttgaggac ttgccgaacc tacaggaagt gtacctcaat     300

aataatgagt tgacagcggt accatccctg ggcgctgctt catcacatgt cgtctctctc     360

tttctgcagc acaacaagat tcgcagcgtg gaggggagcc agctgaaggc ctacctttcc     420

ttagaagtgt tagatctgag tttgaacaac atcacggaag tgcggaacac ctgctttcca     480

cacggaccgc ctataaagga gctcaacctg gcaggcaatc ggattggcac cctggagttg     540

ggagcatttg atggtctgtc acggtcgctg ctaactcttc gcctgagcaa aaacaggatc 600
acccagcttc ctgtaagagc attcaagcta cccaggctga cacaactgga cctcaatcgg 660
aacaggattc ggctgataga gggcctcacc ttccaggggc tcaacagctt ggaggtgctg 720
aagcttcagc gaaacaacat cagcaaactg acagatgggg ccttctgggg actgtccaag 780
atgcatgtgc tgcacctgga gtacaacagc ctggtagaag tgaacagcgg ctcgctctac 840
ggcctcacgg ccctgcatca gctccacctc agcaacaatt ccatcgctcg cattcaccgc 900
aagggctgga gcttctgcca gaagctgcat gagttggtcc tgtccttcaa caacctgaca 960
cggctggacg aggagagcct ggccgagctg agcagcctga gtgtcctgcg tctcagccac 1020
aattccatca gccacattgc ggagggtgcc ttcaagggac tcaggagcct gcgagtcttg 1080
gatctggacc ataacgagat ttcgggcaca atagaggaca cgagcggcgc cttctcaggg 1140
ctcgacagcc tcagcaagct gactctgttt ggaaacaaga tcaagtctgt ggctaagaga 1200
gcattctcgg ggctggaagg cctggagcac ctgaaccttg gagggaatgc gatcagatct 1260
gtccagtttg atgcctttgt gaagatgaag aatcttaaag agctccatat cagcagcgac 1320
agcttcctgt gtgactgcca gctgaagtgg ctgcccccgt ggctaattgg caggatgctg 1380
caggcctttg tgacagccac ctgtgcccac ccagaatcac tgaagggtca gagcattttc 1440
tctgtgccac cagagagttt cgtgtgcgat gacttcctga agccacagat catcacccag 1500
ccagaaacca ccatggctat ggtgggcaag gacatccggt ttacatgctc agcagccagc 1560
agcagcagct cccccatgac ctttgcctgg aagaaagaca atgaagtcct gaccaatgca 1620
gacatggaga actttgtcca cgtccacgcg caggacgggg aagtgatgga gtacaccacc 1680
atcctgcacc tccgtcaggt cactttcggg cacgagggcc gctaccaatg tgtcatcacc 1740
aaccactttg gctccaccta ttcacataag gccaggctca ccgtgaatgt gttgccatca 1800
ttcaccaaaa cgccccacga cataaccatc cggaccacca ccatggcccg cctcgaatgt 1860
gctgccacag gtcacccaaa ccctcagatt gcctggcaga aggatggagg cacggatttc 1920
cccgctgccc gtgagcgacg catgcatgtc atgccggatg acgacgtgtt tttcatcact 1980
gatgtgaaaa tagatgacgc aggggtttac agctgtactg ctcagaactc agccggttct 2040
atttcagcta atgccaccct gactgtccta gagaccccat ccttggtggt ccccttggaa 2100
gaccgtgtgg tatctgtggg agaaacagtg gccctccaat gcaaagccac ggggaaccct 2160
ccgccccgca tcacctggtt caagggggac cgcccgctga gcctcactga gcggcaccac 2220
ttgacccctg acaaccagct cctggtggtt cagaacgtgg tggcagagga tgcgggccga 2280
tatacctgtg agatgtccaa caccctgggc acggagcgag ctcacagcca gctgagcgtc 2340
ctgcccgcag caggctgcag gaaggatggg accacggtag gcatcttcac cattgctgtc 2400
gtgagcagca tcgtcctgac gtcactggtc tgggtgtgca tcatctacca gaccaggaag 2460

aagagtgaag agtacagtgt caccaacaca gatgaaaccg tcgtgccacc agatgttcca 2520

agctacctct cttctcaggg gaccctttct gaccgacaag aaaccgtggt caggaccgag 2580

ggtggccctc aggccaatgg gcacattgag agcaatggtg tgtgtccaag agatgcaagc 2640

cactttccag agcccgacac tcacagcgtt gcctgcaggc agccaaagct ctgtgctggg 2700

tctgcgtatc acaaagagcc gtggaaagcg atggagaaag ctgaagggac acctgggcca 2760

cataagatgg aacacggtgg ccgggtcgta tgcagtgact gcaacaccga agtggactgt 2820

tactccaggg gacaagcctt ccacccccag cctgtgtcca gagacagcgc acagccaagt 2880

gcgccaaatg gcccggagcc gggtgggagt gaccaagagc attctccaca tcaccagtgc 2940

agcaggactg ccgctgggtc ctgccccgag tgccaagggt cgctctaccc cagtaaccac 3000

gatagaatgc tgacggctgt gaagaaaaag ccaatggcat ctctagatgg gaaaggggat 3060

tcttcctgga ctttagcaag gttgtatcac ccggactcca cagagctaca gcctgcatct 3120

tcattaactt caggcagtcc agagcgcgcg gaagcccagt acttgcttgt ttccaatggc 3180

cacctcccca aagcatgtga cgccagtccc gagtccacgc cactgacagg acagctcccc 3240

gggaaacaga gggtgccact gctgttggca ccaaaaagct ag 3282

<210> 3
<211> 1091
<212> PRT
<213> Mus musculus

<400> 3
Met Ala Arg Pro Gly Pro Gly Val Leu Gly Ala Pro Arg Leu Ala Pro
1               5                   10                  15

Arg Leu Leu Leu Trp Leu Leu Leu Leu Leu Leu Gln Trp Pro Glu Ser
            20                  25                  30

Ala Gly Ala Gln Ala Gly Pro Arg Ala Pro Cys Ala Ala Ala Cys Thr
        35                  40                  45

Cys Ala Gly Asp Ser Leu Asp Cys Ser Gly Arg Gly Leu Ala Thr Leu
    50                  55                  60

Pro Arg Asp Leu Pro Ser Trp Thr Arg Ser Leu Asn Leu Ser Tyr Asn
65                  70                  75                  80

Arg Leu Ser Glu Ile Asp Ser Ala Ala Phe Glu Asp Leu Thr Asn Leu
                85                  90                  95

Gln Glu Val Tyr Leu Asn Ser Asn Glu Leu Thr Ala Ile Pro Ser Leu
            100                 105                 110

Gly Thr Ala Ser Ile Gly Val Val Ser Leu Phe Leu Gln His Asn Lys
        115                 120                 125

Ile Leu Ser Val Asp Gly Ser Gln Leu Lys Ser Tyr Leu Ser Leu Glu
    130                 135                 140

Val Leu Asp Leu Ser Ser Asn Asn Ile Thr Glu Ile Arg Ser Ser Cys
145                 150                 155                 160

Phe Pro Asn Gly Leu Arg Ile Arg Glu Leu Asn Leu Ala Ser Asn Arg
                165                 170                 175

Ile Ser Ile Leu Glu Ser Gly Ala Phe Asp Gly Leu Ser Arg Ser Leu
            180                 185                 190

Leu Thr Leu Arg Leu Ser Lys Asn Arg Ile Thr Gln Leu Pro Val Lys
        195                 200                 205

Ala Phe Lys Leu Pro Arg Leu Thr Gln Leu Asp Leu Asn Arg Asn Arg
    210                 215                 220

Ile Arg Leu Ile Glu Gly Leu Thr Phe Gln Gly Leu Asp Ser Leu Glu
225                 230                 235                 240

Val Leu Arg Leu Gln Arg Asn Asn Ile Ser Arg Leu Thr Asp Gly Ala
                245                 250                 255

Phe Trp Gly Leu Ser Lys Met His Val Leu His Leu Glu Tyr Asn Ser
            260                 265                 270

Leu Val Glu Val Asn Ser Gly Ser Leu Tyr Gly Leu Thr Ala Leu His
        275                 280                 285

Gln Leu His Leu Ser Asn Asn Ser Ile Ser Arg Ile Gln Arg Asp Gly
    290                 295                 300

Trp Ser Phe Cys Gln Lys Leu His Glu Leu Ile Leu Ser Phe Asn Asn
305                 310                 315                 320

Leu Thr Arg Leu Asp Glu Glu Ser Leu Ala Glu Leu Ser Ser Leu Ser
                325                 330                 335

Ile Leu Arg Leu Ser His Asn Ala Ile Ser His Ile Ala Glu Gly Ala
            340                 345                 350

Phe Lys Gly Leu Lys Ser Leu Arg Val Leu Asp Leu Asp His Asn Glu
        355                 360                 365

Ile Ser Gly Thr Ile Glu Asp Thr Ser Gly Ala Phe Thr Gly Leu Asp
    370                 375                 380

Asn Leu Ser Lys Leu Thr Leu Phe Gly Asn Lys Ile Lys Ser Val Ala
385                 390                 395                 400

Lys Arg Ala Phe Ser Gly Leu Glu Ser Leu Glu His Leu Asn Leu Gly
                405                 410                 415

Glu Asn Ala Ile Arg Ser Val Gln Phe Asp Ala Phe Ala Lys Met Lys
            420                 425                 430

Asn Leu Lys Glu Leu Tyr Ile Ser Ser Glu Ser Phe Leu Cys Asp Cys
        435                 440                 445

Gln Leu Lys Trp Leu Pro Pro Trp Leu Met Gly Arg Met Leu Gln Ala
    450                 455                 460

Phe Val Thr Ala Thr Cys Ala His Pro Glu Ser Leu Lys Gly Gln Ser
465                 470                 475                 480

Ile Phe Ser Val Leu Pro Asp Ser Phe Val Cys Asp Asp Phe Pro Lys
                485                 490                 495

Pro Gln Ile Ile Thr Gln Pro Glu Thr Thr Met Ala Val Val Gly Lys
            500                 505                 510

Asp Ile Arg Phe Thr Cys Ser Ala Ala Ser Ser Ser Ser Ser Pro Met
        515                 520                 525

Thr Phe Ala Trp Lys Lys Asp Asn Glu Val Leu Ala Asn Ala Asp Met
    530                 535                 540

Glu Asn Phe Ala His Val Arg Ala Gln Asp Gly Glu Val Met Glu Tyr
545                 550                 555                 560

Thr Thr Ile Leu His Leu Arg His Val Thr Phe Gly His Glu Gly Arg
                565                 570                 575

Tyr Gln Cys Ile Ile Thr Asn His Phe Gly Ser Thr Tyr Ser His Lys
            580                 585                 590

Ala Arg Leu Thr Val Asn Val Leu Pro Ser Phe Thr Lys Ile Pro His
        595                 600                 605

Asp Ile Ala Ile Arg Thr Gly Thr Thr Ala Arg Leu Glu Cys Ala Ala
    610                 615                 620

Thr Gly His Pro Asn Pro Gln Ile Ala Trp Gln Lys Asp Gly Gly Thr
625                 630                 635                 640

Asp Phe Pro Ala Ala Arg Glu Arg Arg Met His Val Met Pro Asp Asp
                645                 650                 655

Asp Val Phe Phe Ile Thr Asp Val Lys Ile Asp Asp Met Gly Val Tyr
            660                 665                 670

Ser Cys Thr Ala Gln Asn Ser Ala Gly Ser Val Ser Ala Asn Ala Thr
        675                 680                 685

Leu Thr Val Leu Glu Thr Pro Ser Leu Ala Val Pro Leu Glu Asp Arg
    690                 695                 700

Val Val Thr Val Gly Glu Thr Val Ala Phe Gln Cys Lys Ala Thr Gly
705                 710                 715                 720

Ser Pro Thr Pro Arg Ile Thr Trp Leu Lys Gly Gly Arg Pro Leu Ser
                725                 730                 735

Leu Thr Glu Arg His His Phe Thr Pro Gly Asn Gln Leu Leu Val Val
            740                 745                 750

Gln Asn Val Met Ile Asp Asp Ala Gly Arg Tyr Thr Cys Glu Met Ser
        755                 760                 765

Asn Pro Leu Gly Thr Glu Arg Ala His Ser Gln Leu Ser Ile Leu Pro
    770                 775                 780

Thr Pro Gly Cys Arg Lys Asp Gly Thr Thr Val Gly Ile Phe Thr Ile
785                 790                 795                 800

Ala Val Val Cys Ser Ile Val Leu Thr Ser Leu Val Trp Val Cys Ile
                805                 810                 815

Ile Tyr Gln Thr Arg Lys Lys Ser Glu Glu Tyr Ser Val Thr Asn Thr
            820                 825                 830

Asp Glu Thr Ile Val Pro Pro Asp Val Pro Ser Tyr Leu Ser Ser Gln
        835                 840                 845

Gly Thr Leu Ser Asp Arg Gln Glu Thr Val Val Arg Thr Glu Gly Gly
    850                 855                 860

His Gln Ala Asn Gly His Ile Glu Ser Asn Gly Val Cys Leu Arg Asp
865                 870                 875                 880

Pro Ser Leu Phe Pro Glu Val Asp Ile His Ser Thr Thr Cys Arg Gln
                885                 890                 895

Pro Lys Leu Cys Val Gly Tyr Thr Arg Glu Pro Trp Lys Val Thr Glu
            900                 905                 910

Lys Ala Asp Arg Thr Ala Ala Pro His Thr Thr Ala His Ser Gly Ser
        915                 920                 925

Ala Val Cys Ser Asp Cys Ser Thr Asp Thr Ala Tyr His Pro Gln Pro
    930                 935                 940

Val Pro Arg Asp Ser Gly Gln Pro Gly Thr Ala Ser Ser Gln Glu Leu
945                 950                 955                 960

Arg Gln His Asp Arg Glu Tyr Ser Pro His His Pro Tyr Ser Gly Thr
                965                 970                 975

Ala Asp Gly Ser His Thr Leu Ser Gly Gly Ser Leu Tyr Pro Ser Asn
            980                 985                 990

His Asp Arg Ile Leu Pro Ser Leu Lys Asn Lys Ala Ala Ser Ala Asp
        995                 1000                1005

Gly Asn Gly Asp Ser Ser Trp Thr Leu Ala Lys Leu His Glu Ala Asp
   1010                1015                1020

Cys Ile Asp Leu Lys Pro Ser Pro Thr Leu Ala Ser Gly Ser Pro Glu
1025                1030                1035                1040

Leu Met Glu Asp Ala Ile Ser Thr Glu Ala Gln His Leu Leu Val Ser
               1045                1050                1055

Asn Gly His Leu Pro Lys Ala Cys Asp Ser Ser Pro Glu Ser Val Pro
           1060                1065                1070

Leu Lys Gly Gln Ile Thr Gly Lys Arg Arg Gly Pro Leu Leu Leu Ala
       1075                1080                1085

Pro Arg Ser
   1090


<210>    4
<211>    3276
<212>    DNA
<213>    Mus musculus

<400>    4
atggcgcggc ccggtccggg agtgctcgga gccccgcgcc ttgcgcctcg ccttctgctc          60

tggctgctct tgctgctact gcaatggccg gagtcagcgg gcgcccaggc tcgcccgcgg 120

gccccctgcg cggccgcctg cacttgcgcc gggaactcgc tggactgcag tgggcgcggg 180

ctggcgacgc tgccccggga cctgccctcc tggacgcgca gcctaaacct gagttataac 240

agactctccg agatcgactc tgctgctttt gaggacttga cgaatctgca ggaagtgtac 300

ctcaacagca atgagctgac agccatacca tcactgggca ctgcttccat aggagttgtc 360

tctctctttt tgcagcacaa caagatcctt agtgtggatg ggagccagct gaagtcgtac 420

ctgtccttgg aagtgctgga tctgagttcc aacaacatca cggaaattcg gagctcctgt 480

ttcccgaacg gcctgcgtat aagggaactc aacttggcga gcaaccgcat cagcatcctg 540

gagtctggag catttgatgg tctgtcgcgg tcactgctga ctctccgtct gagcaaaaac 600

aggatcaccc agcttcctgt gaaagcgttc aagctaccca ggctgacaca actagacctg 660

aatcggaatc ggattcggct gattgaaggc ctcacgttcc aggggctcga cagcttagag 720

gtgctgaggc ttcagaggaa caacatcagc aggctgacgg acggggcctt ctgggggctg 780

tctaagatgc acgtgctgca cctggagtac aacagtctgg tggaagtgaa cagtggctcc 840

ctctatggcc tcacagccct gcaccagctg cacctcagca acaactccat ctctcgaatt 900

cagcgtgatg gctggagctt ctgccaaaag ctgcatgagt tgattctgtc cttcaacaac 960

ctcacgcggc tggatgagga gagtctagcg gagttgagca gcctcagtat cctgcgcctc 1020

agtcacaacg ccatcagtca cattgctgaa ggcgccttca agggactcaa gagtctgcgg 1080

gtcttggacc tggaccataa cgagatctcg ggtacaatcg aggataccag tggtgccttt 1140

acggggcttg acaacctcag caagctgact ctgtttggaa acaagatcaa atctgtggct 1200

aagagagcct tctcgggcct ggaaagcctg gaacacctga accttggaga gaatgcaatc 1260

aggtctgtcc agtttgatgc ctttgcaaag atgaagaacc ttaaagagct ctacatcagc 1320

agtgagagct tcctgtgtga ctgccagctc aagtggctgc ccccatggct aatgggtagg 1380

atgctgcagg cctttgtgac agccacctgt gcccatccag agtcgctgaa gggccagagc 1440

attttctcag tgctgccaga cagctttgtg tgtgatgact ttccaaagcc acagatcatc 1500

acccagcctg agacgaccat ggctgtggtg ggcaaggaca tccgtttcac atgctccgca 1560

gccagcagca gcagctcacc aatgaccttc gcctggaaga aggacaatga ggtcctggcc 1620

aatgcagaca tggagaactt tgcccacgtc cgtgcacagg acggcgaagt gatggagtat 1680

accactatcc tgcacctccg tcacgtcacc tttgggcacg agggccgcta ccagtgtatc 1740

atcacaaacc actttggctc cacatactcc cacaaagcca ggctcactgt gaatgtgttg 1800

ccatcattca ctaaaatacc ccatgacatt gccatccgga ctggcaccac agcccgcctc 1860

gagtgtgctg ccacgggcca ccctaaccct cagattgcct ggcagaagga tggaggcacc 1920

gatttcccgg cagctcgtga gcgacgcatg catgttatgc cagacgatga tgtgttcttc 1980

atcactgatg tgaaaataga cgacatgggg gtctacagct gcactgccca gaactcggca 2040

ggctcggttt cagccaacgc taccctcaca gtcttagaaa ctccatcctt ggcagtgcct 2100

ctggaagacc gtgtggtaac tgtgggagaa acagtggcct tccagtgcaa agcaaccggg 2160

agccccacac cacgcatcac ctggcttaag ggaggtcgcc cattgagcct cacagagcgc 2220

caccatttca ctccaggcaa ccagctgctg gttgttcaga atgtgatgat agacgatgca 2280

gggcggtata cctgtgagat gtctaatccc ctgggcactg agcgagcaca tagccagctg 2340

agcattttac ctacccctgg ctgccggaag gatgggacca ccgtaggcat cttcaccatt 2400

gctgtggtgt gcagtattgt cctgacctcg ctggtgtggg tgtgcatcat ctaccagacc 2460

aggaagaaga gcgaggagta cagcgtcact aacacagatg aaaccattgt gcctccagat 2520

gttccaagct acctctcctc tcagggaacc ctttctgacc ggcaggaaac tgtggtcagg 2580

actgagggtg gtcatcaggc caatgggcac attgaaagca atggtgtgtg tctgagagac 2640

ccgagcctct tcccagaggt cgacattcat agcactacat gtaggcagcc caagctgtgt 2700

gttggataca ctagagagcc ctggaaggtg acggagaaag ccgacaggac agctgcccca 2760

catacaacag cacacagtgg ctctgctgta tgtagtgact gcagtactga cacagcctac 2820

catccccagc ctgtgcccag agacagtggg cagccaggca cagcgagcag ccaagagctc 2880

aggcagcatg accgggaata ttctccacac catccttaca gtgggactgc ggatgggtct 2940

cataccctct ctggggggtc cctctatcca agcaaccatg acagaatact gccatccttg 3000

aagaacaagg cggcgtctgc agatgggaac ggagattcct cttggacttt agcaaagtta 3060

catgaagcag actgcataga cctaaagcct tctcctacgt tagcttcagg cagcccagag 3120

ctcatggaag acgccatatc tactgaagcc cagcacttgc ttgtttccaa tggccacctc 3180

cccaaggcct gtgactccag ccctgaatct gtgccactga aagggcagat cactgggaaa 3240

cggaggggac cactgctatt ggcaccgaga agctag 3276

<210> 5
<211> 762
<212> PRT
<213> Homo sapiens

<400> 5
Ala Gly Pro Arg Ala Pro Cys Ala Ala Ala Cys Thr Cys Ala Gly Asp
1               5                   10                  15

Ser Leu Asp Cys Gly Gly Arg Gly Leu Ala Ala Leu Pro Gly Asp Leu
            20                  25                  30

Pro Ser Trp Thr Arg Ser Leu Asn Leu Ser Tyr Asn Lys Leu Ser Glu
        35                  40                  45

Ile Asp Pro Ala Gly Phe Glu Asp Leu Pro Asn Leu Gln Glu Val Tyr
    50                  55                  60

Leu Asn Asn Asn Glu Leu Thr Ala Val Pro Ser Leu Gly Ala Ala Ser
65                  70                  75                  80

Ser His Val Val Ser Leu Phe Leu Gln His Asn Lys Ile Arg Ser Val
                85                  90                  95

Glu Gly Ser Gln Leu Lys Ala Tyr Leu Ser Leu Glu Val Leu Asp Leu
            100                 105                 110

Ser Leu Asn Asn Ile Thr Glu Val Arg Asn Thr Cys Phe Pro His Gly
        115                 120                 125

Pro Pro Ile Lys Glu Leu Asn Leu Ala Gly Asn Arg Ile Gly Thr Leu
    130                 135                 140

Glu Leu Gly Ala Phe Asp Gly Leu Ser Arg Ser Leu Leu Thr Leu Arg
145                 150                 155                 160

Leu Ser Lys Asn Arg Ile Thr Gln Leu Pro Val Arg Ala Phe Lys Leu
                165                 170                 175

Pro Arg Leu Thr Gln Leu Asp Leu Asn Arg Asn Arg Ile Arg Leu Ile
            180                 185                 190

Glu Gly Leu Thr Phe Gln Gly Leu Asn Ser Leu Glu Val Leu Lys Leu
        195                 200                 205

Gln Arg Asn Asn Ile Ser Lys Leu Thr Asp Gly Ala Phe Trp Gly Leu
    210                 215                 220

Ser Lys Met His Val Leu His Leu Glu Tyr Asn Ser Leu Val Glu Val
225                 230                 235                 240

Asn Ser Gly Ser Leu Tyr Gly Leu Thr Ala Leu His Gln Leu His Leu
                245                 250                 255

Ser Asn Asn Ser Ile Ala Arg Ile His Arg Lys Gly Trp Ser Phe Cys
            260                 265                 270

Gln Lys Leu His Glu Leu Val Leu Ser Phe Asn Asn Leu Thr Arg Leu
        275                 280                 285

Asp Glu Glu Ser Leu Ala Glu Leu Ser Ser Leu Ser Val Leu Arg Leu
    290                 295                 300

Ser His Asn Ser Ile Ser His Ile Ala Glu Gly Ala Phe Lys Gly Leu
305                 310                 315                 320

Arg Ser Leu Arg Val Leu Asp Leu Asp His Asn Glu Ile Ser Gly Thr
                325                 330                 335

Ile Glu Asp Thr Ser Gly Ala Phe Ser Gly Leu Asp Ser Leu Ser Lys
            340                 345                 350

Leu Thr Leu Phe Gly Asn Lys Ile Lys Ser Val Ala Lys Arg Ala Phe
        355                 360                 365

Ser Gly Leu Glu Gly Leu Glu His Leu Asn Leu Gly Gly Asn Ala Ile
    370                 375                 380

Arg Ser Val Gln Phe Asp Ala Phe Val Lys Met Lys Asn Leu Lys Glu
385                 390                 395                 400

Leu His Ile Ser Ser Asp Ser Phe Leu Cys Asp Cys Gln Leu Lys Trp
                405                 410                 415

Leu Pro Pro Trp Leu Ile Gly Arg Met Leu Gln Ala Phe Val Thr Ala
            420                 425                 430

Thr Cys Ala His Pro Glu Ser Leu Lys Gly Gln Ser Ile Phe Ser Val
        435                 440                 445

Pro Pro Glu Ser Phe Val Cys Asp Asp Phe Leu Lys Pro Gln Ile Ile
    450                 455                 460

Thr Gln Pro Glu Thr Thr Met Ala Met Val Gly Lys Asp Ile Arg Phe
465                 470                 475                 480

Thr Cys Ser Ala Ala Ser Ser Ser Ser Ser Pro Met Thr Phe Ala Trp
                485                 490                 495

Lys Lys Asp Asn Glu Val Leu Thr Asn Ala Asp Met Glu Asn Phe Val
            500                 505                 510

His Val His Ala Gln Asp Gly Glu Val Met Glu Tyr Thr Thr Ile Leu
        515                 520                 525

His Leu Arg Gln Val Thr Phe Gly His Glu Gly Arg Tyr Gln Cys Val
    530                 535                 540

Ile Thr Asn His Phe Gly Ser Thr Tyr Ser His Lys Ala Arg Leu Thr
545                 550                 555                 560

Val Asn Val Leu Pro Ser Phe Thr Lys Thr Pro His Asp Ile Thr Ile
                565                 570                 575

Arg Thr Thr Thr Val Ala Arg Leu Glu Cys Ala Ala Thr Gly His Pro
            580                 585                 590

Asn Pro Gln Ile Ala Trp Gln Lys Asp Gly Gly Thr Asp Phe Pro Ala
        595                 600                 605

Ala Arg Glu Arg Arg Met His Val Met Pro Asp Asp Asp Val Phe Phe
    610                 615                 620

Ile Thr Asp Val Lys Ile Asp Asp Ala Gly Val Tyr Ser Cys Thr Ala
625                 630                 635                 640

Gln Asn Ser Ala Gly Ser Ile Ser Ala Asn Ala Thr Leu Thr Val Leu
                645                 650                 655

Glu Thr Pro Ser Leu Val Val Pro Leu Glu Asp Arg Val Val Ser Val
            660                 665                 670

Gly Glu Thr Val Ala Leu Gln Cys Lys Ala Thr Gly Asn Pro Pro Pro
        675                 680                 685

Arg Ile Thr Trp Phe Lys Gly Asp Arg Pro Leu Ser Leu Thr Glu Arg
    690                 695                 700

His His Leu Thr Pro Asp Asn Gln Leu Leu Val Val Gln Asn Val Val
705                 710                 715                 720

Ala Glu Asp Ala Gly Arg Tyr Thr Cys Glu Met Ser Asn Thr Leu Gly
                725                 730                 735

Thr Glu Arg Ala His Ser Gln Leu Ser Val Leu Pro Ala Ala Gly Cys
            740                 745                 750

Arg Lys Asp Gly Thr Thr Val Gly Ile Phe
        755                 760

<210>   6
<211>   760
<212>   PRT
<213>   Mus musculus

<400>   6
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser

260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe

595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr
        755                 760


<210>    7
<211>    29
<212>    PRT
<213>    Artificial Sequence

<220>
<223>    Epitope


<400>    7
Thr Arg Ser Leu Asn Leu Ser Tyr Asn Lys Leu Ser Glu Ile Asp Pro
 1               5                  10                  15

Ala Gly Phe Glu Asp Leu Pro Asn Leu Gln Glu Val Tyr
            20                  25


<210>    8
<211>    31
<212>    PRT
<213>    Artificial Sequence

<220>
<223>    Epitope


<400>    8
Tyr Leu Ser Leu Glu Val Leu Asp Leu Ser Leu Asn Asn Ile Thr Glu
 1               5                  10                  15

Val Arg Asn Thr Cys Phe Pro His Gly Pro Pro Ile Lys Glu Leu
            20                  25                  30

<210>    9
<211>    3
<212>    PRT
<213>    Artificial Sequence

<220>
<223>    Epitope

<400>    9
His Gln Leu
1

<210>    10
<211>    19
<212>    PRT
<213>    Artificial Sequence

<220>
<223>    Epitope

<400>    10
Asp His Asn Glu Ile Ser Gly Thr Ile Glu Asp Thr Ser Gly Ala Phe
1               5                   10                  15

Ser Gly Leu

<210>    11
<211>    7
<212>    PRT
<213>    Artificial Sequence

<220>
<223>    Epitope

<400>    11
His Leu Arg Gln Val Thr Phe
1               5

<210>    12
<211>    38
<212>    PRT
<213>    Artificial Sequence

<220>
<223>    Epitope

<400>    12
Gln Lys Asp Gly Gly Thr Asp Phe Pro Ala Ala Arg Glu Arg Arg Met
1               5                   10                  15

His Val Met Pro Asp Asp Asp Val Phe Phe Ile Thr Asp Val Lys Ile
            20                  25                  30

Asp Asp Ala Gly Val Tyr
        35



<210>   13
<211>   36
<212>   PRT
<213>   Artificial Sequence

<220>
<223>   Epitope


<400>   13
Ser Leu Thr Glu Arg His His Leu Thr Pro Asp Asn Gln Leu Leu Val
 1               5                  10                  15

Val Gln Asn Val Val Ala Glu Asp Ala Gly Arg Tyr Thr Cys Glu Met
            20                  25                  30

Ser Asn Thr Leu
        35



<210>   14
<211>   71
<212>   PRT
<213>   Artificial Sequence

<220>
<223>   Epitope


<400>   14
Gly Leu Ala Ala Leu Pro Gly Asp Leu Pro Ser Trp Thr Arg Ser Leu
 1               5                  10                  15

Asn Leu Ser Tyr Asn Lys Leu Ser Glu Ile Asp Pro Ala Gly Phe Glu
            20                  25                  30

Asp Leu Pro Asn Leu Gln Glu Val Tyr Leu Asn Asn Asn Glu Leu Thr
        35                  40                  45

Ala Val Pro Ser Leu Gly Ala Ala Ser Ser His Val Val Ser Leu Phe
    50                  55                  60

Leu Gln His Asn Lys Ile Arg
65                  70


<210>   15
<211>   14
<212>   PRT
<213>   Artificial Sequence

<220>
<223>   Epitope


<400>   15

Ile Gly Thr Leu Glu Leu Gly Ala Phe Asp Gly Leu Ser Arg
1               5                   10

<210>   16
<211>   8
<212>   PRT
<213>   Artificial Sequence

<220>
<223>   Epitope

<400>   16
Leu Thr Gln Leu Asp Leu Asn Arg
1               5

<210>   17
<211>   11
<212>   PRT
<213>   Artificial Sequence

<220>
<223>   Epitope

<400>   17
Leu Thr Asp Gly Ala Phe Trp Gly Leu Ser Lys
1               5                   10

<210>   18
<211>   9
<212>   PRT
<213>   Artificial Sequence

<220>
<223>   Epitope

<400>   18
Gln Val Thr Phe Gly His Glu Gly Arg
1               5

<210>   19
<211>   11
<212>   PRT
<213>   Artificial Sequence

<220>
<223>   Epitope

<400>   19
Leu Thr Val Asn Val Leu Pro Ser Phe Thr Lys
1               5                   10

<210>   20
<211>   16
<212>   PRT

<213>    Artificial Sequence

<220>
<223>    Epitope


<400>    20
Met His Val Met Pro Asp Asp Asp Val Phe Phe Ile Thr Asp Val Lys
1               5                   10                  15




<210>    21
<211>    5
<212>    PRT
<213>    Artificial Sequence

<220>
<223>    Epitope


<400>    21
Ile Thr Trp Phe Lys
1               5


<210>    22
<211>    28
<212>    PRT
<213>    Artificial Sequence

<220>
<223>    Epitope


<400>    22
Tyr Thr Cys Glu Met Ser Asn Thr Leu Gly Thr Glu Arg Ala His Ser
1               5                   10                  15

Gln Leu Ser Val Leu Pro Ala Ala Gly Cys Arg Lys
            20                  25


<210>    23
<211>    18
<212>    PRT
<213>    Artificial Sequence

<220>
<223>    Epitope


<400>    23
Ala Gly Pro Arg Ala Pro Cys Ala Ala Ala Cys Thr Cys Ala Gly Asp
1               5                   10                  15

Ser Leu



<210>    24
<211>    25
<212>    PRT

<213>    Artificial Sequence

<220>
<223>    Epitope


<400>    24
Asp Leu Pro Ser Trp Thr Arg Ser Leu Asn Leu Ser Tyr Asn Lys Leu
1               5                   10                  15

Ser Glu Ile Asp Pro Ala Gly Phe Glu
            20                  25


<210>    25
<211>    30
<212>    PRT
<213>    Artificial Sequence

<220>
<223>    Epitope


<400>    25
Asp Glu Glu Ser Leu Ala Glu Leu Ser Ser Leu Ser Val Leu Arg Leu
1               5                   10                  15

Ser His Asn Ser Ile Ser His Ile Ala Glu Gly Ala Phe Lys
            20                  25                  30


<210>    26
<211>    20
<212>    PRT
<213>    Artificial Sequence

<220>
<223>    Epitope


<400>    26
Lys Asp Asn Glu Val Leu Thr Asn Ala Asp Met Glu Asn Phe Val His
1               5                   10                  15

Val His Ala Gln
            20


<210>    27
<211>    54
<212>    PRT
<213>    Artificial Sequence

<220>
<223>    Epitope


<400>    27
Asp Ile Thr Ile Arg Thr Thr Thr Val Ala Arg Leu Glu Cys Ala Ala
1               5                   10                  15

Thr Gly His Pro Asn Pro Gln Ile Ala Trp Gln Lys Asp Gly Gly Thr
            20                  25                  30

Asp Phe Pro Ala Ala Arg Glu Arg Arg Met His Val Met Pro Asp Asp
        35                  40                  45

Asp Val Phe Phe Ile Thr
    50

<210>    28
<211>    28
<212>    PRT
<213>    Artificial Sequence

<220>
<223>    Epitope

<400>    28
Gly Asp Arg Pro Leu Ser Leu Thr Glu Arg His His Leu Thr Pro Asp
1               5                   10                  15

Asn Gln Leu Leu Val Val Gln Asn Val Val Ala Glu
            20                  25

<210>    29
<211>    29
<212>    PRT
<213>    Artificial Sequence

<220>
<223>    Epitope

<400>    29
Thr Arg Ser Leu Asn Leu Ser Tyr Asn Lys Leu Ser Glu Ile Asp Pro
1               5                   10                  15

Ala Gly Phe Glu Asp Leu Pro Asn Leu Gln Glu Val Tyr
            20                  25

<210>    30
<211>    2
<212>    PRT
<213>    Artificial Sequence

<220>
<223>    Epitope

<400>    30
Gln Lys
100

<210>    31
<211>    14
<212>    PRT
<213>    Artificial Sequence

<220>
<223>    Epitope

<400>    31
Glu Arg Arg Met His Val Met Pro Asp Asp Asp Val Phe Phe
1               5                   10

<210>    32
<211>    23
<212>    PRT
<213>    Artificial Sequence

<220>
<223>    Epitope

<400>    32
Ala Met Val Gly Lys Asp Ile Arg Phe Thr Cys Ser Ala Ala Ser Ser
1               5                   10                  15

Ser Ser Ser Pro Met Thr Phe
            20

<210>    33
<211>    17
<212>    PRT
<213>    Artificial Sequence

<220>
<223>    Epitope

<400>    33
Ala Trp Lys Lys Asp Asn Glu Val Leu Thr Asn Ala Asp Met Glu Asn
1               5                   10                  15

Phe

<210>    34
<211>    6
<212>    PRT
<213>    Artificial Sequence

<220>
<223>    Epitope

<400>    34
Thr Thr Ile Leu His Leu
1               5

<210>    35
<211>    8
<212>    PRT
<213>    Artificial Sequence

<220>
<223>    Epitope

<400>    35
Thr Val Asn Val Leu Pro Ser Phe
1               5

<210>    36
<211>    12
<212>    PRT
<213>    Artificial Sequence

<220>
<223>    Epitope

<400>    36
Thr Lys Thr Pro His Asp Ile Thr Ile Arg Thr Thr
1               5                   10

<210>    37
<211>    14
<212>    PRT
<213>    Artificial Sequence

<220>
<223>    Epitope

<400>    37
Cys Lys Ala Thr Gly Asn Pro Pro Pro Arg Ile Thr Trp Phe
1               5                   10

<210>    38
<211>    18
<212>    PRT
<213>    Artificial Sequence

<220>
<223>    Epitope

<400>    38
Ser Val Leu Pro Ala Ala Gly Cys Arg Lys Asp Gly Thr Thr Val Gly
1               5                   10                  15

Ile Phe

<210>    39
<211>    4
<212>    PRT
<213>    Artificial Sequence

<220>
<223>    Epitope

<400>    39
Ala Gly Pro Arg
1

<210>    40
<211>    47
<212>    PRT
<213>    Artificial Sequence

<220>
<223>    Epitope


<400>    40
Leu Ser Glu Ile Asp Pro Ala Gly Phe Glu Asp Leu Pro Asn Leu Gln
1               5                   10                  15

Glu Val Tyr Leu Asn Asn Asn Glu Leu Thr Ala Val Pro Ser Leu Gly
            20                  25                  30

Ala Ala Ser Ser His Val Val Ser Leu Phe Leu Gln His Asn Lys
        35                  40                  45




<210>    41
<211>    9
<212>    PRT
<213>    Artificial Sequence

<220>
<223>    Epitope


<400>    41
Asn Arg Ile Thr Gln Leu Pro Val Arg
1               5


<210>    42
<211>    11
<212>    PRT
<213>    Artificial Sequence

<220>
<223>    Epitope


<400>    42
Ile His Arg Lys Gly Trp Ser Phe Cys Gln Lys
1               5                   10


<210>    43
<211>    8
<212>    PRT
<213>    Artificial Sequence

<220>
<223>    Epitope


<400>    43
Lys Gly Trp Ser Phe Cys Gln Lys

1               5


<210>   44
<211>   7
<212>   PRT
<213>   Artificial Sequence

<220>
<223>   Epitope


<400>   44
Gly Trp Ser Phe Cys Gln Lys
1               5


<210>   45
<211>   13
<212>   PRT
<213>   Artificial Sequence

<220>
<223>   Epitope


<400>   45
Leu His Glu Leu Val Leu Ser Phe Asn Asn Leu Thr Arg
1               5                   10


<210>   46
<211>   29
<212>   PRT
<213>   Artificial Sequence

<220>
<223>   Epitope


<400>   46
Leu His Glu Leu Val Leu Ser Phe Asn Asn Leu Thr Arg Leu Asp Glu
1               5                   10                  15

Glu Ser Leu Ala Glu Leu Ser Ser Leu Ser Val Leu Arg
            20                  25


<210>   47
<211>   9
<212>   PRT
<213>   Artificial Sequence

<220>
<223>   Epitope


<400>   47
Ser Val Gln Phe Asp Ala Phe Val Lys
1               5


<210>   48

<211>    30
<212>    PRT
<213>    Artificial Sequence

<220>
<223>    Epitope


<400>    48
Met Lys Asn Leu Lys Glu Leu His Ile Ser Ser Asp Ser Phe Leu Cys
1               5                   10                  15

Asp Cys Gln Leu Lys Trp Leu Pro Pro Trp Leu Ile Gly Arg
            20                  25                  30


<210>    49
<211>    17
<212>    PRT
<213>    Artificial Sequence

<220>
<223>    Epitope


<400>    49
Met Leu Gln Ala Phe Val Thr Ala Thr Cys Ala His Pro Glu Ser Leu
1               5                   10                  15

Lys



<210>    50
<211>    38
<212>    PRT
<213>    Artificial Sequence

<220>
<223>    Epitope


<400>    50
Gly Gln Ser Ile Phe Ser Val Pro Pro Glu Ser Phe Val Cys Asp Asp
1               5                   10                  15

Phe Leu Lys Pro Gln Ile Ile Thr Gln Pro Glu Thr Thr Met Ala Met
            20                  25                  30

Val Gly Lys Asp Ile Arg
        35


<210>    51
<211>    19
<212>    PRT
<213>    Artificial Sequence

<220>
<223>    Epitope


<400>    51

Phe Thr Cys Ser Ala Ala Ser Ser Ser Ser Ser Pro Met Thr Phe Ala
1               5                   10                  15

Trp Lys Lys

<210>   52
<211>   34
<212>   PRT
<213>   Artificial Sequence

<220>
<223>   Epitope

<400>   52
Lys Asp Asn Glu Val Leu Thr Asn Ala Asp Met Glu Asn Phe Val His
1               5                   10                  15

Val His Ala Gln Asp Gly Glu Val Met Glu Tyr Thr Thr Ile Leu His
            20                  25                  30

Leu Arg

<210>   53
<211>   9
<212>   PRT
<213>   Artificial Sequence

<220>
<223>   Epitope

<400>   53
Gln Val Thr Phe Gly His Glu Gly Arg
1               5

<210>   54
<211>   16
<212>   PRT
<213>   Artificial Sequence

<220>
<223>   Epitope

<400>   54
Tyr Gln Cys Val Ile Thr Asn His Phe Gly Ser Thr Tyr Ser His Lys
1               5                   10                  15

<210>   55
<211>   15
<212>   PRT
<213>   Artificial Sequence

<220>
<223>   Epitope

<400>    55
His Val Met Pro Asp Asp Asp Val Phe Phe Ile Thr Asp Val Lys
1               5                   10                  15

<210>    56
<211>    57
<212>    PRT
<213>    Artificial Sequence

<220>
<223>    Epitope

<400>    56
Leu Ser Glu Ile Asp Pro Ala Gly Phe Glu Asp Leu Pro Asn Leu Gln
1               5                   10                  15

Glu Val Tyr Leu Asn Asn Asn Glu Leu Thr Ala Val Pro Ser Leu Gly
            20                  25                  30

Ala Ala Ser Ser His Val Val Ser Leu Phe Leu Gln His Asn Lys Ile
        35                  40                  45

Arg Ser Val Glu Gly Ser Gln Leu Lys
    50                  55

<210>    57
<211>    10
<212>    PRT
<213>    Artificial Sequence

<220>
<223>    Epitope

<400>    57
Ile Arg Ser Val Glu Gly Ser Gln Leu Lys
1               5                   10

<210>    58
<211>    14
<212>    PRT
<213>    Artificial Sequence

<220>
<223>    Epitope

<400>    58
Asp Gly Leu Ser Arg Ser Leu Leu Thr Leu Arg Leu Ser Lys
1               5                   10

<210>    59
<211>    19
<212>    PRT
<213>    Artificial Sequence

<220>
<223>    Epitope

<400>    59
Asn Arg Ile Thr Gln Leu Pro Val Arg Ala Phe Lys Leu Pro Arg Leu
1               5                   10                  15

Thr Gln Leu

<210>    60
<211>    11
<212>    PRT
<213>    Artificial Sequence

<220>
<223>    Epitope

<400>    60
Leu Thr Asp Gly Ala Phe Trp Gly Leu Ser Lys
1               5                   10

<210>    61
<211>    41
<212>    PRT
<213>    Artificial Sequence

<220>
<223>    Epitope

<400>    61
Met His Val Leu His Leu Glu Tyr Asn Ser Leu Val Glu Val Asn Ser
1               5                   10                  15

Gly Ser Leu Tyr Gly Leu Thr Ala Leu His Gln Leu His Leu Ser Asn
            20                  25                  30

Asn Ser Ile Ala Arg Ile His Arg Lys
        35                  40

<210>    62
<211>    7
<212>    PRT
<213>    Artificial Sequence

<220>
<223>    Epitope

<400>    62
Gly Trp Ser Phe Cys Gln Lys
1               5

<210>    63
<211>    43
<212>    PRT

<213>    Artificial Sequence

<220>
<223>    Epitope


<400>    63
Gly Leu Arg Ser Leu Arg Val Leu Asp Leu Asp His Asn Glu Ile Ser
1               5                   10                  15

Gly Thr Ile Glu Asp Thr Ser Gly Ala Phe Ser Gly Leu Asp Ser Leu
            20                  25                  30

Ser Lys Leu Thr Leu Phe Gly Asn Lys Ile Lys
        35                  40


<210>    64
<211>    5
<212>    PRT
<213>    Artificial Sequence

<220>
<223>    Epitope


<400>    64
Asp Cys Gln Leu Lys
1               5


<210>    65
<211>    21
<212>    PRT
<213>    Artificial Sequence

<220>
<223>    Epitope


<400>    65
Asp Asp Phe Leu Lys Pro Gln Ile Ile Thr Gln Pro Glu Thr Thr Met
1               5                   10                  15

Ala Met Val Gly Lys
            20


<210>    66
<211>    22
<212>    PRT
<213>    Artificial Sequence

<220>
<223>    Epitope


<400>    66
Asp Ile Arg Phe Thr Cys Ser Ala Ala Ser Ser Ser Ser Ser Pro Met
1               5                   10                  15

Thr Phe Ala Trp Lys Lys
            20

<210>    67
<211>    9
<212>    PRT
<213>    Artificial Sequence

<220>
<223>    Epitope


<400>    67
Lys Asp Asn Glu Val Leu Thr Asn Ala
1               5


<210>    68
<211>    19
<212>    PRT
<213>    Artificial Sequence

<220>
<223>    Epitope


<400>    68
Glu Gly Arg Tyr Gln Cys Val Ile Thr Asn His Phe Gly Ser Thr Tyr
1               5                   10                  15

Ser His Lys



<210>    69
<211>    16
<212>    PRT
<213>    Artificial Sequence

<220>
<223>    Epitope


<400>    69
Ala Arg Leu Thr Val Asn Val Leu Pro Ser Phe Thr Lys Thr Pro His
1               5                   10                  15



<210>    70
<211>    44
<212>    PRT
<213>    Artificial Sequence

<220>
<223>    Epitope


<400>    70
Ala Arg Leu Thr Val Asn Val Leu Pro Ser Phe Thr Lys Thr Pro His
1               5                   10                  15

Asp Ile Thr Ile Arg Thr Thr Thr Val Ala Arg Leu Glu Cys Ala Ala
            20                  25                  30

Thr Gly His Pro Asn Pro Gln Ile Ala Trp Gln Lys
        35                  40

<210>   71
<211>   42
<212>   PRT
<213>   Artificial Sequence

<220>
<223>   Epitope

<400>   71
Leu Ser Glu Ile Asp Pro Ala Gly Phe Glu Asp Leu Pro Asn Leu Gln
 1               5                  10                  15

Glu Val Tyr Leu Asn Asn Asn Glu Leu Thr Ala Val Pro Ser Leu Gly
            20                  25                  30

Ala Ala Ser Ser His Val Val Ser Leu Phe
        35                  40

<210>   72
<211>   19
<212>   PRT
<213>   Artificial Sequence

<220>
<223>   Epitope

<400>   72
Leu Ser Glu Ile Asp Pro Ala Gly Phe Glu Asp Leu Pro Asn Leu Gln
 1               5                  10                  15

Glu Val Tyr

<210>   73
<211>   24
<212>   DNA
<213>   Artificial Sequence

<220>
<223>   forward primer

<400>   73
gacggaattc agtgaggaga acct                                              24

<210>   74
<211>   24
<212>   DNA
<213>   Artificial Sequence

<220>
<223>   reverse primer

<400>    74
caactggtag tggcagcttg tagg                                              24


<210>    75
<211>    24
<212>    DNA
<213>    Artificial Sequence

<220>
<223>    forward primer


<400>    75
tcacaaggaa cattgtctga acca                                              24


<210>    76
<211>    24
<212>    DNA
<213>    Artificial Sequence

<220>
<223>    reverse primer


<400>    76
gcctgatcta acacatcctc ctca                                              24


<210>    77
<211>    24
<212>    DNA
<213>    Artificial Sequence

<220>
<223>    forward primer


<400>    77
cagcaccttg agctgaacag aaac                                              24


<210>    78
<211>    24
<212>    DNA
<213>    Artificial Sequence

<220>
<223>    reverse primer


<400>    78
ccagcctttg gtaatctcgg ttag                                              24


<210>    79
<211>    24
<212>    DNA
<213>    Artificial Sequence

<220>

<223>    forward primer


<400>    79
ctttcaccta tcccaccctt atcc                                              24


<210>    80
<211>    24
<212>    DNA
<213>    Artificial Sequence

<220>
<223>    reverse primer


<400>    80
attcatctac ggtccacact gctc                                              24


<210>    81
<211>    20
<212>    DNA
<213>    Artificial Sequence

<220>
<223>    forward primer


<400>    81
ggcgtcatgg tgggcatggg                                                   20


<210>    82
<211>    20
<212>    DNA
<213>    Artificial Sequence

<220>
<223>    reverse primer


<400>    82
atggcgtggg gaagggcgta                                                   20


<210>    83
<211>    8
<212>    PRT
<213>    Artificial Sequence

<220>
<223>    GTC210-01 heavy chain CDR1


<400>    83
Gly Phe Thr Phe Ser Gly Tyr Asp
1               5


<210>    84
<211>    9
<212>    PRT

<213>    Artificial Sequence

<220>
<223>    GTC210-01 heavy chain CDR2


<400>    84
Leu Ile Tyr Pro Asp Ser Gly Asn Lys
1               5


<210>    85
<211>    14
<212>    PRT
<213>    Artificial Sequence

<220>
<223>    GTC210-01 heavy chain CDR3


<400>    85
Ala Arg Asp Ala Gly Leu Ser Trp Ala Gly Ala Phe Asp Tyr
1               5                   10


<210>    86
<211>    8
<212>    PRT
<213>    Artificial Sequence

<220>
<223>    GTC210-01 light chain CDR1


<400>    86
Ser Ser Asn Ile Gly Ser Asn Tyr
1               5


<210>    87
<211>    3
<212>    PRT
<213>    Artificial Sequence

<220>
<223>    GTC210-01 light chain CDR2


<400>    87
Ser Asp Ser
1


<210>    88
<211>    11
<212>    PRT
<213>    Artificial Sequence

<220>
<223>    GTC210-01 light chain CDR3


<400>    88

Gly Ser Trp Asp Tyr Ser Leu Ser Ala Tyr Val
1               5                   10

<210>   89
<211>   8
<212>   PRT
<213>   Artificial Sequence

<220>
<223>   GTC210-02 heavy chain CDR1

<400>   89
Gly Phe Thr Phe Ser Asn Tyr Tyr
1               5

<210>   90
<211>   9
<212>   PRT
<213>   Artificial Sequence

<220>
<223>   GTC210-02 heavy chain CDR2

<400>   90
Gly Ile Ser Pro Gly Asp Ser Ser Thr
1               5

<210>   91
<211>   14
<212>   PRT
<213>   Artificial Sequence

<220>
<223>   GTC210-02 heavy chain CDR3

<400>   91
Ala Lys Gly Leu Tyr Ser Asn Pro Asn Glu Pro Phe Asp Tyr
1               5                   10

<210>   92
<211>   8
<212>   PRT
<213>   Artificial Sequence

<220>
<223>   GTC210-02 light chain CDR1

<400>   92
Ser Ser Asn Ile Gly Ser Asn Tyr
1               5

<210>   93
<211>   3
<212>   PRT

<213>    Artificial Sequence

<220>
<223>    GTC210-02 light chain CDR2


<400>    93
Asp Asp Ser
1


<210>    94
<211>    11
<212>    PRT
<213>    Artificial Sequence

<220>
<223>    GTC210-02 light chain CDR3


<400>    94
Gly Thr Trp Asp Tyr Ser Leu Asn Gly Tyr Val
1               5                   10


<210>    95
<211>    8
<212>    PRT
<213>    Artificial Sequence

<220>
<223>    GTC210-03 heavy chain CDR1


<400>    95
Gly Phe Thr Phe Ser Ser Tyr Asp
1               5


<210>    96
<211>    9
<212>    PRT
<213>    Artificial Sequence

<220>
<223>    GTC210-03 heavy chain CDR2


<400>    96
Gly Ile Ser Pro Asp Gly Ser Asn Ile
1               5


<210>    97
<211>    20
<212>    PRT
<213>    Artificial Sequence

<220>
<223>    GTC210-03 heavy chain CDR3


<400>    97

Ala Lys Val Gly Leu Arg Cys Arg Tyr Glu Ala Cys Ser Tyr Ala Tyr
1               5                   10                  15

Gly Met Asp Val
            20

<210>    98
<211>    8
<212>    PRT
<213>    Artificial Sequence

<220>
<223>    GTC210-03 light chain CDR1

<400>    98
Ser Ser Asn Ile Gly Ser Asn Tyr
1               5

<210>    99
<211>    3
<212>    PRT
<213>    Artificial Sequence

<220>
<223>    GTC210-03 light chain CDR2

<400>    99
Ser Asp Ser
1

<210>    100
<211>    11
<212>    PRT
<213>    Artificial Sequence

<220>
<223>    GTC210-03 light chain CDR3

<400>    100
Ala Thr Trp Asp Ser Ser Leu Asn Gly Tyr Val
1               5                   10

<210>    101
<211>    8
<212>    PRT
<213>    Artificial Sequence

<220>
<223>    GTC210-04 heavy chain CDR1

<400>    101
Gly Phe Thr Phe Ser Asn Tyr Asp
1               5

<210>    102
<211>    9
<212>    PRT
<213>    Artificial Sequence

<220>
<223>    GTC210-04 heavy chain CDR2


<400>    102
Ser Ile Ser Pro Ser Ser Gly Ser Ile
1               5


<210>    103
<211>    14
<212>    PRT
<213>    Artificial Sequence

<220>
<223>    GTC210-04 heavy chain CDR3


<400>    103
Ala Lys Asp Leu Asp Ala Phe Trp Arg Pro Ser Phe Asp Tyr
1               5                   10


<210>    104
<211>    8
<212>    PRT
<213>    Artificial Sequence

<220>
<223>    GTC210-04 light chain CDR1


<400>    104
Ser Ser Asn Ile Gly Asn Asn Asn
1               5


<210>    105
<211>    3
<212>    PRT
<213>    Artificial Sequence

<220>
<223>    GTC210-04 light chain CDR2


<400>    105
Ser Asp Ser
1


<210>    106
<211>    11
<212>    PRT
<213>    Artificial Sequence

<220>
<223>    GTC210-04 light chain CDR3

<400>    106
Gly Ser Trp Asp Asp Ser Leu Ser Ala Tyr Val
1               5                   10

<210>    107
<211>    8
<212>    PRT
<213>    Artificial Sequence

<220>
<223>    GTC110-01 heavy chain CDR1

<400>    107
Gly Phe Thr Phe Ser Asp Tyr Asp
1               5

<210>    108
<211>    9
<212>    PRT
<213>    Artificial Sequence

<220>
<223>    GTC110-01 heavy chain CDR2

<400>    108
Trp Ile Ser His Gly Gly Gly Ser Ile
1               5

<210>    109
<211>    20
<212>    PRT
<213>    Artificial Sequence

<220>
<223>    GTC110-01 heavy chain CDR3

<400>    109
Ala Arg Gly Leu Gly Leu Cys Lys Thr Gly Leu Cys Tyr Tyr Tyr Asp
1               5                   10                  15

Ala Met Asp Val
            20

<210>    110
<211>    8
<212>    PRT
<213>    Artificial Sequence

<220>
<223>    GTC110-01 light chain CDR1

<400>    110
Ser Ser Asn Ile Gly Asn Asn Ser

1                   5


<210>    111
<211>    3
<212>    PRT
<213>    Artificial Sequence

<220>
<223>    GTC110-01 light chain CDR2


<400>    111
Ala Asp Asn
1


<210>    112
<211>    11
<212>    PRT
<213>    Artificial Sequence

<220>
<223>    GTC110-01 light chain CDR3


<400>    112
Ala Ala Trp Asp Ser Ser Leu Ser Ala Tyr Val
1               5                   10


<210>    113
<211>    8
<212>    PRT
<213>    Artificial Sequence

<220>
<223>    GTC110-02 heavy chain CDR1


<400>    113
Gly Phe Thr Phe Ser Asp Tyr Tyr
1               5


<210>    114
<211>    9
<212>    PRT
<213>    Artificial Sequence

<220>
<223>    GTC110-02 heavy chain CDR2


<400>    114
Gly Ile Ser His Asp Ser Gly Ser Lys
1               5


<210>    115
<211>    9
<212>    PRT
<213>    Artificial Sequence

<220>
<223>    GTC110-02 heavy chain CDR3

<400>    115
Ala Arg His Trp Thr Thr Phe Asp Tyr
1               5

<210>    116
<211>    8
<212>    PRT
<213>    Artificial Sequence

<220>
<223>    GTC110-02 light chain CDR1

<400>    116
Ser Ser Asn Ile Gly Ser Asn Asn
1               5

<210>    117
<211>    3
<212>    PRT
<213>    Artificial Sequence

<220>
<223>    GTC110-02 light chain CDR2

<400>    117
Ala Asn Ser
1

<210>    118
<211>    11
<212>    PRT
<213>    Artificial Sequence

<220>
<223>    GTC110-02 light chain CDR3

<400>    118
Gly Ala Trp Asp Tyr Ser Leu Ser Ala Tyr Val
1               5                   10

<210>    119
<211>    8
<212>    PRT
<213>    Artificial Sequence

<220>
<223>    GTC110-03 heavy chain CDR1

<400>    119
Gly Phe Thr Phe Ser Asn Tyr Ala

1               5


<210>   120
<211>   9
<212>   PRT
<213>   Artificial Sequence

<220>
<223>   GTC110-03 heavy chain CDR2


<400>   120
Ala Ile Tyr Pro Gly Gly Gly Ser Ile
1               5


<210>   121
<211>   20
<212>   PRT
<213>   Artificial Sequence

<220>
<223>   GTC110-03 heavy chain CDR3


<400>   121
Ala Arg Asp Ile Leu Pro Cys Pro Trp Gly Arg Cys Tyr Tyr Asp Tyr
1               5                   10                  15

Ala Met Asp Val
            20


<210>   122
<211>   8
<212>   PRT
<213>   Artificial Sequence

<220>
<223>   GTC110-03 light chain CDR1


<400>   122
Ser Ser Asn Ile Gly Ser Asn Thr
1               5


<210>   123
<211>   3
<212>   PRT
<213>   Artificial Sequence

<220>
<223>   GTC110-03 light chain CDR2


<400>   123
Ala Asp Asn
1


<210>   124

<211>    11
<212>    PRT
<213>    Artificial Sequence

<220>
<223>    GTC110-03 light chain CDR3


<400>    124
Gly Thr Trp Asp Tyr Ser Leu Ser Gly Tyr Val
1               5                   10


<210>    125
<211>    8
<212>    PRT
<213>    Artificial Sequence

<220>
<223>    GTC110-04 heavy chain CDR1


<400>    125
Gly Phe Thr Phe Ser Asn Tyr Ala
1               5


<210>    126
<211>    9
<212>    PRT
<213>    Artificial Sequence

<220>
<223>    GTC110-04 heavy chain CDR2


<400>    126
Val Ile Ser His Gly Gly Gly Ser Thr
1               5


<210>    127
<211>    20
<212>    PRT
<213>    Artificial Sequence

<220>
<223>    GTC110-04 heavy chain CDR3


<400>    127
Ala Arg Val Ile Ser Asn Cys His Leu Gly Val Cys Tyr Tyr Ser Asn
1               5                   10                  15

Gly Met Asp Val
            20


<210>    128
<211>    8
<212>    PRT
<213>    Artificial Sequence

<220>
<223>    GTC110-04 light chain CDR1


<400>    128
Ser Ser Asn Ile Gly Asn Asn Asp
1               5


<210>    129
<211>    3
<212>    PRT
<213>    Artificial Sequence

<220>
<223>    GTC110-04 light chain CDR2


<400>    129
Ser Asp Ser
1


<210>    130
<211>    11
<212>    PRT
<213>    Artificial Sequence

<220>
<223>    GTC110-04 light chain CDR3


<400>    130
Gly Thr Trp Asp Tyr Ser Leu Ser Gly Tyr Val
1               5                   10


<210>    131
<211>    121
<212>    PRT
<213>    Artificial Sequence

<220>
<223>    GTC210-01 heavy chain_variable region


<400>    131
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Gly Tyr
            20                  25                  30

Asp Met Ser Trp Val Arg Gln Ala Pro Gly Lys Gly Leu Glu Trp Val
        35                  40                  45

Ser Leu Ile Tyr Pro Asp Ser Gly Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys

85                  90                  95

Ala Arg Asp Ala Gly Leu Ser Trp Ala Gly Ala Phe Asp Tyr Trp Gly
            100                 105                 110

Gln Gly Thr Leu Val Thr Val Ser Ser
        115                 120


<210>   132
<211>   110
<212>   PRT
<213>   Artificial Sequence

<220>
<223>   GTC210-01 light chain_variable region


<400>   132
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Thr Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Gly Ser Trp Asp Tyr Ser Leu
                85                  90                  95

Ser Ala Tyr Val Phe Gly Gly Gly Thr Lys Leu Thr Val Leu
            100                 105                 110


<210>   133
<211>   121
<212>   PRT
<213>   Artificial Sequence

<220>
<223>   GTC210-02 heavy chain_variable region


<400>   133
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Tyr Met Ser Trp Val Arg Gln Ala Pro Gly Lys Gly Leu Glu Trp Val
        35                  40                  45

Ser Gly Ile Ser Pro Gly Asp Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Leu Tyr Ser Asn Pro Asn Glu Pro Phe Asp Tyr Trp Gly
            100                 105                 110

Gln Gly Thr Leu Val Thr Val Ser Ser
        115                 120


<210>   134
<211>   110
<212>   PRT
<213>   Artificial Sequence

<220>
<223>   GTC210-02 light chain_variable region


<400>   134
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Asp Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Asn Gly Tyr Val Phe Gly Gly Gly Thr Lys Leu Thr Val Leu
            100                 105                 110


<210>   135
<211>   127
<212>   PRT
<213>   Artificial Sequence

<220>
<223>   GTC210-03 heavy chain_variable region


<400>   135
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Asp Met Ser Trp Val Arg Gln Ala Pro Gly Lys Gly Leu Glu Trp Val
        35                  40                  45

Ser Gly Ile Ser Pro Asp Gly Ser Asn Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Gly Leu Arg Cys Arg Tyr Glu Ala Cys Ser Tyr Ala Tyr
            100                 105                 110

Gly Met Asp Val Trp Gly Gln Gly Thr Leu Val Thr Val Ser Ser
        115                 120                 125

<210>    136
<211>    110
<212>    PRT
<213>    Artificial Sequence

<220>
<223>    GTC210-03 light chain_variable region

<400>    136
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Thr Trp Asp Ser Ser Leu
                85                  90                  95

Asn Gly Tyr Val Phe Gly Gly Gly Thr Lys Leu Thr Val Leu
            100                 105                 110

<210>    137
<211>    121
<212>    PRT
<213>    Artificial Sequence

<220>
<223>    GTC210-04 heavy chain_variable region

<400>    137
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Asp Met Ser Trp Val Arg Gln Ala Pro Gly Lys Gly Leu Glu Trp Val
        35                  40                  45

Ser Ser Ile Ser Pro Ser Ser Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Leu Asp Ala Phe Trp Arg Pro Ser Phe Asp Tyr Trp Gly
            100                 105                 110

Gln Gly Thr Leu Val Thr Val Ser Ser
        115                 120


<210>    138
<211>    110
<212>    PRT
<213>    Artificial Sequence

<220>
<223>    GTC210-04 light chain_variable region


<400>    138
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Asn Val Asn Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Asp Ser Leu
                85                  90                  95

Ser Ala Tyr Val Phe Gly Gly Gly Thr Lys Leu Thr Val Leu
            100                 105                 110


<210>    139
<211>    127
<212>    PRT
<213>    Artificial Sequence

<220>
<223>    GTC110-01 heavy chain_variable region

<400>    139
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Asp Met Ser Trp Val Arg Gln Val Pro Gly Lys Gly Leu Glu Trp Val
        35                  40                  45

Ser Trp Ile Ser His Gly Gly Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Leu Gly Leu Cys Lys Thr Gly Leu Cys Tyr Tyr Tyr Asp
            100                 105                 110

Ala Met Asp Val Trp Gly Gln Gly Thr Leu Val Thr Val Ser Ser
        115                 120                 125

<210>    140
<211>    110
<212>    PRT
<213>    Artificial Sequence

<220>
<223>    GTC110-01 light chain_variable region

<400>    140
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Ser Val Thr Trp Tyr Gln Gln Leu Pro Gly Thr Ala Pro Lys Leu Leu
        35                  40                  45

Ile Tyr Ala Asp Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Ser Ser Leu
                85                  90                  95

Ser Ala Tyr Val Phe Gly Gly Gly Thr Lys Leu Thr Val Leu
            100                 105                 110

<210>    141
<211>    116

<212>    PRT
<213>    Artificial Sequence

<220>
<223>    GTC110-02 heavy chain_variable region


<400>    141
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Tyr Met Ser Trp Val Arg Gln Ala Pro Gly Lys Gly Leu Glu Trp Val
        35                  40                  45

Ser Gly Ile Ser His Asp Ser Gly Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Trp Thr Thr Phe Asp Tyr Trp Gly Gln Gly Thr Leu Val
            100                 105                 110

Thr Val Ser Ser
        115


<210>    142
<211>    110
<212>    PRT
<213>    Artificial Sequence

<220>
<223>    GTC110-02 light chain_variable region


<400>    142
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Asn Val Thr Trp Tyr Gln Gln Leu Pro Gly Thr Ala Pro Lys Leu Leu
        35                  40                  45

Ile Tyr Ala Asn Ser Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Tyr Ser Leu
                85                  90                  95

Ser Ala Tyr Val Phe Gly Gly Gly Thr Lys Leu Thr Val Leu
            100                 105                 110

<210>    143
<211>    127
<212>    PRT
<213>    Artificial Sequence

<220>
<223>    GTC110-03 heavy chain_variable region


<400>    143
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ala Ile Tyr Pro Gly Gly Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Ile Leu Pro Cys Pro Trp Gly Arg Cys Tyr Tyr Asp Tyr
            100                 105                 110

Ala Met Asp Val Trp Gly Gln Gly Thr Leu Val Thr Val Ser Ser
        115                 120                 125


<210>    144
<211>    110
<212>    PRT
<213>    Artificial Sequence

<220>
<223>    GTC110-03 light chain_variable region


<400>    144
Gln Ser Val Leu Thr Gln Pro Pro Ser Ala Ser Gly Thr Pro Gly Gln
1               5                   10                  15

Arg Val Thr Ile Ser Cys Ser Asp Ser Ser Ser Asn Ile Gly Ser Asn
            20                  25                  30

Thr Val Ser Trp Tyr Gln Gln Leu Pro Gly Thr Ala Pro Lys Leu Leu
        35                  40                  45

Ile Tyr Ala Asp Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Ser Gly Tyr Val Phe Gly Gly Gly Thr Lys Leu Thr Val Leu
            100                 105                 110

<210>    145
<211>    127
<212>    PRT
<213>    Artificial Sequence

<220>
<223>    GTC110-04 heavy chain_variable region

<400>    145
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Val Ile Ser His Gly Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Ile Ser Asn Cys His Leu Gly Val Cys Tyr Tyr Ser Asn
            100                 105                 110

Gly Met Asp Val Trp Gly Gln Gly Thr Leu Val Thr Val Ser Ser
        115                 120                 125

<210>    146
<211>    110
<212>    PRT
<213>    Artificial Sequence

<220>
<223>    GTC110-04 light chain_variable region

<400>    146
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Asn Asn
            20                  25                  30

Asp Val Tyr Trp Tyr Gln Gln Leu Pro Gly Thr Ala Pro Lys Leu Leu
        35                  40                  45

Ile Tyr Ser Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Ser Gly Tyr Val Phe Gly Gly Gly Thr Lys Leu Thr Val Leu
            100                 105                 110


<210>   147
<211>   451
<212>   PRT
<213>   Artificial Sequence

<220>
<223>   GTC210-01 heavy chain_mouse IgG2 Fc_full sequence


<400>   147
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Gly Tyr
            20                  25                  30

Asp Met Ser Trp Val Arg Gln Ala Pro Gly Lys Gly Leu Glu Trp Val
        35                  40                  45

Ser Leu Ile Tyr Pro Asp Ser Gly Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Ala Gly Leu Ser Trp Ala Gly Ala Phe Asp Tyr Trp Gly
            100                 105                 110

Gln Gly Thr Leu Val Thr Val Ser Ser Ala Ser Thr Thr Ala Pro Ser
        115                 120                 125

Val Tyr Pro Leu Ala Pro Val Cys Gly Asp Thr Thr Gly Ser Ser Val
    130                 135                 140

Thr Leu Gly Cys Leu Val Lys Gly Tyr Phe Pro Glu Pro Val Thr Leu
145                 150                 155                 160

Thr Trp Asn Ser Gly Ser Leu Ser Ser Gly Val His Thr Phe Pro Ala
                165                 170                 175

Val Leu Gln Ser Asp Leu Tyr Thr Leu Ser Ser Ser Val Thr Val Thr
            180                 185                 190

Ser Ser Thr Trp Pro Ser Gln Ser Ile Thr Cys Asn Val Ala His Pro
        195                 200                 205

Ala Ser Ser Thr Lys Val Asp Lys Lys Ile Glu Pro Arg Gly Pro Thr
    210                 215                 220

Ile Lys Pro Cys Pro Pro Cys Lys Cys Pro Ala Pro Asn Leu Leu Gly
225                 230                 235                 240

Gly Pro Ser Val Phe Ile Phe Pro Pro Lys Ile Lys Asp Val Leu Met
                245                 250                 255

Ile Ser Leu Ser Pro Ile Val Thr Cys Val Val Val Asp Val Ser Glu
            260                 265                 270

Asp Asp Pro Asp Val Gln Ile Ser Trp Phe Val Asn Asn Val Glu Val
        275                 280                 285

His Thr Ala Gln Thr Gln Thr His Arg Glu Asp Tyr Asn Ser Thr Leu
    290                 295                 300

Arg Val Val Ser Ala Leu Pro Ile Gln His Gln Asp Trp Met Ser Gly
305                 310                 315                 320

Lys Glu Phe Lys Cys Lys Val Asn Asn Lys Asp Leu Pro Ala Pro Ile
                325                 330                 335

Glu Arg Thr Ile Ser Lys Pro Lys Gly Ser Val Arg Ala Pro Gln Val
            340                 345                 350

Tyr Val Leu Pro Pro Pro Glu Glu Glu Met Thr Lys Lys Gln Val Thr
        355                 360                 365

Leu Thr Cys Met Val Thr Asp Phe Met Pro Glu Asp Ile Tyr Val Glu
    370                 375                 380

Trp Thr Asn Asn Gly Lys Thr Glu Leu Asn Tyr Lys Asn Thr Glu Pro
385                 390                 395                 400

Val Leu Asp Ser Asp Gly Ser Tyr Phe Met Tyr Ser Lys Leu Arg Val
                405                 410                 415

Glu Lys Lys Asn Trp Val Glu Arg Asn Ser Tyr Ser Cys Ser Val Val
            420                 425                 430

His Glu Gly Leu His Asn His His Thr Thr Lys Ser Phe Ser Arg Thr
        435                 440                 445

Pro Gly Lys
    450


<210>   148
<211>   217
<212>   PRT
<213>   Artificial Sequence

<220>
<223>   GTC210-01 light chain_mouse IgG2 Fc_full sequence


<400>   148
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn

20                  25                  30

Tyr Val Thr Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Gly Ser Trp Asp Tyr Ser Leu
                85                  90                  95

Ser Ala Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>   149
<211>   451
<212>   PRT
<213>   Artificial Sequence

<220>
<223>   GTC210-02 heavy chain_mouse IgG2 Fc_full sequence


<400>   149
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Tyr Met Ser Trp Val Arg Gln Ala Pro Gly Lys Gly Leu Glu Trp Val
        35                  40                  45

Ser Gly Ile Ser Pro Gly Asp Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Leu Tyr Ser Asn Pro Asn Glu Pro Phe Asp Tyr Trp Gly
            100                 105                 110

Gln Gly Thr Leu Val Thr Val Ser Ser Ala Ser Thr Thr Ala Pro Ser
        115                 120                 125

Val Tyr Pro Leu Ala Pro Val Cys Gly Asp Thr Thr Gly Ser Ser Val
    130                 135                 140

Thr Leu Gly Cys Leu Val Lys Gly Tyr Phe Pro Glu Pro Val Thr Leu
145                 150                 155                 160

Thr Trp Asn Ser Gly Ser Leu Ser Ser Gly Val His Thr Phe Pro Ala
                165                 170                 175

Val Leu Gln Ser Asp Leu Tyr Thr Leu Ser Ser Ser Val Thr Val Thr
            180                 185                 190

Ser Ser Thr Trp Pro Ser Gln Ser Ile Thr Cys Asn Val Ala His Pro
        195                 200                 205

Ala Ser Ser Thr Lys Val Asp Lys Lys Ile Glu Pro Arg Gly Pro Thr
    210                 215                 220

Ile Lys Pro Cys Pro Pro Cys Lys Cys Pro Ala Pro Asn Leu Leu Gly
225                 230                 235                 240

Gly Pro Ser Val Phe Ile Phe Pro Pro Lys Ile Lys Asp Val Leu Met
                245                 250                 255

Ile Ser Leu Ser Pro Ile Val Thr Cys Val Val Val Asp Val Ser Glu
            260                 265                 270

Asp Asp Pro Asp Val Gln Ile Ser Trp Phe Val Asn Asn Val Glu Val
        275                 280                 285

His Thr Ala Gln Thr Gln Thr His Arg Glu Asp Tyr Asn Ser Thr Leu
    290                 295                 300

Arg Val Val Ser Ala Leu Pro Ile Gln His Gln Asp Trp Met Ser Gly
305                 310                 315                 320

Lys Glu Phe Lys Cys Lys Val Asn Asn Lys Asp Leu Pro Ala Pro Ile
                325                 330                 335

Glu Arg Thr Ile Ser Lys Pro Lys Gly Ser Val Arg Ala Pro Gln Val
            340                 345                 350

Tyr Val Leu Pro Pro Pro Glu Glu Glu Met Thr Lys Lys Gln Val Thr
        355                 360                 365

Leu Thr Cys Met Val Thr Asp Phe Met Pro Glu Asp Ile Tyr Val Glu
    370                 375                 380

Trp Thr Asn Asn Gly Lys Thr Glu Leu Asn Tyr Lys Asn Thr Glu Pro
385                 390                 395                 400

Val Leu Asp Ser Asp Gly Ser Tyr Phe Met Tyr Ser Lys Leu Arg Val
                405                 410                 415

Glu Lys Lys Asn Trp Val Glu Arg Asn Ser Tyr Ser Cys Ser Val Val
            420                 425                 430

His Glu Gly Leu His Asn His His Thr Thr Lys Ser Phe Ser Arg Thr
        435                 440                 445

Pro Gly Lys
    450

<210> 150
<211> 217
<212> PRT
<213> Artificial Sequence

<220>
<223> GTC210-02 light chain_mouse IgG2 Fc_full sequence

<400> 150
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Asp Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Asn Gly Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215

<210>    151
<211>    457
<212>    PRT
<213>    Artificial Sequence

<220>
<223>    GTC210-03 heavy chain_mouse IgG2 Fc_full sequence


<400>    151
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Asp Met Ser Trp Val Arg Gln Ala Pro Gly Lys Gly Leu Glu Trp Val
        35                  40                  45

Ser Gly Ile Ser Pro Asp Gly Ser Asn Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Gly Leu Arg Cys Arg Tyr Glu Ala Cys Ser Tyr Ala Tyr
            100                 105                 110

Gly Met Asp Val Trp Gly Gln Gly Thr Leu Val Thr Val Ser Ser Ala
        115                 120                 125

Ser Thr Thr Ala Pro Ser Val Tyr Pro Leu Ala Pro Val Cys Gly Asp
    130                 135                 140

Thr Thr Gly Ser Ser Val Thr Leu Gly Cys Leu Val Lys Gly Tyr Phe
145                 150                 155                 160

Pro Glu Pro Val Thr Leu Thr Trp Asn Ser Gly Ser Leu Ser Ser Gly
                165                 170                 175

Val His Thr Phe Pro Ala Val Leu Gln Ser Asp Leu Tyr Thr Leu Ser
            180                 185                 190

Ser Ser Val Thr Val Thr Ser Ser Thr Trp Pro Ser Gln Ser Ile Thr
        195                 200                 205

Cys Asn Val Ala His Pro Ala Ser Ser Thr Lys Val Asp Lys Lys Ile
    210                 215                 220

Glu Pro Arg Gly Pro Thr Ile Lys Pro Cys Pro Pro Cys Lys Cys Pro
225                 230                 235                 240

Ala Pro Asn Leu Leu Gly Gly Pro Ser Val Phe Ile Phe Pro Pro Lys
                245                 250                 255

Ile Lys Asp Val Leu Met Ile Ser Leu Ser Pro Ile Val Thr Cys Val
            260                 265                 270

Val Val Asp Val Ser Glu Asp Asp Pro Asp Val Gln Ile Ser Trp Phe

275                 280                 285

Val Asn Asn Val Glu Val His Thr Ala Gln Thr Gln Thr His Arg Glu
    290                 295                 300

Asp Tyr Asn Ser Thr Leu Arg Val Val Ser Ala Leu Pro Ile Gln His
305                 310                 315                 320

Gln Asp Trp Met Ser Gly Lys Glu Phe Lys Cys Lys Val Asn Asn Lys
                325                 330                 335

Asp Leu Pro Ala Pro Ile Glu Arg Thr Ile Ser Lys Pro Lys Gly Ser
            340                 345                 350

Val Arg Ala Pro Gln Val Tyr Val Leu Pro Pro Pro Glu Glu Glu Met
        355                 360                 365

Thr Lys Lys Gln Val Thr Leu Thr Cys Met Val Thr Asp Phe Met Pro
    370                 375                 380

Glu Asp Ile Tyr Val Glu Trp Thr Asn Asn Gly Lys Thr Glu Leu Asn
385                 390                 395                 400

Tyr Lys Asn Thr Glu Pro Val Leu Asp Ser Asp Gly Ser Tyr Phe Met
                405                 410                 415

Tyr Ser Lys Leu Arg Val Glu Lys Lys Asn Trp Val Glu Arg Asn Ser
            420                 425                 430

Tyr Ser Cys Ser Val Val His Glu Gly Leu His Asn His His Thr Thr
        435                 440                 445

Lys Ser Phe Ser Arg Thr Pro Gly Lys
    450                 455


<210>   152
<211>   217
<212>   PRT
<213>   Artificial Sequence

<220>
<223>   GTC210-03 light chain_mouse IgG2 Fc_full sequence


<400>   152
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Thr Trp Asp Ser Ser Leu
                85                  90                  95

Asn Gly Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210> 153
<211> 449
<212> PRT
<213> Artificial Sequence

<220>
<223> GTC210-04 heavy chain_mouse IgG2 Fc_full sequence


<400> 153
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Asp Met Ser Trp Val Arg Gln Ala Pro Gly Lys Gly Leu Glu Trp Val
        35                  40                  45

Ser Ser Ile Ser Pro Ser Ser Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Leu Asp Ala Phe Trp Arg Pro Ser Phe Asp Tyr Trp Gly
            100                 105                 110

Gln Gly Thr Leu Val Thr Val Ser Ser Thr Thr Ala Pro Ser Val Tyr
        115                 120                 125

Pro Leu Ala Pro Val Cys Gly Asp Thr Thr Gly Ser Ser Val Thr Leu
    130                 135                 140

Gly Cys Leu Val Lys Gly Tyr Phe Pro Glu Pro Val Thr Leu Thr Trp
145                 150                 155                 160

Asn Ser Gly Ser Leu Ser Ser Gly Val His Thr Phe Pro Ala Val Leu
                165                 170                 175

Gln Ser Asp Leu Tyr Thr Leu Ser Ser Ser Val Thr Val Thr Ser Ser
            180                 185                 190

Thr Trp Pro Ser Gln Ser Ile Thr Cys Asn Val Ala His Pro Ala Ser
        195                 200                 205

Ser Thr Lys Val Asp Lys Lys Ile Glu Pro Arg Gly Pro Thr Ile Lys
    210                 215                 220

Pro Cys Pro Pro Cys Lys Cys Pro Ala Pro Asn Leu Leu Gly Gly Pro
225                 230                 235                 240

Ser Val Phe Ile Phe Pro Pro Lys Ile Lys Asp Val Leu Met Ile Ser
                245                 250                 255

Leu Ser Pro Ile Val Thr Cys Val Val Val Asp Val Ser Glu Asp Asp
            260                 265                 270

Pro Asp Val Gln Ile Ser Trp Phe Val Asn Asn Val Glu Val His Thr
        275                 280                 285

Ala Gln Thr Gln Thr His Arg Glu Asp Tyr Asn Ser Thr Leu Arg Val
    290                 295                 300

Val Ser Ala Leu Pro Ile Gln His Gln Asp Trp Met Ser Gly Lys Glu
305                 310                 315                 320

Phe Lys Cys Lys Val Asn Asn Lys Asp Leu Pro Ala Pro Ile Glu Arg
                325                 330                 335

Thr Ile Ser Lys Pro Lys Gly Ser Val Arg Ala Pro Gln Val Tyr Val
            340                 345                 350

Leu Pro Pro Pro Glu Glu Glu Met Thr Lys Lys Gln Val Thr Leu Thr
        355                 360                 365

Cys Met Val Thr Asp Phe Met Pro Glu Asp Ile Tyr Val Glu Trp Thr
    370                 375                 380

Asn Asn Gly Lys Thr Glu Leu Asn Tyr Lys Asn Thr Glu Pro Val Leu
385                 390                 395                 400

Asp Ser Asp Gly Ser Tyr Phe Met Tyr Ser Lys Leu Arg Val Glu Lys
                405                 410                 415

Lys Asn Trp Val Glu Arg Asn Ser Tyr Ser Cys Ser Val Val His Glu
            420                 425                 430

Gly Leu His Asn His His Thr Thr Lys Ser Phe Ser Arg Thr Pro Gly
        435                 440                 445

Lys

<210>   154
<211>   217

<212>    PRT
<213>    Artificial Sequence

<220>
<223>    GTC210-04 light chain_mouse IgG2 Fc_full sequence


<400>    154
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Asn Val Asn Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Asp Ser Leu
                85                  90                  95

Ser Ala Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    155
<211>    451
<212>    PRT
<213>    Artificial Sequence

<220>
<223>    GTC110-01 heavy chain_mouse IgG2 Fc_full sequence


<400>    155
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Asp Met Ser Trp Val Arg Gln Ala Pro Gly Lys Gly Leu Glu Trp Val
        35                  40                  45

Ser Ser Ile Ser Pro Ser Ser Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Leu Asp Ala Phe Trp Arg Pro Ser Phe Asp Tyr Trp Gly
            100                 105                 110

Gln Gly Thr Leu Val Thr Val Ser Ser Ala Ser Thr Thr Ala Pro Ser
        115                 120                 125

Val Tyr Pro Leu Ala Pro Val Cys Gly Asp Thr Thr Gly Ser Ser Val
    130                 135                 140

Thr Leu Gly Cys Leu Val Lys Gly Tyr Phe Pro Glu Pro Val Thr Leu
145                 150                 155                 160

Thr Trp Asn Ser Gly Ser Leu Ser Ser Gly Val His Thr Phe Pro Ala
                165                 170                 175

Val Leu Gln Ser Asp Leu Tyr Thr Leu Ser Ser Ser Val Thr Val Thr
            180                 185                 190

Ser Ser Thr Trp Pro Ser Gln Ser Ile Thr Cys Asn Val Ala His Pro
        195                 200                 205

Ala Ser Ser Thr Lys Val Asp Lys Lys Ile Glu Pro Arg Gly Pro Thr
    210                 215                 220

Ile Lys Pro Cys Pro Pro Cys Lys Cys Pro Ala Pro Asn Leu Leu Gly
225                 230                 235                 240

Gly Pro Ser Val Phe Ile Phe Pro Pro Lys Ile Lys Asp Val Leu Met
                245                 250                 255

Ile Ser Leu Ser Pro Ile Val Thr Cys Val Val Val Asp Val Ser Glu
            260                 265                 270

Asp Asp Pro Asp Val Gln Ile Ser Trp Phe Val Asn Asn Val Glu Val
        275                 280                 285

His Thr Ala Gln Thr Gln Thr His Arg Glu Asp Tyr Asn Ser Thr Leu
    290                 295                 300

Arg Val Val Ser Ala Leu Pro Ile Gln His Gln Asp Trp Met Ser Gly
305                 310                 315                 320

Lys Glu Phe Lys Cys Lys Val Asn Asn Lys Asp Leu Pro Ala Pro Ile
                325                 330                 335

Glu Arg Thr Ile Ser Lys Pro Lys Gly Ser Val Arg Ala Pro Gln Val
            340                 345                 350

Tyr Val Leu Pro Pro Pro Glu Glu Glu Met Thr Lys Lys Gln Val Thr
        355                 360                 365

Leu Thr Cys Met Val Thr Asp Phe Met Pro Glu Asp Ile Tyr Val Glu
    370                 375                 380

Trp Thr Asn Asn Gly Lys Thr Glu Leu Asn Tyr Lys Asn Thr Glu Pro
385                 390                 395                 400

Val Leu Asp Ser Asp Gly Ser Tyr Phe Met Tyr Ser Lys Leu Arg Val
                405                 410                 415

Glu Lys Lys Asn Trp Val Glu Arg Asn Ser Tyr Ser Cys Ser Val Val
            420                 425                 430

His Glu Gly Leu His Asn His His Thr Thr Lys Ser Phe Ser Arg Thr
        435                 440                 445

Pro Gly Lys
    450

<210> 156
<211> 217
<212> PRT
<213> Artificial Sequence

<220>
<223> GTC110-01 light chain_mouse IgG2 Fc_full sequence

<400> 156
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Ser Val Thr Trp Tyr Gln Gln Leu Pro Gly Thr Ala Pro Lys Leu Leu
        35                  40                  45

Ile Tyr Ala Asp Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Ser Ser Leu
                85                  90                  95

Ser Ala Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>   157
<211>   446
<212>   PRT
<213>   Artificial Sequence

<220>
<223>   GTC110-02 heavy chain_mouse IgG2 Fc_full sequence


<400>   157
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Tyr Met Ser Trp Val Arg Gln Ala Pro Gly Lys Gly Leu Glu Trp Val
        35                  40                  45

Ser Gly Ile Ser His Asp Ser Gly Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Trp Thr Thr Phe Asp Tyr Trp Gly Gln Gly Thr Leu Val
            100                 105                 110

Thr Val Ser Ser Ala Ser Thr Thr Ala Pro Ser Val Tyr Pro Leu Ala
        115                 120                 125

Pro Val Cys Gly Asp Thr Thr Gly Ser Ser Val Thr Leu Gly Cys Leu
    130                 135                 140

Val Lys Gly Tyr Phe Pro Glu Pro Val Thr Leu Thr Trp Asn Ser Gly
145                 150                 155                 160

Ser Leu Ser Ser Gly Val His Thr Phe Pro Ala Val Leu Gln Ser Asp
                165                 170                 175

Leu Tyr Thr Leu Ser Ser Ser Val Thr Val Thr Ser Ser Thr Trp Pro
            180                 185                 190

Ser Gln Ser Ile Thr Cys Asn Val Ala His Pro Ala Ser Ser Thr Lys
        195                 200                 205

Val Asp Lys Lys Ile Glu Pro Arg Gly Pro Thr Ile Lys Pro Cys Pro

210                 215                 220

Pro Cys Lys Cys Pro Ala Pro Asn Leu Leu Gly Gly Pro Ser Val Phe
225                 230                 235                 240

Ile Phe Pro Pro Lys Ile Lys Asp Val Leu Met Ile Ser Leu Ser Pro
                245                 250                 255

Ile Val Thr Cys Val Val Val Asp Val Ser Glu Asp Asp Pro Asp Val
            260                 265                 270

Gln Ile Ser Trp Phe Val Asn Asn Val Glu Val His Thr Ala Gln Thr
        275                 280                 285

Gln Thr His Arg Glu Asp Tyr Asn Ser Thr Leu Arg Val Val Ser Ala
    290                 295                 300

Leu Pro Ile Gln His Gln Asp Trp Met Ser Gly Lys Glu Phe Lys Cys
305                 310                 315                 320

Lys Val Asn Asn Lys Asp Leu Pro Ala Pro Ile Glu Arg Thr Ile Ser
                325                 330                 335

Lys Pro Lys Gly Ser Val Arg Ala Pro Gln Val Tyr Val Leu Pro Pro
            340                 345                 350

Pro Glu Glu Glu Met Thr Lys Lys Gln Val Thr Leu Thr Cys Met Val
        355                 360                 365

Thr Asp Phe Met Pro Glu Asp Ile Tyr Val Glu Trp Thr Asn Asn Gly
    370                 375                 380

Lys Thr Glu Leu Asn Tyr Lys Asn Thr Glu Pro Val Leu Asp Ser Asp
385                 390                 395                 400

Gly Ser Tyr Phe Met Tyr Ser Lys Leu Arg Val Glu Lys Lys Asn Trp
                405                 410                 415

Val Glu Arg Asn Ser Tyr Ser Cys Ser Val Val His Glu Gly Leu His
            420                 425                 430

Asn His His Thr Thr Lys Ser Phe Ser Arg Thr Pro Gly Lys
        435                 440                 445



<210>   158
<211>   217
<212>   PRT
<213>   Artificial Sequence

<220>
<223>   GTC110-02 light chain_mouse IgG2 Fc_full sequence


<400>   158
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Asn Val Thr Trp Tyr Gln Gln Leu Pro Gly Thr Ala Pro Lys Leu Leu

35                  40                  45

Ile Tyr Ala Asn Ser Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Tyr Ser Leu
                85                  90                  95

Ser Ala Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>   159
<211>   457
<212>   PRT
<213>   Artificial Sequence

<220>
<223>   GTC110-03 heavy chain_mouse IgG2 Fc_full sequence


<400>   159
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ala Ile Tyr Pro Gly Gly Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Ile Leu Pro Cys Pro Trp Gly Arg Cys Tyr Tyr Asp Tyr
            100                 105                 110

Ala Met Asp Val Trp Gly Gln Gly Thr Leu Val Thr Val Ser Ser Ala
        115                 120                 125

Ser Thr Thr Ala Pro Ser Val Tyr Pro Leu Ala Pro Val Cys Gly Asp
    130                 135                 140

Thr Thr Gly Ser Ser Val Thr Leu Gly Cys Leu Val Lys Gly Tyr Phe
145                 150                 155                 160

Pro Glu Pro Val Thr Leu Thr Trp Asn Ser Gly Ser Leu Ser Ser Gly
                165                 170                 175

Val His Thr Phe Pro Ala Val Leu Gln Ser Asp Leu Tyr Thr Leu Ser
            180                 185                 190

Ser Ser Val Thr Val Thr Ser Ser Thr Trp Pro Ser Gln Ser Ile Thr
        195                 200                 205

Cys Asn Val Ala His Pro Ala Ser Ser Thr Lys Val Asp Lys Lys Ile
    210                 215                 220

Glu Pro Arg Gly Pro Thr Ile Lys Pro Cys Pro Pro Cys Lys Cys Pro
225                 230                 235                 240

Ala Pro Asn Leu Leu Gly Gly Pro Ser Val Phe Ile Phe Pro Pro Lys
                245                 250                 255

Ile Lys Asp Val Leu Met Ile Ser Leu Ser Pro Ile Val Thr Cys Val
            260                 265                 270

Val Val Asp Val Ser Glu Asp Asp Pro Asp Val Gln Ile Ser Trp Phe
        275                 280                 285

Val Asn Asn Val Glu Val His Thr Ala Gln Thr Gln Thr His Arg Glu
    290                 295                 300

Asp Tyr Asn Ser Thr Leu Arg Val Val Ser Ala Leu Pro Ile Gln His
305                 310                 315                 320

Gln Asp Trp Met Ser Gly Lys Glu Phe Lys Cys Lys Val Asn Asn Lys
                325                 330                 335

Asp Leu Pro Ala Pro Ile Glu Arg Thr Ile Ser Lys Pro Lys Gly Ser
            340                 345                 350

Val Arg Ala Pro Gln Val Tyr Val Leu Pro Pro Pro Glu Glu Glu Met
        355                 360                 365

Thr Lys Lys Gln Val Thr Leu Thr Cys Met Val Thr Asp Phe Met Pro
    370                 375                 380

Glu Asp Ile Tyr Val Glu Trp Thr Asn Asn Gly Lys Thr Glu Leu Asn
385                 390                 395                 400

Tyr Lys Asn Thr Glu Pro Val Leu Asp Ser Asp Gly Ser Tyr Phe Met
                405                 410                 415

Tyr Ser Lys Leu Arg Val Glu Lys Lys Asn Trp Val Glu Arg Asn Ser
            420                 425                 430

Tyr Ser Cys Ser Val Val His Glu Gly Leu His Asn His His Thr Thr
        435                 440                 445

Lys Ser Phe Ser Arg Thr Pro Gly Lys
    450                 455

<210>   160
<211>   217
<212>   PRT
<213>   Artificial Sequence

<220>
<223>   GTC110-03 light chain_mouse IgG2 Fc_full sequence

<400>   160
Gln Ser Val Leu Thr Gln Pro Pro Ser Ala Ser Gly Thr Pro Gly Gln
1               5                   10                  15

Arg Val Thr Ile Ser Cys Ser Asp Ser Ser Ser Asn Ile Gly Ser Asn
            20                  25                  30

Thr Val Ser Trp Tyr Gln Gln Leu Pro Gly Thr Ala Pro Lys Leu Leu
        35                  40                  45

Ile Tyr Ala Asp Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Ser Gly Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215

<210>   161
<211>   457

<212>    PRT
<213>    Artificial Sequence

<220>
<223>    GTC110-04 heavy chain_mouse IgG2 Fc_full sequence


<400>    161
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Val Ile Ser His Gly Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Ile Ser Asn Cys His Leu Gly Val Cys Tyr Tyr Ser Asn
            100                 105                 110

Gly Met Asp Val Trp Gly Gln Gly Thr Leu Val Thr Val Ser Ser Ala
        115                 120                 125

Ser Thr Thr Ala Pro Ser Val Tyr Pro Leu Ala Pro Val Cys Gly Asp
    130                 135                 140

Thr Thr Gly Ser Ser Val Thr Leu Gly Cys Leu Val Lys Gly Tyr Phe
145                 150                 155                 160

Pro Glu Pro Val Thr Leu Thr Trp Asn Ser Gly Ser Leu Ser Ser Gly
                165                 170                 175

Val His Thr Phe Pro Ala Val Leu Gln Ser Asp Leu Tyr Thr Leu Ser
            180                 185                 190

Ser Ser Val Thr Val Thr Ser Ser Thr Trp Pro Ser Gln Ser Ile Thr
        195                 200                 205

Cys Asn Val Ala His Pro Ala Ser Ser Thr Lys Val Asp Lys Lys Ile
    210                 215                 220

Glu Pro Arg Gly Pro Thr Ile Lys Pro Cys Pro Pro Cys Lys Cys Pro
225                 230                 235                 240

Ala Pro Asn Leu Leu Gly Gly Pro Ser Val Phe Ile Phe Pro Pro Lys
                245                 250                 255

Ile Lys Asp Val Leu Met Ile Ser Leu Ser Pro Ile Val Thr Cys Val
            260                 265                 270

Val Val Asp Val Ser Glu Asp Asp Pro Asp Val Gln Ile Ser Trp Phe
        275                 280                 285

Val Asn Asn Val Glu Val His Thr Ala Gln Thr Gln Thr His Arg Glu

290                 295                 300

Asp Tyr Asn Ser Thr Leu Arg Val Val Ser Ala Leu Pro Ile Gln His
305                 310                 315                 320

Gln Asp Trp Met Ser Gly Lys Glu Phe Lys Cys Lys Val Asn Asn Lys
                325                 330                 335

Asp Leu Pro Ala Pro Ile Glu Arg Thr Ile Ser Lys Pro Lys Gly Ser
            340                 345                 350

Val Arg Ala Pro Gln Val Tyr Val Leu Pro Pro Pro Glu Glu Glu Met
        355                 360                 365

Thr Lys Lys Gln Val Thr Leu Thr Cys Met Val Thr Asp Phe Met Pro
    370                 375                 380

Glu Asp Ile Tyr Val Glu Trp Thr Asn Asn Gly Lys Thr Glu Leu Asn
385                 390                 395                 400

Tyr Lys Asn Thr Glu Pro Val Leu Asp Ser Asp Gly Ser Tyr Phe Met
                405                 410                 415

Tyr Ser Lys Leu Arg Val Glu Lys Lys Asn Trp Val Glu Arg Asn Ser
            420                 425                 430

Tyr Ser Cys Ser Val Val His Glu Gly Leu His Asn His His Thr Thr
        435                 440                 445

Lys Ser Phe Ser Arg Thr Pro Gly Lys
    450                 455


<210>   162
<211>   217
<212>   PRT
<213>   Artificial Sequence

<220>
<223>   GTC110-04 light chain_mouse IgG2 Fc_full sequence


<400>   162
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Asn Asn
            20                  25                  30

Asp Val Tyr Trp Tyr Gln Gln Leu Pro Gly Thr Ala Pro Lys Leu Leu
        35                  40                  45

Ile Tyr Ser Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Ser Gly Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    163
<211>    458
<212>    PRT
<213>    Artificial Sequence

<220>
<223>    GTC110-04 heavy chain_human_full sequence


<400>    163
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Val Ile Ser His Gly Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Ile Ser Asn Cys His Leu Gly Val Cys Tyr Tyr Ser Asn
            100                 105                 110

Gly Met Asp Val Trp Gly Gln Gly Thr Leu Val Thr Val Ser Ser Ala
        115                 120                 125

Ser Thr Lys Gly Pro Ser Val Phe Pro Leu Ala Pro Ser Ser Lys Ser
    130                 135                 140

Thr Ser Gly Gly Thr Ala Ala Leu Gly Cys Leu Val Lys Asp Tyr Phe
145                 150                 155                 160

Pro Glu Pro Val Thr Val Ser Trp Asn Ser Gly Ala Leu Thr Ser Gly
                165                 170                 175

Val His Thr Phe Pro Ala Val Leu Gln Ser Ser Gly Leu Tyr Ser Leu
            180                 185                 190

Ser Ser Val Val Thr Val Pro Ser Ser Ser Leu Gly Thr Gln Thr Tyr
        195                 200                 205

Ile Cys Asn Val Asn His Lys Pro Ser Asn Thr Lys Val Asp Lys Lys
    210                 215                 220

Val Glu Pro Lys Ser Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Tyr Thr Leu Pro Pro Ser Arg Glu Glu Met
        355                 360                 365

Thr Lys Asn Gln Val Ser Leu Thr Cys Leu Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Tyr Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys Tyr
    450                 455


<210>   164
<211>   217
<212>   PRT
<213>   Artificial Sequence

<220>
<223>    GTC110-04 light chain_human_full sequence


<400>    164
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Asn Asn
            20                  25                  30

Asp Val Tyr Trp Tyr Gln Gln Leu Pro Gly Thr Ala Pro Lys Leu Leu
        35                  40                  45

Ile Tyr Ser Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Ser Gly Tyr Val Phe Gly Gly Gly Thr Lys Leu Thr Val Leu Arg Thr
            100                 105                 110

Val Ala Ala Pro Ser Val Phe Ile Phe Pro Pro Ser Asp Glu Gln Leu
        115                 120                 125

Lys Ser Gly Thr Ala Ser Val Val Cys Leu Leu Asn Asn Phe Tyr Pro
    130                 135                 140

Arg Glu Ala Lys Val Gln Trp Lys Val Asp Asn Ala Leu Gln Ser Gly
145                 150                 155                 160

Asn Ser Gln Glu Ser Val Thr Glu Gln Asp Ser Lys Asp Ser Thr Tyr
                165                 170                 175

Ser Leu Ser Ser Thr Leu Thr Leu Ser Lys Ala Asp Tyr Glu Lys His
            180                 185                 190

Lys Val Tyr Ala Cys Glu Val Thr His Gln Gly Leu Ser Ser Pro Val
        195                 200                 205

Thr Lys Ser Phe Asn Arg Gly Glu Cys
    210                 215